(19) Europäisches Patentamt
European Patent Office
Office européen des brevets

(11) EP 1 668 151 B1

(12) **EUROPEAN PATENT SPECIFICATION**

(45) Date of publication and mention of the grant of the patent:
**17.12.2008 Bulletin 2008/51**

(21) Application number: **04768731.4**

(22) Date of filing: **01.10.2004**

(51) Int Cl.:
***C12Q 1/68*** *(2006.01)*

(86) International application number:
**PCT/GB2004/004190**

(87) International publication number:
**WO 2005/033336 (14.04.2005 Gazette 2005/15)**

(54) **MATERIALS AND METHODS RELATING TO BREAST CANCER DIAGNOSIS**

MATERIALIEN UND VERFAHREN IN VERBINDUNG MIT BRUSTKREBSDIAGNOSE

MATERIAUX ET PROCEDES RELATIFS AU DEPISTAGE DU CANCER DU SEIN

(84) Designated Contracting States:
**AT BE BG CH CY CZ DE DK EE ES FI FR GB GR HU IE IT LI LU MC NL PL PT RO SE SI SK TR**

(30) Priority: **03.10.2003 GB 0323226**

(43) Date of publication of application:
**14.06.2006 Bulletin 2006/24**

(73) Proprietor: **NCC Technology Ventures Pte Limited**
**169610 Singapore (SG)**

(72) Inventors:
- **YU, Kun**
  **Singapore 169610 (SG)**
- **TAN, Patrick**
  **Singapore 169610 (SG)**

(74) Representative: **Forrest, Graham Robert et al**
**Mewburn Ellis LLP**
**York House**
**23 Kingsway**
**London WC2B 6HP (GB)**

(56) References cited:
**WO-A-02/103320** **WO-A-03/070979**

- **"Affimetrix GeneChip Human Genome U133 Array Set HG-U133A" GEO, 2002, XP002254749**
- **SATO NORIHIRO ET AL: "Discovery of novel targets for aberrant methylation in pancreatic carcinoma using high-throughput microarrays." CANCER RESEARCH, vol. 63, no. 13, 1 July 2003 (2003-07-01), pages 3735-3742, XP002318399 ISSN: 0008-5472**
- **GRUVBERGER S ET AL: "Estrogen receptor status in breast cancer is associated with remarkable distinct gene expression patterns" CANCER RESEARCH, AMERICAN ASSOCIATION FOR CANCER RESEARCH, BALTIMORE, MD, US, vol. 61, no. 16, 15 August 2001 (2001-08-15), pages 5979-5984, XP002964618 ISSN: 0008-5472 cited in the application**
- **WEST MIKE ET AL: "Predicting the clinical status of human breast cancer by using gene expression profiles" PROCEEDINGS OF THE NATIONAL ACADEMY OF SCIENCES OF THE UNITED STATES OF AMERICA, vol. 98, no. 20, 25 September 2001 (2001-09-25), pages 11462-11467, XP002318400 ISSN: 0027-8424 cited in the application**
- **BERTUCCI F ET AL: "Gene expression profiling of primary breast carcinomas using arrays of cancidate genes" HUMAN MOLECULAR GENETICS, OXFORD UNIVERSITY PRESS, SURREY, GB, vol. 9, no. 20, 2000, pages 2981-2991, XP002225994 ISSN: 0964-6906**
- **VEER VAN 'T L J ET AL: "Gene expression profiling predicts clinical outcome of breast cancer" NATURE, MACMILLAN JOURNALS LTD. LONDON, GB, vol. 415, no. 6871, 31 January 2002 (2002-01-31), pages 530-536, XP002259781 ISSN: 0028-0836**
- **VIJVER VAN DE M J ET AL: "THE NEW ENGLAND JOURNAL OF MEDICINE A GENE-EXPRESSION SIGNATURE AS A PREDICTOR OF SURVIVAL IN BREAST CANCER" NEW ENGLAND JOURNAL OF MEDICINE, MASSACHUSETTS MEDICAL SOCIETY, BOSTON, MA, US, vol. 347, no. 25, 19 December 2002 (2002-12-19), pages 1999-2009, XP008032093 ISSN: 1533-4406**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 668 151 B1

- BERTUCCI F ET AL: "GENE EXPRESSION PROFILES OF POOR-PROGNOSIS PRIMARY BREAST CANCER CORRELATE WITH SURVIVAL" HUMAN MOLECULAR GENETICS, OXFORD UNIVERSITY PRESS, SURREY, GB, vol. 11, no. 8, 15 April 2002 (2002-04-15), pages 863-872, XP001146244 ISSN: 0964-6906
- SORLIE T ET AL: "Gene expression patterns of breast carcinomas distinguish tumor subclasses with clinical implications" PROCEEDINGS OF THE NATIONAL ACADEMY OF SCIENCES OF USA, NATIONAL ACADEMY OF SCIENCE. WASHINGTON, US, vol. 98, no. 19, 11 September 2001 (2001-09-11), pages 10869-10874, XP002215483 ISSN: 0027-8424
- YU KUN ET AL: "A molecular signature of the Nottingham prognostic index in breast cancer" CANCER RESEARCH, AMERICAN ASSOCIATION FOR CANCER RESEARCH, BALTIMORE, MD, US, vol. 64, no. 9, 1 May 2004 (2004-05-01), pages 2962-2968, XP002319405 ISSN: 0008-5472

## Description

### Field

The present invention concerns materials and methods relating to the diagnosis of breast cancer. Particularly, the present invention concerns the diagnosis and/or classification of "low confidence" tumours which exhibit a significantly worse overall survival and shorter time to distant metastasis compared to their "high confidence" counterparts.

### Background

There has been an intense interest in the use of gene expression data for biological classification, particularly in the fields of oncology and medicine. One exciting aspect of this approach has been its ability to define clinically relevant subtypes of cancer that have previously eluded more traditional light-microscopy approaches (15,16). Despite this potential, a number of issues have to be resolved before the use of gene expression data for clinical diagnosis can become a reality. For example, algorithms need to be implemented that, besides delivering the correct classification, can also accurately determine the confidence of the prediction. This is particularly important if the classification affects the subsequent course of treatment - if furnished with such information, the treating physician can then weigh the confidence of prediction with the potential morbidity of a specific intervention to make an informed clinical choice.

The classification of breast tumours into Estrogen Receptor positive (ER+) and negative (ER-) subtypes is a critical distinction in the treatment of breast cancer. ER- tumours are in general more clinically aggressive than their ER+ counterparts, and ER+ tumours are routinely treated using anti- hormonal therapies such as tamoxifen (1). Presently, a tumour's ER status is routinely determined by immunohistochemistry (IHC) or immunoblotting using an antibody to ER. This technique, however, is imperfect - for example, it may fail to detect tumours harboring genetic alterations in ER that render it inactive or constitutively active (2). Thus, it is crucially important to develop more accurate methodologies to improve the ER subtype classification of breast tumours, so that the appropriate therapies can be subsequently applied. A number of groups have recently published reports utilizing expression profile data to classify breast cancers into ER+ and ER- categories. In one study, it was found that the expression profiles of ER+ and ER- tumours are 'remarkably distinct', supporting previous theories that ER+ and ER- tumours may arise from distinct breast epithelial cell types (3).

Another group has reported the use of supervised learning methodologies on expression data to classify breast tumours by ER subtype (4). One common observation in these studies was that that although the majority of breast tumours could usually be accurately classified into ER+ and ER- subtypes to a high degree of certainty, there always existed a set of 'low-confidence' samples that were either misclassified or where the statistical 'confidence' of the predictions was marginal. Although it was proposed that these 'low-confidence' samples might reflect the effects of population heterogeneity (4), the hypothesis that such 'low-confidence' samples might be biologically distinct from their 'high-confidence' counterparts has not been fully explored to date.

### Summary

The present inventors considered the possibility that the 'low confidence' samples might possess distinct biological characteristics. In order to assess this, they performed a classification analysis using an in-house generated breast cancer expression dataset, and determined that in comparison to the 'high confidence' tumours, the 'low-confidence' tumours exhibit widespread perturbations in the expression of multiple genes important for ER subtype discrimination. Although initially derived through purely computational means, the distinction between 'high' and 'low' confidence tumours is clinically meaningful, as 'low-confidence' tumours exhibited a significantly worse overall survival ($p=0.0003$) and shorter time to distant metastasis ($p=0.0001$) than their 'high-confidence' counterparts. Such a distinction is currently not discernible by conventional immunohistochemical strategies used to detect ER.

The inventors have surprisingly further determined that high expression levels of the ERBB2 receptor are significantly correlated with breast tumours exhibiting a 'low confidence' prediction, and validated this association across three independently-derived breast cancer expression datasets generated from different patient populations/array technologies, and analyzed using different computational methods. The association between ERBB2 expression and the widespread perturbations of ER-discriminator genes observed in the 'low-confidence' tumours is intriguing, as ERBB2 activity is known to contribute, in both breast tumours and cell lines, towards the development of resistance to anti-hormonal therapies (5,6), and to inhibit the transcriptional activity of ER (5,7).

However, despite being important for ER subtype discrimination, the inventors found that a significant proportion of these 'perturbed' genes, are not known to be estrogen responsive, and using a recently described bioinformatics algorithm (DEREF) also demonstrated that these genes do not contain potential estrogen-response elements (ERE's) in their promoters. These results suggest that, in addition to current models where ERBB2 acts primarily by disrupting the transcriptional activity of ER, a significant fraction of ERBB2's effects on breast tumours may involve ER-independent mechanisms of gene activation as well, which may collectively contribute to the clinically aggressive nature of the 'low-confidence' breast tumour subtype.

Thus, the present inventors have determined sets of genes ("multigene classifiers"), which may be used to classify a breast tumour sample as a "low confidence" tumour or a "high confidence" tumour. The inventors have determined for the first time that the "low confidence" group of tumours has significant medical implications with regard to prognosis and treatment.

For each of ER+ and ER-, the inventors have provided a number of genes that have altered expression levels between "high confidence" and "low confidence" tumours. These genes are identified in Table 2. The levels of expression of these perturbed genes can be used to discriminate between high confidence and low confidence tumours. A further set of genes, which have distinctive expression levels in low confidence tumours as compared to high confidence tumours, is identified in Table S4. Further sets of genes that have distinctive expression levels in low confidence tumours as compared to high confidence tumours, irrespective of the ER status of the tumour, are identified in Tables A1-A4. The following description will make use of the term "expression profile". This refers to the expression levels in a sample of a set of genes from a multigene classifier.

The expression levels will generally be represented numerically. The expression profile therefore will generally include a set of numbers, each number representing the expression level of a gene of a multigene classifier. The following description will make use of the term "a plurality of genes". This term refers to a subset of the genes from a multigene classifier. The subset may correspond to a sub-grouping of the multigene classifier e.g. upregulated genes in ER+ low confidence breast tumours. The content of the plurality of genes may vary across multigene classifiers and, for a particular multigene classifier. The term may mean all of the genes of a particular multigene classifier or a subset thereof.

Accordingly, at its most general, the present invention provides new diagnostic methods and assays for classifying, using a multigene classifier, a breast tumour sample as a high or low confidence sample. The invention further identifies multigene classifiers for use in classifying breast tumour samples and apparatus comprising a multigene classifier or a plurality of genes therefrom. The multigene classifiers for use in aspects of the invention are shown in Table S4a and Table S4b.

Table S4 lists the genes that exhibit significant differential transcriptional regulation between high confidence and low confidence tumours when examined on a global scale in each of ER+ and ER- tumours.

We describe a method of producing a nucleic acid expression profile for a breast tumour sample comprising the steps of

(a) isolating expression products from said breast tumour sample;
(b) identifying the expression levels of a plurality of genes selected from **Table S4**; and
(c) producing from the expression levels an expression profile for said breast tumour sample.

The tumour sample may be high confidence and/or low confidence. The tumour sample may be an ER+ high confidence breast tumour sample and/or ER+ low confidence breast tumour sample and/or ER- high confidence breast tumour sample and/or ER- low confidence breast tumour sample. Preferably, the ER status of the breast tumour sample is determined. The ER status of the breast tumour sample is preferably determined before step a) of the method. The ER status of the breast tumour sample may be determined using gene expression profiling as described in our co-pending application PCT/GB03/000755.

The genes of Table S4 are shown in subsets. In subset (a) are genes that showed significantly altered expression in ER+ high confidence samples compared to ER+ low confidence tumours. In the first part of Table S4(a) is a group of genes that are upregulated (Table S4(a) 'upregulated') in ER+ low confidence tumours compared to ER+ high confidence tumours. The second part of Table S4(a) shows a group of genes that are downregulated (Table S4(a) down-regulated)in ER+ low confidence tumours compared to ER+ high confidence tumours.

In part (b) of Table S4 are genes that show upregulated expression in ER- low confidence samples compared to ER-high confidence tumours.

The expression profile of the individual genes of the multi-gene classifier will differ slightly between independent samples. However, the inventors have realised that the expression profile of genes of the multigene classifiers provide a characteristic pattern of expression that recognisably differs between high confidence and low confidence tumours.

By creating a number of expression profiles of a multigene classifier from a number of known high and low confidence samples it is possible to create a library of profiles for both high confidence and low confidence samples. The greater the number of expression profiles, the easier it is to create a reliable characteristic expression profile standard (i.e. including statistical variation) that can be used as a control in a diagnostic assay. Thus, a standard profile may be one that is derived from a plurality of individual expression profiles and derived within statistical variation to represent either the high confidence-or low confidence sample profile.

Thus, the method may comprise the steps of

(a) isolating expression products from a breast tumour sample;

(b) contacting said expression products with a plurality of binding members capable of specifically and independently binding to expression products of a plurality of genes selected from **Table S4**, so as to create a first expression profile of a tumour sample from the expression levels of said plurality of genes;

(c) comparing the expression profile with an expression profile characteristic of a high confidence tumour and/or a low confidence breast tumour.

The expression levels of the plurality of genes are assessed to produce the expression profile. The expression levels may be assessed absolutely i.e. a measurement of the amount of an expressed product. The expression levels may be assessed relatively i.e. expression compared to some other factor, such as, but not limited to expression of another gene, or a mean/median/mode of expression of a group of genes (preferably a group of genes not included in the multigene classifier used in the method) in the sample or across a group of samples. For example, expression of a gene may be measured as a multiple or fraction of the average expression of a plurality of genes in the sample. The expression is preferably denoted as positive or negative to indicate an increase or decrease in expression relative to the average value.

The prediction strength is preferably measured using a statistical and/or probabilistic model. The model comprises Weighted Voting (WV) and / or Support Vector Machines. The prediction strength may be determined using Weighted Voting and Leave One Out Cross Validation (see examples). Low confidence may mean a prediction strength of magnitude less than, or equal to, 0.4, when calculated using 2-colour cDNA microarrays, for example those used for assessing the Stanford data set. Preferably, the range of prediction strength for a low confidence tumour is ≥ - 0.4, and preferably s 0.4. The prediction strength may be ≥ - 0.35, and preferably ≤ 0.35 for a low confidence tumour. The prediction strength may be ≥ - 0.3, and preferably ≤ 0.3 for a low confidence tumour.

Preferably, high confidence samples have a prediction strength of magnitude greater than 0.4. Preferably, the prediction strength of high confidence tumours is ≥ 0.4, and preferably ≤ - 0.4.

However, the cut-off value of prediction strength for high / low confidence tumours may vary on the dataset and/or array technology used. For example, in the Rosetta data set, assessed using 2 color oligonucleotide microarrays, high confidence tumours are those with a prediction strength of magnitude greater than 0.7. The high confidence samples preferably have a prediction strength of magnitude greater than 0.7. Therefore, the prediction strength may be ≥ - 0.7, and preferably ≤ 0.7 for a low confidence tumour. The prediction strength may be ≥ - 0.6, and preferably ≤ 0.6 for a low confidence tumour. The prediction strength may be ≥ - 0.5, and preferably ≤ 0.5 for a low confidence tumour. More preferably, the range of prediction strength for a low confidence tumour is ≥ - 0.4, and preferably ≤ 0.4.

When the prediction strengths in a breast tumour population are compared in both Stanford and Rosetta data sets, the boundaries between high and low confidence tumours are identifiable as the points at which the prediction strength of tumours in the data set begin to demonstrate qualitatively reduced prediction strengths (the 'cliff-points') from the majority of the prediction strengths in the tumour population. Although each dataset was analyzed independently, the proportions of low-confidence tumours for the independent Rosetta and Stanford data sets are similar.

A low-confidence tumour may therefore fall within the lowest 20% of the ER prediction strengths in a breast tumour population, and more preferably the lowest 15-19% of ER prediction strengths. A breast tumour population preferably comprises a minimum data set of at least 25, more preferably at least 25-30 tumours, more preferably at least 30 tumours, more preferably at least 50 tumours, more preferably at least 80 tumours and most preferably around 80-100 tumours.

The expression products are preferably mRNA, or cDNA made from said mRNA, or cDNA. Alternatively, the expression product could be an expressed polypeptide. Identification of the expression profile is preferably carried out using binding members capable of specifically identifying the expression products of the plurality of genes identified in Table S4. For example, if the expression products are cDNA then the binding members will be nucleic acid probes capable of specifically hybridising to the cDNA.

Preferably, either the expression product or the binding member will be labelled so that binding of the two components can be detected. The label is preferably chosen so as to be able to detect the relative levels/quantity and/or absolute levels/quantity of the expressed product so as to determine the expression profile based on the up-regulation or down-regulation of the individual genes of the multigene classifier. Generally, the binding members should be capable of not only detecting the presence of an expression product but its relative abundance (i.e. the amount of product available).

There are, however, a number of newer technologies that have recently emerged that utilize 'label-free' techniques for quantitation, for example, those produced by Xagros. The expression product and/or the binding member may be unlabelled. Binding to the binding member may be detected and/or quantitated by measuring the change in electrical resistance as a result of two primers docking onto a target expressed product and subsequent extension by polymerase.

The determination of the nucleic acid expression profile may be carried out within certain previously set parameters, to avoid false positives and false negatives. A computer may be used to determine the nucleic acid expression profile.

The computer may then be able to provide an expression profile standard characteristic of a low confidence or high confidence breast cell as discussed above. The determined expression profiles may then be used to classify breast tissue samples as a way of diagnosis.

We therefore describe an expression profile database comprising a plurality of gene expression profiles of high confidence and/or low confidence breast tumour samples wherein each gene expression profile is derived from a plurality of genes selected from **Table S4**, and wherein the database is retrievably held on a data carrier. Preferably, the expression profiles making up the database are produced by the method for producing a nucleic acid expression profile described above.

With the knowledge of the multigene classifiers, it is possible to devise many methods for determining the expression pattern or profile of the genes in a particular test sample. For example, the expressed nucleic acid (RNA, mRNA) can be isolated from the sample using standard molecular biological techniques. The expressed nucleic acid sequences corresponding to the said plurality of genes from the genetic identifiers given in **Table S4** can then be amplified using nucleic acid primers specific for the expressed sequences in a PCR. If the isolated expressed nucleic acid is mRNA, this can be converted into cDNA for the PCR reaction using standard methods.

The primers may conveniently introduce a label into the amplified nucleic acid so that it may be identified. Ideally, the label is able to indicate the relative quantity or proportion of nucleic acid sequences present after the amplification event, reflecting the relative quantity or proportion present in the original test sample. For example, if the label is fluorescent or radioactive, the intensity of the signal will indicate the relative quantity/proportion or even the absolute quantity, of the expressed sequences. The relative quantities or proportions of the expression products of each of the genetic identifiers will establish a particular expression profile for the test sample. By comparing this profile with known profiles or standard expression profiles, it is possible to determine whether the test sample was from normal breast tissue or malignant breast tissue. The primers and/or amplified nucleic acid may be unlabelled, as discussed above.

Alternatively, the expression pattern or profile can be determined using binding members capable of binding to the expression products of the genetic identifiers, e.g. mRNA, corresponding cDNA or expressed polypeptide. By labelling either the expression product or the binding member it is possible to identify the relative quantities or proportions of the expression products and determine the expression profile of the genetic identifiers. In this way the sample can be classified high confidence or low confidence by comparison of the expression profile with known profiles or standards. The binding members may be complementary nucleic acid sequences or specific antibodies. Microarray assays using such binding members are discussed in more detail below.

According to a 1st aspect of the present invention, we provide a method for classifying a breast tumour sample as low confidence or high confidence, the method comprising providing the expression profile of said breast tumour sample, wherein the expression profile comprises the expression level of the genes from Table S4a and/or the genes from Table S4b, and classifying the tumour as a high or low confidence tumour based on the expression profile.

The method may comprise determining the estrogen receptor (ER) status of the sample.

A method of the first aspect of the invention may comprise the steps of:

(a) obtaining expression products from a breast tumour sample obtained from a patient;
(b) determining the expression levels of a multi-gene classifier comprising the genes identified in Table S4a and/or the genes identified in Table S4b by contacting said expression products with binding members, each binding member being capable of specifically binding to an expression product of the plurality of genes; and
(c) identifying the presence of a low confidence breast tumour in said patient based on the expression levels.

The expression products may be cDNA and the binding members may be nucleic acid probes capable of specifically hybridising to the cDNA.

The expression products may be RNA or mRNA and the binding members may be nucleic acid primers capable of specifically hybridising to the RNA or mRNA and amplifying them in a PCR.

The expression products are polypeptides and the binding members may be antibody binding domains capable of binding specifically to the polypeptides.

Preferably the method further includes the step of determining the ER status of the tumour, preferably before providing the expression profile of the tumour.

The method may comprise comparing the binding profile of the expression products from the breast tumour sample under test with a database of other previously obtained profiles and/or a previously determined "standard" profile which is characteristic of the presence of low confidence tumour.

The method may be carried out by a computer. The computer may be programmed to report the statistical similarity between the profile under test and the standard profiles so that a classification may be made.

The step of classifying the breast tumour sample may comprise the use of Weighted Voting, Support Vector Machines and/or Hierarchical Clustering.

The step of classifying the breast tumour sample may comprise the use of statistical and/or probabilistic techniques, such as Weighted Voting (WV) (13), a supervised learning technique. In WV, binary classifications may be performed. The expression level of genes in the multigene classifier in the breast tumour sample is compared to the mean average level of expression of that gene across the different classes. The mean average may, for example, be calculated from expression profiles that have an assigned class, e.g. database of expression profiles of high and/or low confidence samples. Preferably, the profiles have an assigned ER status.

The difference between the expression level and the mean average gene expression across the classes is weighted and corresponds to a 'vote' for that gene for a particular class. For a particular tumour, the votes for all the genes are summed together for each class to create totals for each class. The tumour is assigned to the class having the highest number of votes. The margin of victory of the winning class can then be expressed as prediction strength.

The difference in expression level is weighted using a formula that includes mean and standard deviations of expression levels of the genes in each of the two classes. Generally, the mean and standard deviations for each class are calculated from expression profiles that have, or represent, a particular class of tumour e.g. high confidence and low confidence.

Additionally, or alternatively, step (c) may comprise the use of hierarchical clustering, particularly if the tumour sample has been assessed using a different array technology from the one used to assess the expression profiles with assigned classes, or standard profile(s) to which the sample expression profile is compared. The result of step (c) may be validated using an established leave-one-out cross validation (LOOCV) assay (see examples). Step (c) may be performed using a computer.

In Hierarchical Clustering, each expression profile can be represented as a vector that consists of n genes where (g1, g2..gn) represent the expression levels of the genes. Each vector is then compared with every other profile in the analysis, and the two vectors with the highest correlation to one another are paired together until as many profiles as possible in the analysis have been paired up.

There are many ways known in the art to calculate the correlation, such as the Pearson's correlation coefficient (28). In the next step, a composite vector is then derived from each pair (in average-linkage clustering this is usually the average of both profiles), and then the process of pairing is repeated. This continues until no more pairings are possible. The process is 'hierarchical' as one starts from the bottom (individual profiles) and builds up. Individual profiles build up to preferably two composite vectors, each vector representing a class (i.e. high confidence and low confidence). For a new sample of unknown class, the sample is clustered with the standard profiles/samples. The class of 'unknown' sample will be determined based on which cluster/vector it belongs to at the end of the iterative rounds of pairing.

The methods described here may be used to identify an aggressive breast tumour in a patient, for example by comparing the said tumour's expression profile to a profile that is characteristic of tumour class, preferably by comparing the tumour's expression profile to a profile characteristic of a high confidence and/or of a low confidence tumour. The method may further comprise the step of assigning a poor prognosis to the patient where the tumour has an expression profile characteristic of a low confidence tumour expression profile.

The prognosis may affect the course of treatment of the patient. After identifying the low confidence tumour, the patient may be treated using aggressive techniques to treat the low confidence tumour.

A poor prognosis includes significantly worse overall survival rate of the patient and/or significantly shorter time to distant metastasis than a patient with a high confidence tumour.

As mentioned above, the present inventors have identified several key genes which have a different expression pattern in low confidence breast tumours as opposed to high confidence breast tumours, i.e. they are able to distinguish high and low confidence classes of breast tumour.

The multigene classifier may comprise genes that are given in Table S4. By determining an expression profile of a test sample and comparing the expression profile to expression profiles characteristic of low and/or high confidence breast tumours (and/or analysing the expression profile using techniques such as Weighted Voting), it is possible to classify the sample as a low confidence or high confidence tumour, e.g. an increase or decrease in their expression, relative to a standard pattern or profile seen in high confidence samples.

The plurality of genes may comprise the genes of Table S4(a) and/or Table S4(b).

The plurality of genes may include all of the genes of Table S4(a).

The plurality of genes may be all, or substantially all, of the upregulated and/or downregulated genes from Table S4(a).

Genes from Table S4(a) include the upper portion of the upregulated group of genes and/or the upper portion of the downregulated group of genes. The upper protion is the upper half of the table or group, as the genes are ranked in order of significance in each group. Genes that show the most differential expression between high confidence and low confidence tumours appear in the upper portion in each group of Table S4(a), whereas those genes that are less differentially expressed appear in the lower portion.

The plurality of genes may be all, or substantially all, of the genes from Table S4(b). The plurality of genes may be all, or substantially all, of the genes from Table S4(b). The plurality of genes may include all of the genes of Table S4(b).

Genes from Table S4(b) include those from the upper portion of the Table. The upper portion is preferably the upper half of the table, as the genes are ranked in order of significance in each group. Genes that show the most differential expression between high confidence and low confidence tumours appear in the upper portion of Table S4 (b), whereas those genes that are less differentially expressed appear in the lower portion.

As discussed previously, those skilled in the art will appreciate that fewer of the most significant genes are required to produce a characteristic expression profile compared to the number of the least significant genes required to produce a characteristic expression profile.

The number and choice of said plurality of genes provide an expression signature that is capable of distinguishing between high confidence and low confidence tumours.

The plurality of genes includes upregulated and downregulated genes from Table S4(a) and/or Table S4(b).

The step of classifying the tumour may comprise assessing genes that have been upregulated in a low confidence tumour compared to a high confidence tumour.

Additionally or alternatively, step (c) may comprise assessing genes that have been downregulated in a low confidence tumour compared to a high confidence tumour.

Genes that make up a further multigene classifier are shown in Table 2.

There is provided, according to a 2$^{nd}$ aspect of the present invention, a method of producing a nucleic acid expression profile for a breast tumour sample comprising the steps of

(a) isolating expression products from said breast tumour sample;
(b) identifying the expression levels of a multi-gene classifier comprising the genes from Table S4a and/or Table S4b; and
(c) producing from the expression levels an expression profile.

The breast tumour sample may be any class of breast tumour, as discussed above. Preferably, the ER status of the breast tumour sample is determined, preferably before step (a).

The method may comprise the steps of (a) isolating expression products from a breast tumour sample; contacting said expression products with multi-gene classifier comprising binding members capable of specifically and independently binding to expression products of the genes of Table S4a and/or Table S4b so as to create a first expression profile of a tumour sample from the expression levels of said multi-gene classifier; (c) comparing the expression profile with an expression profile characteristic of a high confidence tumour and/or a low confidence breast tumour.

We further describe an expression profile database comprising a plurality of gene expression profiles of high confidence and/or low confidence breast samples wherein each expression profile is derived from a plurality of genes from **Table 2**, and wherein the database is retrievably held on a data carrier. Preferably, the expression profiles making up the database are produced by the method described above.

The genes of Table 2 provide an alternative multigene classifier.

We describe a method for classifying a breast tumour sample as either low confidence or high confidence, the method comprising providing the expression profile of said sample, wherein the expression profile comprises the expression levels of a plurality of genes from Table 2, and classifying the tumour as a high or low confidence tumour based on the expression profile.

The method may comprise the steps of:

(a) obtaining expression products from a breast tumour sample obtained from a patient;
(b) determining the expression levels of a plurality of genes identified in **Table 2** by contacting said expression products with binding members, each binding member being capable of specifically binding to an expression product of the plurality of genes; and
(c) identifying the presence of a low confidence breast tumour in said patient based on the expression levels.

Step (c) may comprise comparing the binding profile to the profile characteristic of a low confidence tumour. The low confidence tumour may be ER+ or ER-. Step (c) may comprise the use of a statistical technique, such as Weighted Voting and/or Support Vector Machines (SVM).

The plurality of genes may comprise, or consist of, all, or substantially all, of the genes from Table 2, or all, or substantially all of the genes from either Table 2a or Table 2b.

The plurality of genes may include at least 10, 20, 30, 40, 50, 60, 70, 80, 90 or all of the genes of Table 2.

Preferably, the plurality of genes comprises, or consists of, about fifty or about forty or about thirty or about twenty or about ten genes from Table 2a and/or from Table 2b. Genes from Table 2 are preferably selected from the upper portion, preferably the upper half, of Table 2a and/or of Table 2b, as the genes are ranked in order of significance in each of Tables 2a and 2b. Genes that show the most perturbation between high confidence and low confidence tumours appear in the upper portion in each of Table 2a and Table 2b, whereas those genes that are less perturbed appear in the lower portion.

Those skilled in the art will appreciate that fewer of the most significant genes are required to produce an expression profile characteristic of a low and/or high confidence breast tumour compared to the number of the least significant genes required to produce a said characteristic expression profile. For example, fewer genes are required from the upper half of Table 2a than genes selected from the lower half of the Table.

The number and choice of said plurality of genes are selected so as to provide an expression signature that is capable of distinguishing between high confidence and low confidence tumours.

The plurality of genes may include no more than fifty genes of Table 2a and/or of Table 2b. The plurality of genes may include no more than forty genes of Table 2a and/or of Table 2b. The plurality of genes may include no more than thirty genes of Table 2a and/or of Table 2b. The plurality of genes may include no more than twenty genes of Table 2a and/or of Table 2b. The plurality of genes may include no more than ten genes of Table 2a and/or of Table 2b. The plurality of genes may include no more than five genes of Table 2a and/or of Table 2b.

The plurality of genes may comprise, or consist essentially of, five to fifty genes of Table 2a and/or of Table 2b. The plurality of genes may comprise, or consist essentially of, ten to forty genes of Table 2a and/or of Table 2b. The plurality of genes may comprise, or consist essentially of, ten to thirty genes of Table 2a and/or of Table 2b. The plurality of genes may comprise, or consist essentially of, ten to twenty genes of Table 2a and/or of Table 2b, or twenty to thirty genes of Table 2a and/or of Table 2b.

The said genes, preferably about ten genes, may be selected from the first about forty, or about thirty, or about twenty genes of Table 2a. The about ten genes may be selected from the first about fifteen genes of Table 2a. The about ten genes may be the first ten genes of Table 2a. The said genes, preferably about ten genes, may be selected from the first about forty, or about thirty, or about twenty, genes of Table 2b. The about ten genes may be selected from the first about fifteen genes of Table 2b. The about ten genes may be first ten genes of Table 2b.

The said genes, preferably about ten to twenty genes, are preferably selected from the first about thirty genes of Table 2a and/or Table 2b.

The plurality of genes may comprise, or consist of, about thirty or about twenty or about ten genes selected from the group consisting of the first about twenty genes of Table 2a and the first about twenty genes of Table 2b. The plurality of genes may comprise, or consist of, about ten or about fifteen or about twenty genes selected from the group consisting of the first about ten genes of Table 2a and the first about ten genes of Table 2b.

The methods preferably further comprise the preclassification step of determining ER+ or ER- status. The ER status may be determined by immunohistochemistry (e.g. using antibodies to ER) or by using a probabilistic / statistical model that is adapted to assess gene expression profiles.

The inventors have conducted further analyses and identified further multi-gene classifiers for discriminating between high and low confidence tumours. The objective of these analyses was to identify an optimal set of genes that could be used to classify "high" and "low-confidence" tumours regardless of their ER status. A series of three independent analytical methods (Significance Analysis of Microarrays, Gene Ranking, and The Wilcoxon Test) were used to identify genes that were differentially expressed between the two groups (LC and HC). The results of the analyses are the further multigene classifiers shown in Tables A1, A2, A3 and A4.

In Table A1, there are 88 genes that can be used to discriminate between high and low confidence tumours. Table A1 genes were identified using SAM (Significance Analysis of Microarrays). 86 of the genes are upregulated in low confidence tumours, whilst 2 of the genes are upregulated in high confidence tumours.

In Table A2, there are 251 genes that can be used to discriminate between high and low confidence tumours. Table A2 genes were identified using GR (Gene Ranking) by SVM.

In Table A3, there are 38 genes that can be used to discriminate between high and low confidence tumours. Table A3 genes were identified using a WT (Wilcoxon Test) at a P-value of <0.05 and a >=2-fold change cutoff.

In Table A4, there are 13 common genes (i.e. genes that are found in Tables A1, A2, A3). These 13 'common genes' are robust significant markers and can achieve comparable discriminatory performance as other 'complete' marker sets.

We further describe a method of producing a nucleic acid expression profile for a breast tumour sample comprising the steps of:

(a) isolating expression products from said breast tumour sample;
(b) identifying the expression levels of a plurality of genes from **Table A4 and/or Table A1 and/or Table A2 and/or Table A3**; and
(c) producing from the expression levels an expression profile.

The breast tumour sample may be any class of breast tumour, as discussed above.

We provide an expression profile database comprising a plurality of gene expression profiles of high confidence and/or low confidence breast samples wherein each expression profile is derived from a plurality of genes from **Table A4 and/or Table A1 and/or Table A2 and/or Table A3**, and wherein the database is retrievably held on a data carrier. Preferably, the expression profiles making up the database are produced by the method described above.

We describe a method for classifying a breast tumour sample as either low confidence or high confidence, the method comprising providing the expression profile of said sample, wherein the expression profile comprises the expression levels of a plurality of genes from Table A4 and/or Table A1 and/or Table A2 and/or Table A3, and classifying the tumour as a high or low confidence tumour based on the expression profile.

The method may comprise the steps of:

(a) obtaining expression products from a breast tumour sample obtained from a patient;
(b) determining the expression levels of a plurality of genes identified in **Table A4 and/or Table A1 and/or Table A2 and/or Table A3** by contacting said expression products with binding members, each binding member being capable of specifically binding to an expression product of the plurality of genes; and
(c) identifying the presence of a low confidence breast tumour in said patient based on the expression levels.

Step (c) may comprise deriving comparing the expression levels to a profile characteristic of a low and/or high confidence tumour. The low confidence tumour may be ER+ or ER-. Step (c) may comprise the use of a statistical technique, such as Weighted Voting and/or Support Vector Machines (SVM).

The plurality of genes preferably comprises, or consists essentially of, substantially all of the genes of Table A4. Further genes from each of Tables A1, A2 and A3 may be included, although, independently, the plurality of genes may be from any one or more of Tables A1, A2, and A3. The plurality of genes does not necessarily need to include the genes of Table A4.

The plurality of genes may be from any one or more of Table A1 and Table A2 and Table A3. The embodiments and preferred / optional features apply *mutatis mutandis* to Tables A1, A2, A3 and A4.

The plurality of genes may include at least 10, 20, 30, 40, 50, 60, 70, 80, or all of the genes of Table A1.

The plurality of genes may be all, or substantially all, of the 'upregulated in low confidence' and/or 'upregulated in high confidence genes' from Table A1. The plurality of genes may comprise, or consist of, about eighty, or about seventy, or about sixty, or about fifty, or about forty, or about thirty, or about twenty, or about ten, or about five of the 'upregulated in low confidence' genes from Table A1. The plurality of genes may include either one or both of the 'upregulated in high confidence' genes from Table A1.

Genes from Table A1 are preferably selected from the upper portion of the 'upregulated in low confidence' group of genes. The upper portion is preferably the upper half of the Table, as the genes are ranked in order of significance. Genes that show the most differential expression between high confidence and low confidence tumours appear in the upper portion of Table A1, whereas those genes that are less differentially expressed appear in the lower portion.

The plurality of genes may include no more than eighty, or seventy, or sixty, or fifty, or forty, or thirty, or twenty, or ten, or five genes of Table A1.

The plurality of genes may comprise, or consist essentially of, five to seventy genes of Table A1. The plurality of genes may comprise, or consist essentially of, ten to sixty genes of Table A1. The plurality of genes may comprise, or consist essentially of, ten to fifty, or ten to forty, or ten to thirty genes of Table A1.

The plurality of genes, which may be about ten to fifteen genes, may be selected from the first about forty, or about thirty, or about twenty genes of Table A1. Preferably, the plurality of genes comprises about ten to twenty genes of the first about thirty genes of Table A1.

The plurality of genes may include at least 10, 20, 30, 40, 50, 60, 70, 80, 90, 100, 110, 120, 130, 140, 150 or all of the genes of Table A2.

The plurality of genes may include no more than 250, or 240, or 230, or 220, or 210, or 200, or 190, or 180, or 170, or 160, or 150, or 140, or 130, or 120, or 110, or 100, or 90, or 80, or 70, or 60, or 50, or 40, or 30, or 20, or 10, or 5 genes of Table A2.

The plurality of genes may comprise, or consist essentially of, 5 to 200 genes of Table A2. The plurality of genes may comprise, or consist essentially of, 10 to 150 genes of Table A2. The plurality of genes may comprise, or consist essentially of, 10 to 100, or 10 to 70, or 10 to 50 genes of Table A2.

The plurality of genes, which may be about ten to fifteen genes, may be selected from the first about fifty, or about forty, or about thirty, or about twenty genes of Table A2. Preferably, the plurality of genes comprises about ten to twenty genes of the first about thirty genes of Table A2.

The plurality of genes may include at least 10, 20, 30, 35, or all of the genes of Table A3.

The plurality of genes may include no more than 35, or 30, or 20, or 10, or 5 genes of Table A3.

The plurality of genes may comprise, or consist essentially of, 5 to 35 genes of Table A3. The plurality of genes may comprise, or consist essentially of, 10 to 30 genes of Table A3. The plurality of genes may comprise, or consist essentially of, 10 to 20, or 20 to 30 genes of Table A3.

The plurality of genes, which may be about ten to fifteen genes, may be selected from the first thirty, or about twenty genes of Table A3. Preferably, the plurality of genes comprises about ten to twenty genes of the first about thirty genes of Table A3.

The plurality of genes may include at least 5, 10, 15 or all of the genes of Table A4.

The plurality of genes may include no more than 10, or 8, or 6, or 5 genes of Table A4.

The plurality of genes may comprise, or consist essentially of, 5 to 13 genes of Table A4. The plurality of genes may comprise, or consist essentially of, 10 to 13 genes of Table A4.

In the context of the plurality of genes, the term 'about' means the number of genes stated plus or minus the greater of: 10% of the number of genes stated or one gene.

As before, the expression product may be a transcribed nucleic acid sequence or the expressed polypeptide. The transcribed nucleic acid sequence may be RNA or mRNA. The expression product may also be cDNA produced from said mRNA. The expression product may be cRNA.

The binding member may a complementary nucleic acid sequence which is capable of specifically binding to the transcribed nucleic acid under suitable hybridisation conditions. Typically, cDNA or oligonucleotide sequences are used.

Where the expression product is the expressed protein, the binding member is preferably an antibody, or molecule comprising an antibody binding domain, specific for said expressed polypeptide.

The binding member may be labelled for detection purposes using standard procedures known in the art. Alternatively, the expression products may be labelled following isolation from the sample under test. A preferred means of detection is using a fluorescent label which can be detected by a light meter. Alternative means of detection include electrical signalling. For example, the Motorola e-sensor system has two probes, a "capture probe" which is freely floating, and a "signalling probe" which is attached to a solid surface which doubles as an electrode surface. Both probes function as binding members to the expression product. When binding occurs, both probes are brought into close proximity with each other resulting in the creation of an electrical signal which can be detected.

As discussed above, the binding members may be oligonucleotide primers for use in a PCR (e.g. multi-plexed PCR) to specifically amplify the number of expressed products of the genetic identifiers. The products would then be analysed on a gel. However, preferably, the binding member a single nucleic acid probe or antibody fixed to a solid support. The expression products may then be passed over the solid support, thereby bringing them into contact with the binding member. The solid support may be a glass surface, e.g. a microscope slide; beads (Lynx); or fibre-optics. In the case of beads, each binding member may be fixed to an individual bead and they are then contacted with the expression products in solution.

Various methods exist in the art for determining expression profiles for particular gene sets and these can be applied to the methods and compositions described here. For example, bead-based approaches (Lynx) or molecular bar-codes (Surromed) are known techniques. In these cases, each binding member is attached to a bead or "bar-code" that is individually readable and free-floating to ease contact with the expression products. The binding of the binding members to the expression products (targets) is achieved in solution, after which the tagged beads or bar-codes are passed through a device (e.g. a flow-cytometer) and read.

A further known method of determining expression profiles is instrumentation developed by Illumina, namely, fibre-optics. In this case, each binding member is attached to a specific "address" at the end of a fibre-optic cable. Binding of the expression product to the binding member may induce a fluorescent change which is readable by a device at the other end of the fibre-optic cable.

The present inventors have successfully used a nucleic acid microarray comprising a plurality of nucleic acid sequences fixed to a solid support. By passing nucleic acid sequences representing expressed genes e.g. cDNA, over the microarray, they were able to create an binding profile characteristic of the expression products from tumour samples and normal cells derived from breast tissue.

We provide, according to a third aspect of the present invention, an apparatus for classifying a breast tumour sample as "high confidence" or "low confidence", comprising a plurality of binding members attached to a solid support, each binding member being capable of specifically binding to expression product of a multi-gene classifier comprising the genes from Table S4a and/or Table S4b, and wherein the solid support houses binding members for no more than 500 different genes.

The apparatus may comprise a microarray wherein the binding members are nucleic acid sequences capable of specifically hybridising to RNA or mRNA expression products, or cDNA derived therefrom.

We further describe apparatus, preferably a microarray, for classifying a breast tumour sample comprising a plurality of binding members attached to a solid support, preferably nucleic acid sequences, each binding member being capable of specifically binding to an expression product of a gene from any one or more of the group of multigene classifiers: **Table S4**, Table 2, Table A1, Table A2, Table A3 and Table A4. Preferably the apparatus comprises, or consists essentially of, binding members capable of binding to expression products of a plurality of genes, as previously defined for each of the said multigene classifiers (see above). The apparatus may comprise, or consist essentially of, binding members capable of binding to expression products of a plurality of genes from each of the multigene classifiers, or of a plurality of genes from one or more of the multigene classifiers.

The apparatus apparatus may include binding members capable of specifically binding to expression products from at least 5 genes, more preferably, at least 10 genes or at least 15 genes from a said multigene classifier or from a subset of a said multi-gene classifier. A subset of a said multi-gene classifier may be, for example, genes from ER+/Low vs. ER+/High in **Table 2**, or genes from the upregulated group in ER+/Low from **Table S4(a)**. In a most preferred embodiment, the solid support will house binding members being capable of specifically and independently binding to expression products of all genes identified in Table A4.

The apparatus preferably includes binding members capable of specifically binding to expression products from a multigene classifier, or to a plurality of genes thereof, and may include binding members capable of specifically binding to expression products of no more than 14396 of the genes on the U133A microarray. The apparatus may include binding members capable of specifically binding to expression products of no more than 90% of the genes on the U133A microarray. The apparatus may include binding members capable of specifically binding to expression products of no more than 80% or 70% or 50% or 40% or 30% or 20% or 10% or 5% of the genes on the U133A microarray.

Additionally or alternatively, the solid support may house binding members for no more than 14000, no more than 10000, no more than 5000, no more than 3000, no more than 1000, no more than 500, or no more than 400, or no more than 300, or no more than 200, or no more than 100, or no more than 90, or no more than 80, or no more than 70, or no more than 60, or no more than 50, or no more than 40, or no more than 30, or no more than 20, or no more than 10, or no more than 5 different genes.

Typically, high density nucleic acid sequences, usually cDNA or oligonucleotides, are fixed onto very small, discrete areas or spots of a solid support. The solid support is often a microscopic glass side or a membrane filter, coated with a substrate (or chips). The nucleic acid sequences are delivered (or printed), usually by a robotic system, onto the coated solid support and then immobilized or fixed to the support.

In a preferred embodiment, the expression products derived from the sample are labelled, typically using a fluorescent label, and then contacted with the immobilized nucleic acid sequences. Following hybridization, the fluorescent markers are detected using a detector, such as a high resolution laser scanner. In an alternative method, the expression products could be tagged with a non-fluorescent label, e.g. biotin. After hybridisation, the microarray could then be 'stained' with a fluorescent dye that binds/bonds to the first non-fluorescent label (e.g. fluorescently labelled strepavidin, which binds to biotin).

A binding profile indicating a pattern of gene expression (expression pattern or profile) is obtained by analysing the signal emitted from each discrete spot with digital imaging software. The pattern of gene expression of the experimental sample can then be compared with that of a control (i.e. an expression profile from a high confidence or low confidence sample) for differential analysis.

As mentioned above, the control or standard, may be one or more expression profiles previously judged to be characteristic of normal or malignant cells. These one or more expression profiles may be retrievable stored on a data carrier as part of a database. This is discussed above. However, it is also possible to introduce a control into the assay procedure. In other words, the test sample may be "spiked" with one or more "synthetic tumour" or "synthetic normal" expression products which can act as controls to be compared with the expression levels of the genetic identifiers in the test sample.

Most microarrays utilize either one or two fluorophores. For two-colour arrays, the most commonly used fluorophores are Cy3 (green channel excitation) and Cy5 (red channel excitation). The object of the microarray image analysis is to extract hybridization signals from each expression product. For one-color arrays, signals are measured as absolute intensities for a given target (essentially for arrays hybridized to a single sample). For two-colour arrays, signals are measured as ratios of two expression products, (e.g. sample and control (controls are otherwise known as a 'reference')) with different fluorescent labels.

The apparatus (e.g. microarray) preferably comprises a plurality of discrete spots, each spot containing one or more oligonucleotides and each spot representing a different binding member for an expression product of a gene selected from a said multigene classifier. In one embodiment, the microarray will contain spots for each of the genes provided in one or more of the multigene classifiers. Each spot will comprise a plurality of identical oligonucleotides each capable of binding to an expression product, e.g. mRNA or cDNA, of the gene of **Table S4** it is representing.

As a 4$^{th}$ aspect of the present invention, there is provided a kit for classifying a breast tumour sample as "high' confidence" or "low confidence", said kit comprising a plurality of binding members, each binding member being capable of specifically binding to an expression product of one of a multi-gene classifier comprising the genes identified in Table S4a and/or Table S4b and a detection reagent, wherein the binding members are antibody binding domains.

The kit may further comprise one or more standard expression profiles retrievably held on a data carrier for comparison with expression profiles of a test sample.

The kit may be such that the one or more standard expression profiles are produced according to a method as set out above.

We describe a kit for classifying a breast tumour sample as high confidence or low confidence, said kit comprising binding members, each binding member being capable of specifically binding to an expression product of a plurality of genes identified in a said multigene classifier, and a detection reagent.

The genes of the multigene classifiers are listed with their Unigene accession numbers (corresponding to build 160 of Unigene). The sequence of each gene can therefore be retrieved from the Unigene database. Furthermore, for certain of the genes, Affymetrix (www.affymetrix.com) provide examples of probe sets, including the sequences of the probes, (i.e. binding members in the form of oligonucleotide sequences) which are capable of detecting expression of the gene when used on a solid support. The probe details are accessible from the U133 section of the Affymetrix website using the Unigene ID of the target gene.

If, in the future, one of the Unigene ID's listed in the table were to be merged into a new ID, or split into two or more ID's (e.g. in a new build of the database) or deleted altogether, the sequence of the gene, as intended by the present inventors, is retrievable by accessing build 160 of Unigene.

Preferably, the one or more binding members (antibody binding domains or nucleic acid sequences e.g. oligonucleotides) in the kit are fixed to one or more solid supports e.g. a single support for microarray or fibre-optic assays, or multiple supports such as beads. The detection means is preferably a label (radioactive or dye, e.g. fluorescent) for labelling the expression products of the sample under test. The kit may also comprise means for detecting and analysing the binding profile of the expression products under test.

Alternatively, the binding members may be nucleotide primers capable of binding to the expression products, such that they can be amplified in a PCR. The primers may further comprise detection means, i.e. labels that can be used to identify the amplified sequences and their abundance relative to other amplified sequences.

The kit may also comprise one or more standard expression profiles retrievably held on a data carrier for comparison with expression profiles of a test sample. The one or more standard expression profiles may be produced according to the methods described in this document.

The breast tissue sample may be obtained as excisional breast biopsies or fine-needle aspirates.

Again, the expression products are preferably mRNA or cDNA produced from said mRNA or cRNA. The binding members are preferably oligonucleotides fixed to one or more solid supports in the form of a microarray or beads (see above). The binding profile is preferably analysed by a detector capable of detecting the label used to label the expression products. The determination of the presence or risk of breast cancer can be made by comparing the binding profile of the sample with that of a control e.g. standard expression profiles.

It is preferred to use binding members capable of specifically binding (and, in the case of nucleic acid primers, amplifying) expression products of a said multigene classifier. This is because the expression levels of all genes make up the expression profile specific for the sample under test. The classification of the expression profile is more reliable the greater number of gene expression levels tested. Thus, preferably expression levels of more than 5 genes selected from one or more of said multi-gene classifiers are assessed, more preferably, more than 10, more than 20, more than 30, even more preferably, more than 40 and preferably all genes from a said multi-gene classifier. For example, the binding members may be capable of binding to expression products from all of the genes of Table S4, or a plurality of genes therefrom, as previously defined.

The known microarray and genechip technologies allow large numbers of binding members to be utilized. Therefore, the more preferred method would be to use binding members representing all of the genes in a said multigene classifier, or a plurality of genes therefrom, as previously defined for each multigene classifier. However, the skilled person will appreciate that a proportion of these genes may be omitted and the method still carried out in a reliable and statistically accurate fashion. In most cases, it would be preferable to use binding members representing at least 70%, 80% or 90% of the genes in a said multigene classifier. In this context, a multigene classifier preferably means the genes of Table S4 or a subset or group of a said Table. The multigene classifier may be the genes of **Table A4**.

Therefore, plurality may mean at least 50%, more preferably at least 70% and even more preferably at least 90% of the multigene classifier as mentioned above.

The provision of the genetic identifier allows diagnostic tools, e.g. nucleic acid microarrays to be custom made and used to predict, diagnose or subtype tumours. Further, such diagnostic tools may be used in conjunction with a computer which is programmed to determine the expression profile obtained using the diagnostic tool (e.g. microarray) and compare it to a "standard" expression profile characteristic of high confidence tumour v low confidence tumour. In doing so, the computer not only provides the user with information which may be used classifying the type of a tumour in a patient, but at the same time, the computer obtains a further expression profile by which to determine the "standard" expression profile and so can update its own database.

Thus, our methods and compositions allow, for the first time, specialized chips (microarrays) to be made containing probes corresponding to the said multigene classifiers, or a plurality of genes therefrom. The exact physical structure of the array may vary and range from oligonucleotide probes attached to a 2-dimensional solid substrate to free-floating probes which have been individually "tagged" with a unique label, e.g. "bar code".

A database corresponding to the various biological classifications (e.g. high confidence or low confidence $ER^+/ER^-$) may be created which will consist of the expression profiles of various breast tissues as determined by the specialized microarrays. The database may then be processed and analysed such that it will eventually contain (i) the numerical data corresponding to each expression profile in the database, (ii) a "standard" profile which functions as the canonical profile for that particular classification; and (iii) data representing the observed statistical variation of the individual profiles to the "standard" profile.

In one embodiment, to evaluate a patient's sample, the expression products of that patient's breast sample (obtained via excisional biopsy or find needle aspirate) will first be isolated, and the expression profile of that sample determined using the specialized microarray. To classify the patient's sample, the expression profile of the patient's sample will be queried against the database described above. Querying can be done in a direct or indirect manner. The "direct" manner is where the patient's expression profile is directly compared to other individual expression profiles in the database to determined which profile (and hence which classification) delivers the best match. Alternatively, the querying may be done more "indirectly", for example, the patient expression profile could be compared against simply the "standard" profile in the database. The advantage of the indirect approach is that the "standard" profiles, because they represent the aggregate of many individual profiles, will be much less data intensive and may be stored on a relatively inexpensive computer system which may then form part of the kit (i.e. in association with the microarrays). In the direct approach, it is likely that the data carrier will be of a much larger scale (e.g. a computer server), as many individual profiles will have to be stored.

By comparing the patient expression profile to the standard profile (indirect approach) and the pre-determined statistical variation in the population, it will also be possible to deliver a "confidence value" as to how closely the patient expression profile matches the "standard" canonical profile for high or low confidence tumours. This value will provide the clinician with valuable information on the trustworthiness of the classification, and, for example, whether or not the analysis should be repeated.

As mentioned above, it is also possible to store the patient expression profiles on the database, and these may be used at any time to update the database.

Aspects and embodiments of the present invention will now be illustrated, by way of example, with reference to the accompanying figures. Further aspects and embodiments will be apparent to those skilled in the art. All documents mentioned in this text are incorporated herein by reference.

## Brief Description of the Drawings

**Figure 1**. Identification of Tumours with Low Prediction Strength ("Low-confidence").
Each sample in the training (a) and test set (b) is plotted (x-axis) against the sample's prediction strength (PS, y-axis). The training data set consists of 55 tumours and the test data set consists of 41 tumours. Samples exhibiting high positive PS values are classified as ER+, while samples with a high negative PS are ER-. Blue samples were correctly classified while red samples were misclassified. In general, a group of 'low-confidence' samples is observed (grey box) in both the training and test tumours.

**Figure 2**. Kaplan-Meier analysis comparing the clinical behaviour of 'high' and 'low -confidence' tumours.
Overall survival data in (a) and (b) is obtained from Stanford data set (9), while Time to Distance Metastasis data in (c) and (d) is obtained from Rosetta data set (10). Patients with 'high-confidence' tumours are depicted as green, while patients with 'low-confidence' tumours are depicted in pink. a) Overall survival of patients with 'high' (60 patients) and 'low-confidence' (14 patients) tumours regardless of ER status, b) Overall survival of patients with ER+ 'high' (48) and 'low-confidence' (7) tumours; c) Time from initial tumour diagnosis to appearance of distant metastasis of patients with 'high' (82) and 'low-confidence' (15) tumours regardless of ER status, (d) Time from initial tumour diagnosis to appearance of distant metastasis of patients with ER+ 'high' (63) and 'low-confidence' (5) tumours.

**Figure 3.** Widespread perturbations in ER-correlated genes in low Vs high confidence samples.
(a) and (b) Depicted are the relative expression levels of the top 122 ER discriminating genes (obtained from the SAM-133 gene set, see text) that are positively correlated to ER+ status in (a) ER+/High (yellow) and ER+/Low (turquoise), and (b) ER- /High (dark blue) and ER-/Low (pink) samples. The order of the 122 genes along the x axis is determined by their S2N ratio (see Materials and Methods). The S2N metric for a particular gene takes into account both the difference in mean expression level between two classes, as well as the standard deviation in expression for that gene within each class being compared. Note that the specific order of the 122 genes in (a) and (b) are different, depending on their S2N ratio (Table 2). (c) and (d) depicted are the relative expression levels of the top 54 ER discriminating genes that are negatively correlated to ER+ status (11 belonging to the SAM-133 gene set, see supplementary info for details) in (c) ER/High (yellow) and ER+/Low (turquoise), and (d) ER-/High (dark blue) and ER-/Low (pink) samples. There are considerably less perturbations observed than in (a) and (b) .

**Figure 4**. ERBB2+ is associated with 'low-confidence' prediction across multiple breast cancer expression datasets. Data is taken from ref. 3. a) Identification of tumour samples (columns) expressing high levels of ERBB2 and other genes (MLN64, GRB7) physically linked to the 17q ERBB2 chromosomal locus (rows). High expression is represented by a red square. Tumour samples 5141,8443,7636,4527, 5955,10444, 5985,6936 exhibit high expression of ERBB2 and ERBB2-linked genes, while 6080 and 10188 exhibit elevated but weaker expression. b) Summary of ANN models for ER classification (adapted from Figure 1b in ref. 3). Tumour samples classified as ER+ are blue while ER- tumours are orange. Prediction confidence is represented by each sample's standard deviation (SD), with 'low confidence' samples having a high SD. The eight 'highly expressing' ERBB2 +ve samples are depicted (ERBB2 at the left or right of the sample SD). Note that tumour samples with high SDs tend to be ERBB2 +ve.

**Figure 5.** Principle component analysis (PCA), a mathematical technique that provides a projection of complex data sets onto a reduced, easily visualized space, provides a useful visual assessment of how clearly the samples are discriminated on the basis of the SAM-133 gene set. ER+ and ER- tumours are clearly distinguishable from one another, while ERBB2+ samples lie in the intermediate space. Color-coding scheme: ER+ERBB2-, yellow; ER+ERBB2-, turquoise; ER-ERBB2+, blue; and ER-ERBB2+, pink. Color-coding scheme: ER+ ERBB2-, yellow; ER+ ERBB2+, turquoise; ER- ERBB2-, blue; and ER- ERBB2+, pink. X-axis is principle component 1 and Y-axis is component 2. Samples that lie at the left of the red line are ER+ except two ER- samples; while the samples on the right are ER- samples except one misclassification. Samples close to the boundary (in the square) are all ERBB2+.

Figure 6 shows the clinical prognoses of patients with 'high-confidence' ER negative tumours to those patients harboring 'low-confidence' ER negative tumours. Two independent data sets were analyzed, referred to as the 'Rosetta' and 'Stanford' data sets. Figure 6(a) shows Rosetta tumours: Relapse free survival was measured. 11/19 (58%) High-confidence patients developed distant metastasis within 5 years; while in Low-confidence ER- the number is 8/10. (80%). Figure 6(b) shows Stanford tumours: Overall survival was measured. 7/12 (58%) High-confidence patients are dead; while in Low-confidence ER- the number is 5/7 (71%).

**Figure 7 shows** identification of Tumors with Low Prediction Strength ("Low-confidence") in the Stanford and Rosetta Data Sets

## Results

### Classification of Breast Tumours by ER Status Using Expression Profiles from Chinese Patients Reveals a Distinct Population of 'Low Confidence' Samples

The overall incidence patterns of breast cancer in Caucasian and Asian populations are distinct (8), prompting the inventors to investigate if findings from previous reports (3,4) could also be observed in their local patient population. They first used gene expression profile data to classify a set of breast tumours by their ER status. A training set of 55 breast tumours was selected, where the ER status of each tumour was pre-determined using IHC. Two classification methods were tested: weighted-voting (WV) and support vector machines (SVM), and classification accuracy was assessed through leave-one-out cross validation (LOOCV) (Supplementary Information). In addition to classifying a sample, quantitative metrics were used to provide an assessment of classification uncertainty (Materials and Methods). The overall classification accuracy on the training set was 95% (WV) and 96% (SVM), with seven samples characterized by 'low confidence' or marginal predictions (grey box, Figure 1a). To determine if such 'low-confidence' samples could also be observed in an independent set of tumours, a second set of 41 tumours was used as an independent test set. Although the overall classification accuracy on the independent test set was 91% (WV and SVM), nine samples once again displayed a 'low-confidence' prediction (Figure 1b). Thus, using two different classification methods (WV and SVM), certain breast tumours were found to exhibit a distinct 'low-confidence' character when being classified by ER status on the basis of their gene expression profiles.

### Patients with 'Low-Confidence' Tumours Exhibit Decreased Overall Survival and Shorter Time to Distant metastasis in Comparison to Patients with 'High confidence' Tumours

Since the differentiation of tumours into 'high' and 'low-confidence' sub-populations was achieved through a purely computational analysis of tumour gene expression profiles, it is unclear if this distinction is biologically or clinically meaningful, and if the use of gene expression profiles in this manner affords any substantial advantage over conventional immunohistochemical techniques to determine the ER status of breast tumours. To address this issue, the inventors investigated if the 'low-confidence' tumours might exhibit any clinical behaviors distinct from their 'high-confidence' counterparts. They used two publicly available breast cancer expression data sets for which related but distinct types of clinical information was available. The first set (9) consists of a cDNA microarray data set of 78 breast carcinomas and 7 nonmalignant samples with overall patient survival information (referred to as the Stanford data set). The second one (10) consists of 71 ER+ and 46 ER lymph- node negative tumours profiled using oligonucleotide-based microarrays, out of them 97 samples had the clinical information being the time interval from initial tumour diagnosis to the appearance of a new distant metastasis (referred to as the Rosetta dataset). The inventors used WV to classify the breast tumours in the Stanford and Rosetta datasets by their ER subtype. Consistent with their own data set, among the 56 ER + and 18 ER tumours in the Stanford data set (4 tumours were removed due to lack of ER status information), they observed an overall LOOCV accuracy of 93%, with 14 tumours being classified as 'low-confidence'. Similarly, the WV analysis also identified 15 tumours in the Rosetta data set as exhibiting a 'low-confidence' classification, with an overall LOOCV accuracy of 92%. These numbers are comparable to that observed in the inventors' own patient population.

They then compared the clinical behaviour of the 'high' and 'low-confidence' tumour populations using Kaplan-Meier analysis. As shown in Figure 2, patients with 'low-confidence' tumours exhibited a significantly worse overall survival (p=0.0003, log rank test) and shorter time to distant metastasis (p=0.0001, log-rank test) than their 'high confidence' counterparts. This result indicates that the 'high' vs 'low-confidence' binary distinction is indeed clinically meaningful. The inventors then repeated this analysis, but first subdividing the tumours into independent ER+ and ER- categories. For ER+ tumours, they once again found that 'low-confidence' ER+ tumours were associated with a significantly worse overall survival (p=0.03, log-rank test) and shorter time to metastasis (p=0.004, log-rank test) (Figure 2) than 'high-confidence' ER+ tumours. No statistically significant differences in overall survival and time to metastasis were observed for the ER- tumours. These results indicate that ER+ tumours can be subdivided on the basis of the 'high' and 'low-confidence' binary classification into distinct disease groups exhibiting different clinical behaviours. Since distinguishing between these two groups is currently not possible by conventional immunohistochemical methods used for ER detection, this result also demonstrates how gene expression profile data can be a useful adjunct to conventional strategies for breast cancer prognostication and staging.

**'Low-Confidence' Tumours Exhibit Widespread Perturbations in the Expression of Genes Important for ER Subtype Discrimination**

The classification algorithms used in these and other studies (e.g. WV, SVM, ANN, see below) all rely upon the combinatorial input of multiple discriminator genes whose individual contributions are then combined to arrive at a particular classification decision (i.e. if the tumour is ER+ or ER-). It is formally possible that the 'low-confidence' prediction status of these breast tumours is due to either the dramatic deregulation of a few key discriminator elements (i.e. specific effects), or the more subtle perturbation of a large number of discriminator genes (i.e. widespread effects). To distinguish between these two possibilities, the inventors compared the expression levels of genes important for ER subtype discrimination between 'high' and 'low' confidence tumours. First, to identify ER discriminating genes which where differentially regulated between ER+ and ER- tumours, they utilized a statistical technique called significance analysis of microarrays (SAM) (11).

Employing their combined dataset (total number = 96 tumours), a total of 133 differentially regulated genes (SAM-133) were identified at a 'false discovery rate' (FDR) of 0% (the FDR is an index used by SAM to estimate the number of false positives - an FDR of 10% for 100 genes indicates that 10 genes are likely to be false positives). In this set, 122 genes were up-regulated in ER+ samples (ie positively correlated to ER status), while the remaining 11 were down-regulated in ER+ tumours (ie negatively correlated to ER). As predicted, the SAM-133 gene set includes a number of genes related to the ER pathway, such as ESR1, LIV1 (an estrogen-inducible genes), and TFF1, and some genes (e.g. GATA-3) were identified multiple times. A number of genes in the SAM-133 list are also found in similar lists reported by others (3, 4).

The inventors then subdivided the ER+ and ER- tumours each into 'high' and 'low' confidence categories (ie ER+/High, ER+/Low, ER-/High, ER-/Low), and the expression levels of the SAM-133 genes were compared between the groups (Figure 3). Of the 122 genes in the SAM-133 gene set that were positively correlated to ER status, approximately 62% exhibited a significantly lower average expression level (referred as 'perturbed expression') in the ER+/Low samples compared to the ER+/High tumours (p<0.05, Figure 3a and Table 2). Genes with 'perturbed' expression included ER, GATA3, BCL2, IGF1R, and RARA, while other ER-discriminator genes, such as TFF1, TFF3 and XBP1 were unaffected. Similarly, in the ER- 'high' and 'low' confidence samples, the inventors witnessed a reciprocal pattern where approximately 42% of the 122 genes exhibited a higher average expression level in the ER-/Low samples compared to the ER-/High tumours (p<0.05, Figure 3b and Table 2). Intriguingly, although the expression levels of certain genes (e.g. GATA3, BCL2) were perturbed between 'low' and 'high' confidence samples in both the ER+ and ER- subtypes, the perturbation of other genes appeared to be subtype-specific. For example, ESR1 and IGFR1 were only perturbed in the ER+ samples, while XBP1 was only perturbed in the ER-samples. Finally, there were minimal changes in the expression levels of ER-discriminating genes that were negatively correlated to ER+ status (i.e. highly expressed in ER- tumours) (Figure 3c and d). This result suggests that the expression perturbations observed in the ’low-confidence’ samples, although widespread, are primarily observed in genes whose expression is positively correlated to ER (Supplementary Information).

**Elevated Expression of the ERBB2 Oncogene is Significantly Associated with the ’Low-Confidence’ Predictions**

The expression perturbations observed in the ’low-confidence’ breast tumours could be due to multiple reasons, ranging from experimental variation (e.g. poor sample quality, tumour excision and handling), choice of the classification method, to population and sample heterogeneity. To gain insights into the possible mechanisms underlying these expression perturbations, the inventors attempted to determine if there were any specific histopathological parameters that might be correlated to the ’low-confidence’ state. No significant associations were observed between the ’low-confidence’ status of a tumour and patient age, lymph node status, tumour grade, p53 mutation status or progesterone receptor status (Table 1). The inventors discovered, however, a significant positive association ($p<0.001$, Supplementary Information) between a tumours’ ERBB2 status and a ’low confidence’ prediction. This correlation, observed using the training set data, was then assessed using the independent test set samples. Of the nine ’low-confidence’ samples in the independent test set, eight tumours were also ERBB2+ (8/9), indicating that this association is not dataset-specific.

The inventors also investigated if the correlation between the ’low-confidence’ predictions with high ERBB2 expression could have been independently discovered by comparing the global expression profiles of ’high’ and ’low’ confidence tumours. First, they compared the ’high-confidence’ and ’low-confidence’ tumours belonging to the ER+ subtype. A total of 89 genes were identified as being significantly regulated (FDR= 14%). Among the top 50 most significantly up-regulated genes in the ER+ ’low-confidence’ samples, 3 genes - PMNT (ranked 4th), GRB7V (8th), and ERBB2 (36th) were of particular interest (Supplementary Information), as they are all physically located on the 17q region, a frequent target of DNA amplification in breast cancer (12). In a separate analysis, the ER- ’high-confidence’ and ER-’low-confidence’ samples were also compared. Among the top 50 genes identified as being differentially regulated (FDR = 4%), the inventors once again identified the 17q genes PMNT (ranked 5th), GRB7V (10th) and ERBB2 (28th) as exhibiting increased expression in the ’low-confidence’ samples (Supplementary Information). Taken collectively, these results suggest that for both the ER+ and ER- subtypes, the ’low-confidence’ breast tumours are significantly associated with increased expression of ERBB2 in comparison to the ’high confidence’ tumours, most likely resulting from DNA amplification of the 17q locus. However, please note that the association between ’low-confidence’ prediction and ERBB2+ expression, although highly significant, is not perfect, as a few tumours that were designated as ERBB2+ by conventional IHC exhibited ’high-confidence’ predictions, while not all ’low-confidence’ tumours are ERBB2+. One possibility may be that other genes, besides ERBB2, may also contribute to a breast tumour exhibiting a ’low-confidence’ state.

To validate their finding, the inventors then analyzed the other independently derived breast cancer expression datasets. First, of the nine ERBB2+ tumours in the Stanford data set, all nine were predicted as being in the ’low-confidence’ group ($p<0.001$, Supplementary Information). Second, in the Rosetta data set, they once again found a significant association between the confidence level of prediction and ERBB2 expression ($p<0.001$, Supplementary Information). Third, Gruvberger and his colleagues utilized artificial neural networks (ANNs) on a cDNA microarray data set of 28 ER+ and 30 ER- samples to predict the ER status of breast tumours (3). Their results, shown in Fig. 4b, depicts the output of the ANN model with sample standard deviations (SDs), as assessed using the top 100 discriminator genes for ER subtype. Samples with a wide SD are analogous to the ’low-confidence’ status of the WV and SVM methodologies. As can be seen from Figure 4b, ERBB2+ samples (determined in Figure 4a) tend to be associated with large SDs, which indicate high uncertainty, particularly for ER+ tumours. Taken collectively, the association between the confidence level of ER prediction and ERBB2 status was observed on a wide range of data sets originating from different laboratories utilizing different microarray technologies (Affymetrix, cDNA and oligonucleotide) on different patient populations (Asian, European/Caucasian), and predicted by different classification algorithms (WV, SVM, ANN). The commonality of these results on both the inventor data set and publicly available data sets suggests that the correlation between high ERBB2 expression to ’low-confidence’ prediction status may be an inherent feature of breast cancer in general.

**A Significant Proportion of Genes Perturbed in the Low Confidence Samples are Not Known to be Regulated by Estrogen and Lack Potential EREs in their Promoters**

The strong correlation between high ERBB2 levels and the widespread perturbations of ER-subtype discriminating genes observed in the ’low-confidence’ tumours raises the possibility that ERBB2 may be functionally contribute towards this phenomenon. One possible mechanism by which this could occur is through ERBB2 signaling which has been proposed to inhibit the transcriptional activity of ER (see Discussion). Under this scenario, one might expect that a significant proportion of the genes perturbed between the 'high-confidence' (ERBB2-) and 'low-confidence (ERBB2+) tumours would consist of genes regulated by ER. The inventors tested this hypothesis in two ways. First, they compared their list of significantly-perturbed genes (Table 2) to SAGE expression data derived from estrogen (E2) stimulated MCF-7 cells (13) to determine if the extent of overlap between the two. Only two genes (STC2, TFF1) were found in common between the SAGE data and the 'perturbed' gene list, and one (TFF1) was regulated in the opposite manner from that expected, exhibiting higher expression in the ERBB2+ samples. This result, within the limits of the cell line assay, suggests that many of the 'perturbed' genes in the 'low confidence' tumours may not be directly regulated by estrogen. Second, as in-vitro cell line studies may not fully recapitulate the effects of estrogen in vivo, the inventors then adopted a bioinformatics approach using a recently described algorithm, Dragon Estrogen Response Element Finder (DEREF), to search for putative estrogen-response elements (EREs) in the promoter regions of the perturbed genes (14). The prediction accuracy of DEREF has been validated in a number of *in vivo* examples - it detects ERE patterns 2.8x more frequently in the promoter regions of estrogen responsive versus non-responsive genes in a microarray experiment, and 5.4x more frequently in the promoters of genes belonging to the estrogen-induced SAGE dataset versus genes whose expression is negatively correlated to ER in breast cancers (Supplementary Information). Of the top 50 perturbed genes in the ER+ tumours (Table 2), the transcriptional start sites of 35 could be accurately determined and thus were subsequently analyzed by DEREF. Of this 35, EREs were detected with high-confidence in only 12 promoters (total frequency 34%) (Table 2) .

Conversely, of the top 50 perturbed genes in the ER-tumours, 33 were analyzed by DEREF and high-confidence EREs were detected in only 3 (total frequency 9%) (Table 2). Thus, EREs were detected in the promoters of perturbed genes in ER+ tumours at 3.7x higher frequency than in the ER-tumours. This difference was significant by a chi-square analysis (p=0.012), suggesting that ERBB2 may affect transcription in ER+ and ER tumours via distinct mechanisms (see Discussion). Regardless, EREs were not detected as over represented in the perturbed genes in both subtypes (ER+ and ER-), suggesting that these genes may not be direct transcriptional targets of ER. These genes may represent either indirect targets of ER, or may be transcriptionally regulated via ER-independent mechanisms.

**Definition of a Optimal Gene Set to Classify Low and High Confidence Tumours Irrespective of ER Subtype**

The objective of this analysis was to identify an optimal set of genes which could be used to classify "high" and "low-confidence" tumours regardless of their ER status.

**Details**

A total of 96 tumours were analyzed, of which 16 were LC and 80 were HC. A series of three independent analytical methods (SAM, GR, and WT, see below) were used to identify genes that were differently regulated between the two groups (LC and HC). The ability of these gene sets to classify the HC or LC status of a tumour was assessed by a leave-one-out cross validation assay using either Support Vector Machine or Weighted Voting as the classification algorithm.

**Results**

SAM (Significance Analysis of Microarrays): At a FDR (False-discovery rate) of <15%, a total of 86 up-regulated and 2 down-regulated genes in low-confidence tumours were identified. Using this gene set, the LOOCV assay produced a classification accuracy of 84%. The 88 genes are shown in Table A1.

GR (Gene Ranking by SVM): A total of 251 genes were identified with the ability to classify the HC or LC status of a tumour, with a classification accuracy of 86%. The 251 genes are shown in Table A2.

WT (Wilcoxon Test): At a P-value of <0.05 and a >=2-fold change cutoff, a total of 38 genes were identified. This 38 gene set delivered a LOOCV accuracy of 80%. The 38 genes are shown in Table A3.

13 'common' genes among the three gene sets (SAM-88, GR-251, WT-38) were then identified. This 13 member gene achieved a classification accuracy of 84% by LOOCV. In essence, these 13 'common genes' are robust significant markers and can archive comparable performance as other 'complete' marker sets. Hence they could be taken as 'optimal' genes. The 13 genes are shown in Table A4.

**Clinical Outcome of ER negative 'High-confidence' vs 'Low-confidence' tumours**

The objective of this analysis was to compare the clinical prognoses of patients with 'high-confidence' ER negative tumours to those patients harbouring 'low-confidence' ER negative tumours.

**Details**

Two independent data sets were analysed, referred to as the 'Rosetta' and 'Stanford' data sets. The Rosetta data set contains 29 ER negative tumours, of which 19 are 'high-confidence' while 10 are 'low-confidence'. The Stanford data set contains 19 ER negative tumours, of which 12 are 'high-confidence' and 7 are 'low-confidence'. The results of the analysis are shown in Figures 6(a) and 6(b).

In both cases, patients with 'low-confidence' tumours exhibited a worse prognosis than their high-confidence counterparts. Although this difference is not statistically significant, this may be due to low numbers of patients analyzed in these studies.

**Discussion**

The findings in this report complement and extend the previous work in this area related to the classification of breast tumours by ER subtype. In general, these studies have shown that while gene expression data can be successfully used to classify the ER subtype of most tumours, there invariably exists a certain population of tumours that exhibit a low-confidence of prediction and thus cannot be accurately classified (3,4). The inventors decided to investigate these 'low-confidence' samples, by performing an in-depth analysis of these 'low-confidence' tumours. They made a number of surprising findings. They found that in comparison to patients with 'high-confidence' tumours, patients with 'low-confidence' tumours exhibited a significantly worse overall survival and shorter time to distant metastasis. The 'high' vs 'low-confidence' classification, arrived at by computational analysis of gene expression profiles, also served to separate ER + tumours into groups exhibiting distinct clinical behaviours (Figure 2). As the discernment of such subgroups is currently not possible using conventional immuno-histopathological techniques, these results also demonstrate how the classification of a breast tumour's ER status by expression profiling and computational analysis can be medically extremely useful.

The inventors also made the surprising finding that the 'low-confidence' state is significantly associated with elevated expression of the ERBB2 receptor. However, they emphasize that the connection between ERBB2 and 'low-confidence' predictions remains an *association,* and that at this point they have no evidence (from their own data) that ERBB2 is functionally responsible for causing the 'low-confidence' state. Nevertheless, given that ER and ERBB2 are currently the two most clinically relevant molecular biomarkers in breast cancer, it is tempting to speculate that these results suggest that there may exist substantial cross-talk between these two signaling pathways in breast cancer, a possibility that has also been proposed by others (7). Intriguingly, the association between ERBB2+ and 'low-confidence' prediction, although highly significant, is not perfect, as a few ERBB2+ tumours were also found to exhibit 'high-confidence' predictions, while not all 'low-confidence' tumours are ERBB2+. Thus, it is unlikely the 'low-confidence' population of breast tumours could have been discerned by conventional histopathological techniques used to detect ERBB2 such as IHC and FISH. Instead, the inventors believe that for tumours designed ERBB2+ by routine histopathology, that the further examination of these tumours for the presence of such characteristic 'expression perturbations' may be a promising method to distinguish between tumours that are likely to be more clinically aggressive versus those that will progress along a comparatively more indolent course.

Exploring this possibility will be an important task for future research. Clinically, elevated ERBB2 expression in ER+ breast tumours has long been associated with decreased sensitivity to anti-hormonal therapies, and a number of experimental papers have been reported addressing possible mechanisms by which ERBB2 activity might cause this effect. In general, the most popular model has been one in which elevated ERBB2 signaling causes ER to exhibit diminished transcriptional activity, either through transcriptional down-regulation of the ER gene (17), posttranslational modifications of ER (e.g. phosphorylation) (18), or via induction of ER binding corepressors such as MTA1 (19). If the effects of ERBB2 were mediated primarily through effects on ER transcriptional activity, then one might expect that a substantial number of the genes whose transcription is significantly perturbed in the ERBB2+ 'low-confidence' samples should correspond to genes which are direct targets of ER. The inventors found, however, that a significant proportion of the genes that were significantly perturbed in both ER+ and ER- tumours have not been previously identified as estrogen-induced genes, and these genes also appear to lack potential EREs in their promoters. This is particularly the case in the ER- tumours, in which only 9% of the significantly perturbed genes were found to contain high-confidence putative EREs in their promoters. Although the inventors cannot rule out the possibility that these perturbed genes may be indirect targets of ER or may be activated by ER via non-ERE mechanisms, these findings raise the possibility that ERBB2 activity may regulate a significant fraction of genes in breast tumours in an ER-independent fashion. There are numerous avenues by which this could occur. For example, ERBB2 might regulate other transcription factors besides ER through activation of the RAS/MAPK or PI3/Akt pathways (18).

Alternatively, ERBB2 activity may results in the induction of chromatin factors such as MTA1 which may play more pleiotropic effects (19).

## Materials and Methods

Breast Tissue Samples and Patient Data Breast tissue samples and clinical data were obtained from the Tissue Repository in the institution National Cancer Center of Singapore, after appropriate approvals had been obtained from the institution's Repository and Ethics Committees. Samples were grossly dissected in the operating theater immediately after surgical excision, and flash-frozen in liquid N2. Histological information (ER, ERBB2) was provided by the Department of Pathology at Singapore General Hospital, and samples were selected to provide a comparable number of ER+ and ER- tumours (as determined by IHC) for each data set.

Tumour samples contained >50% tumour content as assessed by cryosections. 55 tumours (35 ER+ samples and 20 ER-samples), was used as training data, while a separate set of 41 tumours (21 ER+ and 20 ER- samples) was used for blind testing. A detailed list of all samples and clinical data for the patient is included in Table S1.

## Sample Preparation and Microarray Hybridization

RNA was extracted from tissues using Trizol reagent and processed for Affymetrix Genechip hybridizations using U133A Genechips according to the manufacturer's instructions.

## Data Preprocessing

Raw chip scans, were quality controlled using the Genedata Refiner program and deposited into a central data storage facility. The expression data was pre-processed by removing genes whose expression was absent throughout all samples (i.e. 'A' calls), subjecting the remaining genes to a log2 transformation, and mediate-centering by samples.

## Prediction of ER status

Two classification algorithms, weighted voting (WV) (20) and support vector machines (SVMs) (21), were used to classify breast tumours according to ER subtype. Classification accuracy is defined as the number of correctly classified samples divided by the total number of samples. For the WV analyses, classification accuracy was determined using a gene set of the top 50 discriminating genes for ER status, while the SVM-based binary classifier utilized all genes.

***Weighted Voting (WV)***: The weighted voting algorithm utilizes a signal-to-noise (S2N) metric to perform binary classifications. Each gene belonging to a predictor set is assigned a 'vote', expressed as the weighted difference between the gene expression level in the sample to be classified and the average class mean expression level. Weighting is determined using the correlation metric $P(g,c)=\frac{\mu_1-\mu_2}{\sigma_1+\sigma_2}$ ($\mu$ and $\sigma$ denotes means and standard deviations of expression levels of the gene in each of the two classes). The ultimate vote for a particular class assignment is computed by summing all weighted votes made by each gene used in the class discrimination. The "prediction strength" (PS) is defined as : $PS=\frac{V_{WIN}-V_{LOSE}}{V_{WIN}+V_{LOSE}}$ where $V_{WIN}$ and $V_{LOSE}$ are the vote totals for the winning and losing classes, respectively. PS reflects the relative margin of victory and hence provides a quantitative reflection of prediction certainty.

***Support Vector Machine (SVM)*** : Support Vector Machines are classification algorithms which define a discrimination surface in the utilized feature (gene) space that attempts to maximally separate classes of training data (21). An unknown test sample's position relative to the discrimination surface determines its class. Distances are usually calculated in the n-dimensional gene space, corresponding to the total number of gene expression values considered. The inventors used SVM-FU (available at www.ai.mit.edu/projects/cbcl/) with the linear kernel to implement the SVM analysis. The confidence of each SVM prediction is based on the distance of a test sample from the discrimination surface, as previously described (22).

Identification of Low Confidence Tumours

Due to the clinical importance of achieving good prediction confidence, the inventors conservatively chose a high confidence threshold to minimize potential false positive classifications. On the basis of the leave-one-out cross validation (LOOCV) results, they used a threshold of 0.4 and identified 16 samples (out of a total of 96) as being in the 'low confidence' group. A tumour sample was assigned to the "low-confidence" category if its prediction strength (PS) from WV was less than this threshold.

Selection of Differentially Expressed Genes and Determination of Expression Perturbations Significance analysis of microarrays (SAM) is a statistical methodology developed to identify genes that are differentially expressed between separate groups (11). Genes are ranked are according to their statistical likelihood of being regulated. The SAM algorithm also performs a permutation analysis of the expression data to estimate the number of genes identified as being 'differentially regulated' by random chance (i.e. false positives). This number is the 'false discovery rate' (FDR). Depending upon the desired stringency, different reports have used FDRs ranging from <5% to 33% (23,24).

Student's t-test was used to compare levels of expression in the SAM-133 gene set between 'high' and 'low-confidence' groups. A gene was classified as exhibiting significant 'perturbed expression' if its p-value was less than 0.05.

Computational Identification of Estrogen Response Elements (EREs) using DEREF A computational algorithm, Dragon ERE Finder (DEREF) (14), was used to identify putative estrogen response elements (EREs), which are DNA binding sites of ER within promoters (see http://sdmc.lit.org.sg/ERE- V2/index for a description of the underlying methodology of DEREF). On the default setting, DEREF produces on average one ERE pattern prediction per 13,000 nt on human genomic DNA, with a sensitivity of 83%. To reduce the number of false positives, the inventors applied in this report an additional criteria that a predicted ERE pattern of 17 nucleotides (14) also had to match (based on BLAST (25) matching without allowed gaps) a similar ERE pattern from at least one other human gene promoter, under conditions where the latter pattern could be predicted by DEREF at a sensitivity of 97%. The ERE searches in this report were performed against a database of approximately 11,000 reference human promoter sequences covering the range [-3000,+1000] relative to the 5'end of the gene, which was generated using the FIE2 program (26,27). Some genes to be analyzed were not contained in this promoter database, and the ERE searches for these genes were thus not performed. Such genes are denoted in Table 2 by N/A.

**Identification of Tumours with Low Prediction Strength ("Low-confidence") in Stanford and Rosetta Data Sets**

Weighted Voting and Leave One Out Cross Validation was independently performed for two independent data sets (referred to as "Stanford" and "Rosetta" data sets). The results are plotted in a similar manner to those of Figure 1, and the plots are shown in Figure 7. In both data sets, the low-confidence tumours can be identified as the points at which tumours begin to demonstrate qualitatively reduced prediction strengths (PS's) (the 'cliff-points') from the majority of the tumour population. Although each dataset was analysed independently, the proportions of 'low-confidence' tumours for all datasets are highly comparable, ranging from 15-19% of all tumours (Rosetta data set shown in Figure 7(a) = 18/117 (15.4%); Stanford data set shown in Figure 7(b) =14/74 (18.9%)), our data set = 16/96 (16.7%))

**Details of Different Array Technologies used to produce Figure 7 data**

Stanford data set: This data was produced using 2-colour cDNA microarrays, in which PCR-amplified cDNA fragments (representing different genes) were robotically deposited onto a solid substrate to create the microarray

Rosetta data set: This data was produced using 2 colour oligonucleotide microarrays, in which 70-80mer oligonucleotides (representing different genes) were chemically synthesized in-situ on a solid substrate to create the microarray.

<u>Details of Patient Populations</u>

The Stanford data set consists of cDNA microarray data for 78 breast carcinomas (tumours) and 7 nonmalignant samples with overall patient survival information.

The Rosetta set consists of 117 early stage (lymph-node negative) breast tumours profiled using oligonucleotide-based microarrays

<u>Population Size</u>

As shown above, the low-confidence tumours occupy around 15-19% of each breast tumour population. To confidently identify this tumour subpopulation, a minimum data set of at least 25-30 profiles, preferably higher (around 80-100 tumours, as in the three data sets above) is preferably required.

**Sample Data**

Table S7 shows the mean ($\mu$) and standard deviation ($\sigma$) parameters for use in a Weighted Voting algorithm for each gene of the SAM-133 geneset. These data could be used to assign the an unknown breast tumour sample as high or low confidence, given a set of expression levels for genes of the SAM-133 geneset. The genes of Table 2 are included in the SAM-133 geneset. The data is specific to Weighted Voting techniques applied to expression data from the Affymetrix U133 genechip.

Table S8 shows expression data for the Table A4 multigene classifier (common 13 genes) across high confidence and low confidence samples. The data are specific for the Affymetrix U133A genechip and have been through data preprocess. The gene expression profiles of the Table A4 multigene classifier can be used as training data to build a predictive model (eg, WV and SVM), which then can assign the confidence of an unknown breast tumour.

The data is tab delimited, and has the following format:

Columns:

1st column: Probe_ID of prognostic set genes

2nd column: Gene Name

3rd and other columns: gene expression data

Rows:

1st row: Sample Ids (35 samples)

2nd row: Confidence (high or low) of sample.

3rd and other rows: gene expression data

The gene expression data is derived as described in the 'Sample Preparation and Microarray Hybridization' and 'Data Preprocessing' (see Materials and Methods section).

Table S9 shows the mean ($\mu$) and standard deviation ($\sigma$) parameters for use in a Weighted Voting algorithm for each gene of the Table A4 geneset. These data could be used to assign the an unknown breast tumour sample as high or low confidence, irrespective of ER status of the tumour, given a set of expression levels for genes of the Table A4 geneset.

The data is specific to Weighted Voting techniques applied to expression data from the Affymetrix U133 genechip.

**References**

1. Tavassoli, F. A. and Schnitt S. J. (1992) Pathology of the Breast. In (Elsevier)
2. Biswas, D.K., Averboukh, L., Sheng, S., Martin, K. Ewaniuk, D.S., Jawde, T.F., Wang, F., Pardee, A.B. (1998) Classification of breast cancer cells on the basis of a functional assay for estrogen receptor. Mol Med, 4, 454-467
3. Gruvberger, S., M. Ringner, Y. Chen, S. Panavally, L. H. Saal, A. Borg, M. Ferno, C. Peterson, and P. Meltzer (2001) Estrogen Receptor Status in Breast Cancer is Associated with Remarkably Distinct Gene Expression Patterns. Cancer Research, 61, 5979-5984
4. West, M., Blanchette, C., Dressman, H., Huang, E., Ishida, S., Spang, R., Zuzan, H., Olson, J.A. Jr, Marks, J.R., Nevins, J.R. (2001) Predicting the clinical status of human breast cancer by using gene expression profiles. Proc Natl Acad Sci U S A. 98, 11462-67.
5. Pietras R.J., Arboleda, J., Reese, D.M., Wongvipat, N., Pegram, M.D., Ramos, L., Gorman, C.M., Parker, M.G., Sliwkowski, M.X., Slamon, D.J. (1995) HER-2 tyrosine kinase pathway targets estrogen receptor and promotes hormone-independent growth in human breast cancer cells. Oncogene, 10, 2435-2446
6. Kurokawa, H. and Arteaga, C.L. (2001) Inhibition of erbB receptor (HER) tyrosine kinases as a strategy to abrogate antiestrogen resistance in human breast cancer. Clinical Cancer Research, 12, 4436s-4442s
7. Bange, J., Zwick, E., and Ullrich, A. (2001) Molecular targets for breast cancer therapy and prevention. Nature Medicine, 7, 548-552
8. Chia, K. S., A. Seow, H. P. Lee, and K. Shanmugaratnam (2000) Cancer Incidence in Singapore, 1993-1997. In (Singapore Cancer Registry)
9. Sorlie T, Perou CM, Tibshirani R, Aas T, Geisler S, Johnsen H, Hastie T, Eisen MB, van de Rijn M, Jeffrey SS, Thorsen T, Quist H, Matese JC, Brown PO, Botstein D, Eystein Lonning P, Borresen-Dale AL. (2001) Gene expression patterns of breast carcinomas distinguish tumour subclasses with clinical implications. Proc Natl Acad Sci U S A. 98, 10869-74.

10. Van 't Veer LJ, Dai H, van de Vijver MJ, He YD, Hart AA, Mao M, Peterse HL, van der Kooy K, Marton MJ, Witteveen AT, Schreiber GJ, Kerkhoven RM, Roberts C, Linsley PS, Bernards R, Friend SH. (2002) Gene expression profiling predicts clinical outcome of breast cancer. Nature, 415, 530-6.

11. Tusher, V. G., R. Tibshirani, and G. Chu (2001) Significance Analysis of Microarrays Applied to the Ionizing Radiation Response. Proc. Natl. Acad. Sci U S A. 98, 5116-5121

12. Kallioniemi A, Kallioniemi OP, Piper J, Tanner M, Stokke T, Chen L, Smith HS, Pinkel D, Gray JW, Waldman FM. (1994) Detection and mapping of amplified DNA sequences in breast cancer by comparative genomic hybridization. Proc Natal Acad Sci U S A. 91, 2156-60.

13. Charpentier AH, Bednarek AK, Daniel RL, Hawkins KA, Laflin KJ, Gaddis S, MacLeod MC, Aldaz CM. (2000) Effects of estrogen on global gene expression: identification of novel targets of estrogen action. Cancer Research, 60, 5977-83.

14. Bajic, V.B., Tan, S.L., Chong, A., Tang, S., Strom, A., Gustafsson, J., Lin, C.Y., Liu, E. (2002) Dragon ERE Finder ver.2 : A tool for accurate detection and analysis of estrogen response elements in vertebrate genomes. Nucleic Acid Res., in press

15. Alizadeh, A.A., M. B. Eisen, R. E. Davis, C. Ma, I. S. Lossos, A. Rosenwald, J. C. Boldrick, H. Sabet, T. Truc, Y. Xin, J. I. Powell, L. Yang, G. E. Marti, T. Moore, J. Hudson, L. Lisheng, D. B. Lewis, R. Tibshirani, G. Sherlock, W. C. Chan, T. C. Greiner, D. D. Weisenburger, J. O. Armitage, R. Warnke, R. Levy, W. Wilson, M. R. Grever, J. C. Byrd, D. Botstein, P. O. Brown, and L. M. Staudt (2000) Distinct types of diffuse large B-cell lymphoma identified by gene expression profiling. Nature, 403, 503-511

16. Bittner, M., P. Meltzer, Y. Chen, Y. Jiang, E. Seftor, M. Hendeix, M. Radmacher, R. Simon, Z. Yakhini, A. Ben-Dor, N. Sampas, E. Dougherty, E. Wang, F. Marincola, C. Gooden, J. Lueders, A. Glatfelter, P. Pollock, J. Carpten, E. Gillanders, D. Leja, K. Dietrich, C. Beaudry, M. Berens, D. Alberts, V. Sondak, N. Hayward, and J. Trent (2000) Molecular classification of cutaneous malignant melenoma by gene expression profiling. Nature, 406, 536-540

17. Grunt TW, Saceda M, Martin MB, Lupu R, Dittrich E, Krupitza G, Harant H, Huber H, Dittrich C (1995). Bidirectional interactions between the estrogen receptor and the cerbB-2 signaling pathways: heregulin inhibits estrogenic effects in breast cancer cells. Int J Cancer, 63, 560-567

18. Stoica GE, Franke TF, Wellstein A, Morgan E, Czubayko F, List HJ, Reiter R, Martin MB, Stoica A (2003). Heregulin-beta1 regulates the estrogen receptor-alpha gene expression and activity via the ErbB2/PI 3-K/Akt pathway. Oncogene, 22, 2073- 2087.

19. Mazumdar, A., Wang, R.A., Mishra, S.K., Adam, L., Bagheri-Yarmand, R., Mandal, M., Vadlamudi, R.K., Kumar, R. (2000) Transcriptional repression of oestrogen receptor by metastasis-associated protein 1 corepressor. Nature Cell Biol, 3, 30-37

20. Golub TR, Slonim DK, Tamayo P, Huard C, Gaasenbeek M, Mesirov JP, Coller H, Loh ML, Downing JR, Caligiuri MA, Bloomfield CD, Lander ES. (1999). Molecular classification of cancer: class discovery and class prediction by gene expression monitoring. Science, 286, 531-7.

21. Vapnik V. (1998) Statistical Learning Theory. Wiley, New York.

22. Ramaswamy S, Tamayo P, Rifkin R, Mukherjee S, Yeang CH, Angelo M, Ladd C, Reich M, Latulippe E, Mesirov JP, Poggio T, Gerald W, Loda M, Lander ES, Golub TR. (2001) Multiclass cancer diagnosis using tumour gene expression signatures. Proc Natl Acad Sci U S A. 98, 15149-54.

23. Mueller, A., O'Rourke, J., Grimm, J., Guillemin, K., Dixon, M.F., Lee, A. and Falkow, S. (2003) Distinct gene expression profiles characterize the histopathological stages of disease in Helicobacter-induced mucosa-associated lymphoid tissue lymphoma. Proc Natl Acad Sci USA, 100, 1292 - 1297.

24. Sanoudou, D., Haslett, J.N., Kho, A.T., Guo, S., Gazda, H.T., Greenberg, S.A., Lidov, H.G.V., Kohane, I.S., Kunkel, L.M., and Beggs, A.H. (2003) Expression profiling reveals altered satellite cell numbers and glycolytic enzyme transcription in nemaline myopathy muscle. Proc Natl Acad Sci U S A, 100, 4666 - 4671.

25. Altschul,S.F., Madden,T.L., Schaffer,A.A., Zhang,J., Zhang,Z., Miller,W. and Lipman,D.J. (1997) Gapped BLAST and PSI-BLAST: a new generation of protein database search programs, Nucleic Acids Res. 25, 3389-3402.

26. Chong, A., Zhang, G., Bajic, V.B. (2002) Information and sequence extraction around the 5'-end and translation initiation site of human genes, In Silico Biology, 2, 461-465.

27. Chong, A., Zhang, G., Bajic, V.B. (2003) FIE2: A program for the extraction of genomic DNA sequences around the start and translation initiation site of human genes, Nucleic Acids Research, in press.

28. Eisen MB, Spellman PT, Brown PO, Botstein D. (1998) Cluster analysis and display of genome-wide expression patterns. Proc Natl Acad Sci USA. 95(25), 14863-14868.

| **Table 1**. Association Between Clinical Parameters and ER Classification Confidence | | | | | | | |
|---|---|---|---|---|---|---|---|
| **Training Data Set (This Report)** | | | | **Stanford data set** | | | |
| **Parameter** | **No. of patients** | **Mean Confidence** | **P value** | **Parameter** | **No. of patients** | **Mean Confidence** | **P value** |
| ERBB2 | | | <0.001 | ERBB2 | | | <0.001 |
| Positive | 18 | 0.58 | | Positive | 9 | 0.233 | |
| Negative | 37 | 0.89 | | Negative | 65 | 0.667 | |
| Age | | | 0.45 | Age | | | 0.03 |
| <55 yr | 25 | 0.76 | | <55 yr | 33 | 0.545 | |
| >=55 yr | 30 | 0.81 | | >=55 yr | 41 | 0.669 | |
| Node | | | 0.98 | Node | | | 0.91 |
| 0 | 21 | 0.787 | | 0 | 22 | 0.619 | |
| 1-2 | 30 | 0.785 | | 1-2 | 52 | 0.612 | |
| Histology grade | | | 0.98 | Histology grade | | | 0.28 |
| I | 7 | 0.804 | | I | 9 | 0.727 | |
| II | 36 | 0.784 | | II | 32 | 0.631 | |
| III-IV | 8 | 0.779 | | III | 32 | 0.583 | |
| PR | | | 0.03 | TP53 | | | 0.11 |
| Positive | 19 | 0.88 | | wild type | 38 | 0.659 | |
| Negative | 31 | 0.71 | | mutation | 36 | 0.567 | |

**Table 2.** The top 50 genes that are significantly perturbed between ER+/Low and ER+/High samples (a), and ER-/Low and ER-/High samples (b). In the ERE column, "ERE" indicates that the promoter contains a high confidence putative ERE as predicted by DEREF, "non-ERE" indicates that a putative ERE was not found, while "Low" indicates that an ERE was found for that promoter at medium confidence. N/A means that the promoter was not analyzed as it was not possible to determine their transcription start sites based on full-length transcripts. Genes are ranked in order of their S2N ratio between High and Low-confidence samples.

**Table 2**

| **(a) ER+/Low vs. ER+/High** | | | |
|---|---|---|---|
| **Gene Name** | **UniGene** | **ERE** | **Rank** |
| estrogen receptor 1 | Hs.1657 | Non-ERE | 1 |
| dynein, axonemal, light intermediate polypeptide 1 | Hs.406050 | Low | 2 |
| cytochrome c oxidase subunit Vlc | Hs.351875 | Non-ERE | 3 |
| annexin A9 - | Hs.279928 | ERE | 4 |
| N-acetyltransferase 1 (arylamine N-acetyltransferase) | Hs.155956 | ERE | 5 |
| cytochrome P450, subfamily IIB (phenobarbital-inducible), polypeptide 6 | Hs.1360 | Low | 6 |
| retinoic acid receptor, alpha | Hs.361071 | ERE | 7 |
| insulin-like growth factor 1 receptor | Hs.239176 | N/A | 8 |
| serine (or cysteine) proteinase inhibitor, clade A (alpha-1 antiproteinase, antitrypsin), member 5 | Hs.76353 | Low | 9 |
| Homo sapiens cDNA: FLJ21695 fis, clone COL09653, mRNA sequence mRNA | Hs.306803 | N/A | 10 |
| B-cell CLL/lymphoma 2 | Hs.79241 | ERE | 11 |
| GREB1 protein | Hs.193914 | Non-ERE | 12 |
| RNB6 | Hs.241471 | ERE | 13 |
| GATA binding protein 3 | Hs.169946 | Non-ERE | 14 |

(continued)

| (a) ER+/Low vs. ER+/High | | | |
|---|---|---|---|
| **Gene Name** | **UniGene** | **ERE** | **Rank** |
| Homo sapiens mRNA; cDNA DKFZp564F053 (from clone DKFZp564F053), mRNA sequence | Hs.71968 | N/A | 15 |
| WW domain-containing protein 1 | Hs.355977 | Non-ERE | 16 |
| GDNF family receptor alpha 1 | Hs.105445 | Non-ERE | 17 |
| chromosome 1 open reading frame 34 | Hs.125783 | N/A | 18 |
| lymphoid nuclear protein related to AF4 | Hs.38070 | N/A | 19 |
| interleukin 6 signal transducer (gp130, oncostatin M receptor) | Hs.82065 | Non-ERE | 20 |
| regulator of G-protein signalling 11 | Hs.65756 | ERE | 21 |
| Human insulin-like growth factor 1 receptor mRNA, 3' sequence, mRNA sequence | Hs.405998 | N/A | 22 |
| hepsin (transmembrane protease, serine 1) | Hs.823 | Non-ERE | 23 |
| sema domain, immunoglobulin domain (Ig), short basic domain, secreted, (semaphorin) 3B | Hs.82222 | Non-ERE | 24 |
| UDP-glucose ceramide glucosyltransferase | Hs.432605 | ERE | 25 |
| cytochrome P450, subfamily IIB (phenobarbital-inducible), polypeptide 7 | Hs.330780 | N/A | 26 |
| troponin T1, skeletal, slow | Hs.73980 | N/A | 27 |
| microtubule-associated protein tau | Hs.101174 | Non-ERE | 28 |
| seven in absentia homolog 2 (Drosophila) | Hs.20191 | Non-ERE | 29 |
| progesterone receptor | Hs.2905 | Non-ERE | 30 |
| KIAA0882 protein | Hs.90419 | N/A | 31 |
| hypothetical protein FLJ20151 | Hs.279916 | Low | 32 |
| ATP-binding cassette, sub-family A (ABC1), member 3 | Hs.26630 | ERE | 33 |
| carbonic anhydrase XII | Hs.5338 | ERE | 34 |
| solute carrier family 16 (monocarboxylic acid transporters), member 6 | Hs.114924 | Low | 35 |
| hypothetical protein FLJ12910 | Hs.15929 | Non-ERE | 36 |
| hypothetical protein FLJ20627 | Hs.238270 | Non-ERE | 37 |
| trichorhinophalangeal syndrome I | Hs.26102 | Non-ERE | 38 |
| calsyntenin 2 | Hs.12079 | N/A | 39 |
| serine (or cysteine) proteinase inhibitor, clade A (alpha-1 antiproteinase, antitrypsin), member 3 | Hs.234726 | ERE | 40 |
| vav 3 oncogene | Hs.267659 | Non-ERE | 41 |
| LIV-1 protein, estrogen regulated | Hs.79136 | N/A | 42 |
| Homo sapiens mRNA; cDNA DKFZp434E082 (from clone DKFZp434E082), mRNA sequence | Hs.432587 | N/A | 43 |
| adenylate cyclase 9 | Hs.20196 | ERE | 44 |
| KIAA0876 protein | Hs.301011 | N/A | 45 |
| heme binding protein 1 | Hs.294133 | ERE | 46 |
| stanniocalcin 2 | Hs.155223 | Low | 47 |
| complement component 4B | Hs.433721 | N/A | 48 |
| solute carrier family 27 (fatty acid transporter), member 2 | Hs.11729 | N/A. | 49 |
| T-box 3 (ulnar mammary syndrome) | Hs.267182 | Non-ERE | 50 |

| (b) ER-/Low vs. ER-/High | | | |
|---|---|---|---|
| hypothetical protein FLJ20151 | Hs.279916 | Low | 1 |
| carbonic anhydrase XII | Hs.5338 | Low | 2 |
| GATA binding protein 3 | Hs.169946 | Non-ERE | 3 |
| homolog of yeast long chain polyunsaturated fatty acid elongation enzyme 2 | Hs.250175 | Non-ERE | 4 |
| WW domain-containing protein 1 | Hs.355977 | Non-ERE | 5 |

(continued)

| (a) ER+/Low vs. ER+/High | | | |
|---|---|---|---|
| **Gene Name** | **UniGene** | **ERE** | **Rank** |
| X-box binding protein 1 | Hs.149923 | Non-ERE | 6 |
| adipose specific 2 | Hs.74120 | Low | 7 |
| melanoma antigen, family D, 2 | Hs.4943 | N/A | 8 |
| anterior gradient 2 homolog (Xenepus laevis) | Hs.91011 | Non-ERE | 9 |
| cytochrome c oxidase subunit Vlc | Hs.351875 | Non-ERE | 10 |
| aldo-keto reductase family 7, member A3 (aflatoxin aldehyde reductase) | Hs.284236 | N/A | 11 |
| tight junction protein 3 (zona occludens 3) | Hs.25527 | N/A | 12 |
| LAG1 longevity assurance homolog 2 (S. cerevisiae) | Hs.285976 | ERE | 13 |
| inositol 1,4,5-triphosphate receptor, type 1 | Hs.198443 | Non-ERE | 14 |
| fructose-1,6-bisphosphatase 1 | Hs.574 | ERE | 15 |
| KIAA0882 protein | Hs.90419 | N/A | 16 |
| hypothetical protein FLJ12910 | Hs.15929 | Non-ERE | 17 |
| LIV-1 protein, estrogen regulated | Hs.79136 | N/A | 18 |
| methylcrotonoyl-Coenzyme A carboxylase 2 (beta) | Hs.167531 | Non-ERE | 19 |
| cytochrome P450, subfamily IIB (phenobarbital-inducible), polypeptide 7 | Hs.330780 | N/A | 20 |
| trefoil factor 3 (intestinal) | Hs.82961 | Low | 21 |
| Human clone 23948 mRNA sequence | Hs.159264 | N/A | 22 |
| N-acetyltransferase 1 (arylamine N-acetyltransferase) | Hs.155956 | Low | 23 |
| GREB1 protein | Hs.193914 | Non-ERE | 24 |
| retinoic acid induced 3 | Hs.194691 | Non-ERE | 25 |
| solute carrier family 16 (monocarboxylic acid transporters), member 6 | Hs.114924 | Low | 26 |
| dynein, axonemal, light intermediate polypeptide 1 | Hs.406050 | Low | 27 |
| solute carrier family 7 (cationic amino acid transporter, y+ system), member 8 | Hs.22891 | Low | 28 |
| WD repeat domain 10 | Hs.70202 | Non-ERE | 29 |
| calsyntenin 2 | Hs.12079 | N/A | 30 |
| v-myb myeloblastosis viral oncogene homolog (avian) | Hs.1334 | Low | 31 |
| trefoil factor 1 (breast cancer, estrogen-inducible sequence expressed in) | Hs.350470 | Low | 32 |
| hypothetical protein MGC2601 | Hs.124915 | ERE | 33 |
| dachshund homolog (Drosophila) | Hs.63931 | Non-ERE | 34 |
| mucin 1, transmembrane | Hs.89603 | N/A | 35 |
| complement component 4B | Hs.433721 | N/A | 36 |
| cysteine-rich protein 1 (intestinal) | Hs.423190 | N/A | 37 |
| NPD009 protein | Hs.283675 | Low | 38 |
| sema domain, immunoglobulin domain (Ig), short basic domain, secreted, (semaphorin) 3B | Hs.82222 | Non-ERE | 39 |
| HRAS-like suppressor 3 | Hs.37189 | N/A | 40 |
| ATP-binding cassette, sub-family A (ABC1), member 3 | Hs.26630 | Low | 41 |
| microtubule-associated protein tau | Hs.101174 | Non-ERE | 42 |
| Myosin VI [Homo sapiens], mRNA sequence | Hs.385834 | N/A | 43 |
| CGI-49 protein | Hs.238126 | N/A | 44 |
| retinoic acid receptor, alpha | Hs.361071 | Low | 45 |
| vav 3 oncogene | Hs.267659 | Non-ERE | 46 |
| chromosome 1 open reading frame 34 | Hs.125783 | N/A | 47 |
| estrogen receptor 1 | Hs.1657 | Non-ERE | 48 |
| solute carrier family 27 (fatty acid transporter), member 2 | Hs.11729 | N/A | 49 |
| TBX3-iso protein | Hs.332150 | NIA. | 50 |

Table S1. Clinical information of breast tumor samples.

| Table S1. Clinical information for our data sets | | | | | | | |
|---|---|---|---|---|---|---|---|
| **Sample ID** | **ER** | **ERBB2*** | **PR** | **AGE** | **NODE** | **STAGE** | **RACE** |
| **The initial collection (55 samples)** | | | | | | | |
| 980177 | + | neg | + | 75 | 2 | IIIA | CHINESE |
| 980178 | + | neg | - | 69 | 1 | IIB | CHINESE |
| 980194 | - | pos | - | 58 | 1 | IIB | CHINESE |
| 980197 | + | pos | + | 55 | 1 | IIB | CHINESE |
| 980203 | + | neg | + | 44 | 0 | I | CHINESE |
| 980208 | + | neg | + | 42 | 1 | IIB | CHINESE |
| 980214 | + | pos | - | 49 | 1 | IIIB | CHINESE |
| 980215 | + | neg | - | 54 | | | CHINESE |
| 980216 | - | neg | - | 65 | 1 | IIB | Indian |
| 980217 | + | neg | | 54 | 1 | IIB | CHINESE |
| 980220 | + | pos | | 43 | 0 | IIA | CHINESE |
| 980221 | + | neg | + | 34 | 1 | IV | CHINESE |
| 980238 | - | pos | | 62 | | | CHINESE |
| 980247 | - | neg | | 35 | | | CHINESE |
| 980261 | + | neg | - | 60 | | | CHINESE |
| 980338 | - | neg | - | 55 | 0 | IIA | CHINESE |
| 980346 | + | neg | + | 54 | 0 | I | CHINESE |
| 980353 | - | neg | - | 59 | 0 | IIA | CHINESE |
| 980373 | - | pos | - | 77 | 0 | IIA | CHINESE |
| 980380 | - | pos | - | 55 | 0 | I | CHINESE |
| 980383 | + | neg | | 66 | 0 | IIA | CHINESE |
| 980391 | + | neg | + | 56 | 0 | I | CHINESE |
| 980395 | - | pos | - | 68 | 1 | IIB | CHINESE |
| 980396 | - | pos | - | 66 | 1 | IIB | CHINESE |
| 980403 | + | neg | + | 73 | 0 | IIA | CHINESE |
| 980404 | + | neg | + | 46 | 1 | IIB | CHINESE |
| 980409 | + | neg | - | 48 | 0 | I | CHINESE |
| 980411 | - | neg | - | 72 | 0 | IIA | CHINESE |
| 980434 | + | neg | + | 73 | 0 | IIA | CHINESE |
| 980441 | - | neg | - | 66 | 1 | IIB | CHINESE |
| 990075 | + | neg | + | 66 | 1 | IIB | CHINESE |
| 990082 | + | neg | + | 49 | 1 | IIB | CHINESE |
| 990107 | + | neg | - | 51 | 1 | IIB | Indian |
| 990113 | + | neg | + | 70 | 1 | IIIA | CHINESE |
| 990115 | + | pos | + | 38 | 1 | IIB | CHINESE |
| 990123 | + | neg | + | 53 | 1 | IIIA | CHINESE |
| 990134 | - | pos | - | 43 | 0 | IIA | CHINESE |
| 990148 | + | pos | - | 60 | 1 | IIB | CHINESE |
| 990174 | - | neg | - | 56 | 1 | IIB | CHINESE |
| 990223 | + | pos | - | 52 | 1 | IIA | CHINESE |
| 990262 | - | pos | - | 68 | 1 | IIB | CHINESE |
| 990299 | - | neg | - | 58 | 1 | IIIA | CHINESE |
| 990375 | + | neg | - | 38 | 0 | I | CHINESE |
| 2000209 | + | pos | - | 58 | 0 | IIA | CHINESE |
| 2000422 | + | neg | + | 52 | 1 | IIIA | CHINESE |
| 2000500 | - | neg | - | 44 | 1 | IV | CHINESE |
| 2000683 | + | neg | + | 72 | 0 | IIA | CHINESE |
| 2000759 | - | pos | - | 57 | 0 | I | CHINESE |

(continued)

| Table S1. Clinical information for our data sets | | | | | | | |
|---|---|---|---|---|---|---|---|
| **Sample ID** | **ER** | **ERBB2*** | **PR** | **AGE** | **NODE** | **STAGE** | **RACE** |
| **The initial collection (55 samples)** | | | | | | | |
| 2000768 | + | neg | + | 39 | 0 | IIA | CHINESE |
| 2000775 | + | neg | - | 51 | 0 | IIA | CHINESE |
| 2000779 | + | neg | - | 48 | 0 | IIB | CHINESE |
| 2000804 | + | neg | + | 39 | 1 | IIB | CHINESE |
| 2000813 | - | pos | - | 60 | 1 | IIB | CHINESE |
| 2000829 | - | pos | - | 51 | 1 | IIB | CHINESE |
| 2000948 | + | neg | - | 56 | 1 | IIB | CHINESE |
| **The second collection (41 samples)** | | | | | | | |
| 980058 | + | neg | | 72 | | | CHINESE |
| 980193 | - | neg | | 49 | | | CHINESE |
| 980256 | - | neg | | 46 | | | CHINESE |
| 980278 | + | neg | | 64 | | | CHINESE |
| 980285 | - | neg | | 49 | | | CHINESE |
| 980288 | + | pos | | 45 | | | INDIAN |
| 980315 | - | neg | | 59 | | | CHINESE |
| 980333 | + | neg | | 51 | | | CHINESE |
| 980335 | - | pos | | 33 | | | CHINESE |
| 2000104 | + | pos | | 59 | | | CHINESE |
| 2000171 | - | pos | | 50 | | | CHINESE |
| 2000210 | - | pos | | 50 | | | MALAY |
| 2000215 | + | neg | | 50 | | | CHINESE |
| 2000220 | + | neg | | 52 | | | CHINESE |
| 2000237 | + | pos | | 43 | | | CHINESE |
| 2000272 | + | neg | | 50 | | | INDIAN |
| 2000274 | + | neg | | 40 | | | CHINESE |
| 2000287 | - | pos | | 53 | | | CHINESE |
| 2000320 | - | neg | | 67 | | | CHINESE |
| 2000376 | - | pos | | 65 | | | CHINESE |
| 2000399 | - | pos | | 44 | | | CHINESE |
| 2000401 | + | neg | | 51 | | | CHINESE |
| 2000593 | - | neg | | 60 | | | CHINESE |
| 2000597 | + | neg | | 57 | | | CHINESE |
| 2000609 | + | neg | | 62 | | | CHINESE |
| 2000638 | - | neg | | 60 | | | CHINESE |
| 2000641 | - | pos | | 47 | | | MALAY |
| 2000651 | + | neg | | 45 | | | CHINESE |
| 2000652 | - | pos | | 56 | | | CHINESE |
| 2000675 | - | pos | | 78 | | | CHINESE |
| 2000709 | - | pos | | 45 | | | CHINESE |
| 2000731 | - | neg | | 68 | | | INDIAN |
| 2000787 | + | neg | | 57 | | | CHINESE |
| 2000818 | + | neg | | 52 | | | CHINESE |
| 2000880 | - | neg | | 54 | | | CHINESE |
| 20020021 | + | neg | | 64 | | | CHINESE |
| 20020051 | + | neg | | 38 | | | MALAY |

(continued)

| The second collection (41 samples) | | | | |
|---|---|---|---|---|
| 20020056 | + | neg | 71 | INDIAN |
| 20020071 | + | neg | 58 | CHINESE |
| 20020090 | - | pos | 60 | CHINESE |
| 20020160 | + | neg | 82 | CHINESE |

* **Determination of ERBB2 status**: In the training set (55 samples), ERBB2 status was determined by conventional immunohistochemistry and in agreement with expression profiling. 21 are reported as ERBB2+. For other data sets, ERBB2 status was determined by expression profiling and analysis oFERBB2 and other 17q-linked genes.

**Table S2 : Classification Results of Independent Test and External**

**Breast Cancer Datasets**

*Leave-One-Out Cross Validation (LOOCV) :* We used a standard leave-one-out cross-validation (LOOCV) approach to assess classification accuracy in the training set. In LOOCV, one sample in the training set is initially 'left out', and the classifier operations (eg gene selection and classifier training) are performed on the remaining samples. The 'left out' sample is then classified using the trained algorithm, and this process is then repeated for all samples in the training set.

The output of the WV analyses for all four data sets (including PS) and corresponding p-values for the association of ERBB2 expression with prediction confidence can be obtained as an Excel file from http://www.omniarray.com/ERClassification.html.

**Table S3 : Identification of Genes Important for ER Subtype**

**Discrimination**

Significance Analysis of Microarrays (SAM) was used to identify and rank 133 genes that were differentially regulated between ER+ and ER- tumors (FDR of 0%, ≥2-fold expression change). 122 of them are up-regulated in ER+ (positive gene) and 11 are down-regulated in ER+ (negative genes). The S2N ratio of a particular gene reflects the extent of the expression perturbation observed between Low and High confidence samples.

**Table S3. SAM-133 Gene List**

| **122 Genes Positively Correlated to ER+ Status** | | | | | **S2N Ratio** | |
|---|---|---|---|---|---|---|
| **Rank** | **Probe_ID** | **UG** | **Gene Name** | **GB_Accession** | **ER-** | **ER+** |
| 1 | 205225_at | Hs.1657 | estrogen receptor 1 | NM_000125.1 | -0.29577 | 1.273725 |
| 2 | 209603_at | Hs.169946 | GATA-binding protein 3 | AI796169_RC | -1.08401 | 0.863193 |
| 3 | 204506_s_at | Hs.279916 | hypothetical protein FLJ20151 | BC001012.1 | -1.78617 | 0.608118 |
| 4 | 209604_s_at | Hs.169946 | GATA-binding protein 3 | BC003070.1 | -1.45575 | 0.776251 |
| 5 | 209602_s_at | Hs.169946 | GATA-binding protein 3 | AI796169_RC | -0.8137 | 0.654881 |
| 6 | 206754_s_at | Hs.1360 | cytochrome P450, subfamily IIB (phenobarbital-inducible), polypeptide 6 | NM_000767.2 | -0.2593 | 1.022511 |
| 7 | 203963_at | Hs.5338 | carbonic anhydrase XII | NM_001218.2 | -1.46907 | 0.598453 |

(continued)

| Table S3. SAM-133 Gene List | | | | | | |
|---|---|---|---|---|---|---|
| 122 Genes Positively Correlated to ER+ Status | | | | | S2N Ratio | |
| 8 | 214164_x_at | Hs.5344 | adaptor-related protein complex 1, gamma 1 subunit | BF752277 | -1.38937 | 0.650127 |
| 9 | 212956_at | Hs.90419 | KIAA0882 protein | A1348094_RC | -0.64903 | 0,68526 |
| 10 | 215867_x_at | Hs.5344 | adaptor-related protein complex 1, gamma 1 subunit | AL050025.1 | -1.63678 | 0.613887 |
| 11 | 210735_s_at | Hs.5338 | carbonic anhydrase XII | BC000278.1 | -1.44687 | 0.484214 |
| 12 | 214440_at | His, 155956 | N-acetyltransferase 1 (arylamine N-acetyltransferase) | NM_000662,1 | -0.52605 | 1.043165 |
| 13 | 202089_s_at | Hs.79136 | LIV-1 protein, estrogen regulated | NM_012319.2 | -0.61899 | 0.528173 |
| 14 | 210085_s_at | Hs.279928 | annexin A9 | AF230929.1 | -0.24463 | 1.123041 |
| 15 | 205862_at | Hs.193914 | KIAA0575 gene product | NM_014668.1 | -0.51927 | 0.883508 |
| 16 | 202088_at | Hs.79136 | LIV-1 protein, estrogen regulated | AI635449_RC | -0.5332 | 0.584697 |
| 17 | 211712_s_at | | Homo sapiens, clone MGC:1925, mRNA, complete cds. | BC005830.1 | | |
| 18 | 206401_s_at | Hs.101174 | microtubule-associated protein tau | J03778.1 | -0.33797 | 0.700836 |
| 19 | 215304_at | Hs.159264 | Human clone 23948 mRNA sequence | U79293.1 | -0.52908 | 0.19541 |
| 20 | 218195_at | Hs.15929 | hypothetical protein FLJ12910 | NM_024573.1 | -0.62769 | 0.590894 |
| 21 | 212195_at | Hs.71968 | Homo sapiens mRNA; cDNA DKFZp564F053 (from clone DKFZp564F053) | AL049265.1 | -0.22898 | 0.854505 |
| 22 | 203928_x_at | Hs.101174 | microtubule-associated protein tau | AI870749_RC | -0.35356 | 0.682993 |
| 23 | 209460_at | Hs.283675 | NPD009 protein | AF237813.1 | -0.18444 | 0.451265 |
| 24 | 212960_at | Hs.90419 | KIAA0882 protein | BE646554_RC | -0.58169 | 1.072165 |
| 25 | 209443_at | Hs.76353 | serine (or cysteine) proteinase inhibitor, clade A (alpha-1 antiproteinase, antitrypsin), member 5 | J02639.1 | 0.065273 | 0.94045 |
| 26 | 209173_at | Hs.91011 | anterior gradient 2 (Xenepus laevis) homolog | AF088867.1 | -0,80392 | -0.25677 |

(continued)

| Table S3. SAM-133 Gene List | | | | | | |
|---|---|---|---|---|---|---|
| 122 Genes Positively Correlated to ER+ Status | | | | | S2N Ratio | |
| 27 | 203071_at | Hs.82222 | sema domain, immunoglobulin domain (Ig), short basic domain, secreted, (semaphorin) 3B | NM_004636.1 | -0.39014 | 0.726153 |
| 28 | 203571_s_at | Hs.74120 | adipose specific 2 | NM_006829.1 | -0.81429 | 0.240008 |
| 29 | 205354_at | Hs.81131 | guanidinoacetate N-methyltransferase | NM_000156.3 | -0.01557 | 0.074452 |
| 30 | 213712_at | Hs.30504 | Homo sapiens mRNA; cDNA DKFZp434E082 (from clone DKFZp434E082) | BF508639_RC | 0.008265 | 0.522867 |
| 31 | 41660_at | | Cluster Incl. AL031588:dJ1163J1-1 (ortholog of mouse transmembrane receptor Celsr1 (KIAA0279 LIKE EGF-like domain containing protein similar to rat MEG | | | |
| 32 | 220744_s_at | Hs.70202 | WD repeat domain 10 | NM_018262-1 | -0.48046 | 0.159954 |
| 33 | 204798_at | Hs.1334 | v-myb avian myeloblastosis viral oncogene homolog | NM_005375.1 | -0.46303 | 0-284211 |
| 34 | 215552_s_at | Hs.272288 | Human DNA sequence from clone RP1-6315 on chromosome 6q25.1-26. Contains the 3 part of a novel gene and an exon of the ESR1 gene for estrogen receptor 1 (NR3A1, estradiol receptor), ESTs, STSs and GSSs | A1073549_RC | -0.19227 | 0.946801 |
| 35 | 209339_at | Hs.20191 | seven in absentia (Drosophila) homolog 2 | U76248.1 | -0.0458 | 0.698282 |
| 36 | 210272_at | Hs.330780 | Human cytochrome P450-IIB (hIIB3) mRNA, complete cds | M29873.1 | -0.58159 | 0.717949 |
| 37 | 205186_at | Hs.33846 | dynein, axonemal, light intermediate polypeptide | NM_003462.2 | -0.49548 | 1.221071 |
| 38 | 207414_s_at | Hs.170414 | paired basic amino acid cleaving system 4 | NM_002570.1 | -0.00943 | 0.222009 |
| 39 | 205009_at | Hs.1406 | trefoil factor 1 (breast cancer, estrogen-inducible sequence expressed in) | NM_003225.1 | -0.44277 | 0.213135 |

(continued)

| Table S3. SAM-133 Gene List | | | | | | |
|---|---|---|---|---|---|---|
| 122 Genes Positively Correlated to ER+ Status | | | | | S2N Ratio | |
| 40 | 203628_at | Hs.239176 | insulin-like growth factor 1 receptor | H05812_RC | 0.241512 | 0.748503 |
| 41 | 211323_s_at | Hs.198443 | inositol 1,4,5-triphosphate receptor, type 1 | L38019.1 | -0.72886 | 0.116021 |
| 42 | 201825_s_at | Hs.238126 | CGI-49 protein | AL572542_RC | -0.32444 | 0.398111 |
| 43 | 211234_x_at | Hs.1657 | estrogen receptor 1 | AF258449.1 | 0.268077 | 0.482442 |
| 44 | 209459_s_at | Hs.283675 | NPD009 protein | AF237813.1 | -0.40497 | 0.048419 |
| 45 | 212196_at | Hs.71968 | Homo sapiens mRNA; cDNA DKFZp564F053 (from clone DKFZp564F053) | AW242916_RC | -0.0843 | 0.516679 |
| 46 | 203438_at | Hs.155223 | stanniocalcin 2 | AI435828_RC | -0.15925 | 0.456003 |
| 47 | 217838_s_at | Hs.241471 | RNB6 | NM_016337.1 | 0.38602 | 0.872588 |
| 48 | 204041_at | Hs.82163 | monoamine oxidase B | NM_000898.1 | 0.050799 | 0.120203 |
| 49 | 203929_s_at | Hs.101174 | microtubule-associated protein tau | AI056359_RC | -0.27747 | 0.427658 |
| 50 | 200670_at | Hs.149923 | X-box binding protein 1 | NM_005080.1 | -0.83621 | 0.279976 |
| 51 | 219414_at | Hs.12079 | calsyntenin-2 | NM_022131.1 | -0.47893 | 0.553864 |
| 52 | 203627_at | Hs.239176 | insulin-like growth factor 1 receptor | A1830698_RC | 0.088492 | 0.976305 |
| 53 | 208451_s_at | Hs.278625 | complement component 4B | NM_000592.2 | -0.42162 | 0.448767 |
| 54 | 213419_at | Hs.324125 | amyloid beta (A4) precursor protein-binding, family B, member 2 (Fe65-like) | U62325.1 | -0.01491 | -0.06708 |
| 55 | 205768_s_at | Hs.11729 | fatty-acid-Coenzyme A ligase, very long-chain 1 | NM-003645.1 | -0.26778 | 0.41298 |
| 56 | 204862_s_at | Hs.81687 | non-metastatic cells 3, protein expressed in | NM_002513.1 | -0.24568 | 0.320418 |
| 57 | 210480_s_at | Hs.22564 | myosin VI | U90236.2 | -0.3344 | -0.15111 |
| 58 | 205696_s_at | Hs.105445 | GDNF family receptor alpha 1 | NM_005264.1 | 0.013863 | 0.846687 |
| 59 | 203685_at | Hs.79241 | B-cell CLLlymphoma 2 | NM_000633.1 | 0.385651 | 0.915025 |
| 60 | 218976_at | Hs.260720 | J domain containing protein 1 | NM_021800.1 | -0.17876 | 0.280663 |
| 61 | 219197_s_at | Hs.222399 | CEGP1 protein | AI424243_RC | -0.09661 | 0.157384 |

(continued)

| Table S3. SAM-133 Gene List | | | | | | |
|---|---|---|---|---|---|---|
| 122 Genes Positively Correlated to ER+ Status | | | | | S2N Ratio | |
| 62 | 202996_at | Hs.82520 | polymerase (DNA-directed), delta 4 | NM_021173.1 | 0.158087 | 0.060137 |
| 63 | 205734_s_at | Hs.38070 | lymphoid nuclear protein related to AF4 | AI990465_RC | 0.187651 | 0.796703 |
| 64 | 211235_s_at | Hs.1657 | estrogen receptor 1 | AF258450.1 | 0.269909 | 0.7271 |
| 65 | 211000_s_at | Hs.82065 | interleukin 6 signal transducer (gp130, oncostatin M receptor) | AB015706.1 | 0.204138 | 0.785104 |
| 66 | 217190_x_at | Hs.247976 | Estrogen receptor {exon 6} human, tamoxifen-resistant breast tumor 17, Genomic Mutant, 187 nt | S67777 | 0.17102 | 0.653981 |
| 67 | 202752_x_at | Hs.22891 | solute carrier family 7 (cationic amino acid transporter, y+ system), member 8 | NM_012244.1 | -0.48423 | 0.153806 |
| 68 | 201754_at | Hs.74649 | cytochrome c oxidase subunit Vlc | NM_004374.1 | -0.79843 | 1.207003 |
| 69 | 204623_at | Hs.82961 | trefoil factor 3 (intestinal) | NM_003226.1 | -0.53903 | 0.149093 |
| 70 | 207038_at | Hs.114924 | solute carrier family 16 (monocarboxylic acid transporters), member 6 | NM_004694.1 | -0.50672 | 0.593732 |
| 71 | 212637_s_at | Hs.324275 | Homo sapiens mRNA; cDNA DKFZp434D2111 (from clone DKFZp434D2111) | AU155187_RC | -0.851 | 0.852788 |
| 72 | 208682_s_at | Hs.4943 | hepatocellular carcinoma associated protein; breast cancer associated gene 1 | AF126181.1 | -0.80969 | -0.06845 |
| 73 | 218502_s_at | Hs.26102 | trichorhinophalangeal syndrome I | NM_014112.1 | -0.26191 | 0.571226 |
| 74 | 202376_at | Hs.234726 | serine (or cysteine) proteinase inhibitor, clade A (alpha-1 antiproteinase, antitrypsin), member 3 | NM_001085.2 | 0.02888 | 0.549323 |
| 75 | 215616_s_at | Hs.301011 | KIAA0876 protein | AB020683.1 | -0.00184 | 0.507129 |
| 76 | 211233_x_at | Hs.1657 | estrogen receptor 1 | M12674.1 | 0.360947 | 0.949046 |
| 77 | 205081_at | Hs.17409 | cysteine-rich protein 1 (intestinal) | M_001311.1 | -0.41153 | -0.05483 |

(continued)

| Table S3. SAM-133 Gene List | | | | | | |
|---|---|---|---|---|---|---|
| 122 Genes Positively Correlated to ER+ Status | | | | | S2N Ratio | |
| 78 | 214428_x_at | Hs.170250 | complement component 4A | K02403.1 | -0.22882 | 0.346824 |
| 79 | 209696_at | Hs.574 | fructose-1,6-bisphosphatase 1 | D26054.1 | -0.68072 | 0.137814 |
| 80 | 219682_s_at | Hs.332150 | TBX3-iso protein | NM_016569.1 | -0.26452 | 0.412502 |
| 81 | 212496_s_at | Hs.301011 | KIAA0876 protein | BE256900 | -0.272 | 0.841331 |
| 82 | 203108_at | Hs.194691 | retinoic acid induced 3 | NM_003979.2 | -0.51766 | 0.212322 |
| 83 | 206107_at | Hs.65756 | regulator of G-protein signalling 11 | NM_003834.1 | -0.0233 | 0.778074 |
| 84 | 218806_s_at | Hs.267659 | vav 3 oncogene | AF118887.1 | -0.3126 | 0.544105 |
| 85 | 209581_at | Hs.37189 | similar to rat HREV107 | BC001387.1 | -0.37261 | 0.359298 |
| 86 | 213412_at | Hs.25527 | tight junction protein 3 (zona occludens ) | NM_014428.1 3 | -0.76231 | 0.227893 |
| 87 | 212638_s_at | Hs.324275 | Homo sapiens mRNA; cDNA DKFZp434D2111 (from clone DKFZp434D2111) | BF131791 | -0.76733 | 0.888627 |
| 88 | 206469_x_at | Hs.284236 | aldo-keto reductase family 7, member A3 (aflatoxin aldehyde reductase) | NM_012067.1 | -0.77705 | 0.278936 |
| 89 | 210652_s_at | Hs.125783 | DEME-6 protein | BC004399.1 | -0.29655 | 0.806265 |
| 90 | 216381_x_at | Hs.284236 | aldo-keto reductase family 7, member A3 (aflatoxin aldehyde reductase) | AL035413 | -0.61275 | 0.253454 |
| 91 | 216092_s_at | Hs.22891 | solute carrier family 7 (cationic amino acid transporter, y+ system), member 8 | AL365347.1 | -0.67193 | 0.152525 |
| 92 | 208788_at | Hs.250175 | homolog of yeast long chain polyunsaturated fatty acid elongation enzyme 2 | AL136939.1 | -0.87121 | 0.346787 |
| 93 | 204792_s_at | His.111862 | KIAA0590 gene product | NM_014714.1 | 0.085973 | 0.134751 |
| 94 | 207847_s_at | Hs.89603 | mucin 1, transmembrane | NM_002456.1 | -0.42941 | -0.24975 |
| 95 | 213201_s_at | Hs.73980 | troponin T1, skeletal, slow | AJ011712 | -0_11892 | 0.71764 |
| 96 | 204497_at | Hs.20196 | adenylate cyclase 9 | AB011092.1 | 0.007184 | 0.509774 |
| 97 | 222314_x_at | Hs.205660 | ESTs | AW970881_RC | -0.1322 | 0.201872 |

(continued)

Table S3. SAM-133 Gene List

| 122 Genes Positively Correlated to ER+ Status | | | | | S2N Ratio | |
|---|---|---|---|---|---|---|
| 98 | 222212_s_at | Hs.285976 | tumor metastasis-suppressor | AK001105.1 | -0.74148 | 0.357607 |
| 99 | 219919_s_at | Hs.279808 | hypothetical protein FLJ10928 | NM_018276.1 | 0.085456 | 0.152147 |
| 100 | 214053_at | Hs.7888 | Homo sapiens clone 23736 mRNA sequence | AW772192_RC | -0.21533 | 0.32841 |
| 101 | 204934_s_at | Hs.823 | hepsin (transmembrane protease, serine 1) | NM_002151,1 | -0.03851 | 0.743961 |
| 102 | 216109_at | Hs.306803 | Homo sapiens cDNA: FLJ21695 fis, clone COL09653 | AK025348.1 | -0_03594 | 0.921802 |
| 103 | 203749_s_at | Hs.250505 | retinoic acid receptor, alpha | A1806984_RC | -0.3159 | 1.006049 |
| 104 | 220329_s_at | Hs.238270 | hypothetical protein FLJ20627 | NM_017909.1 | 0.068053 | 0.588123 |
| 105 | 204881_s_at | Hs.152601 | UDP-glucose ceramide glucosyltransferase | NM_003358.1 | -0.248 | 0.724338 |
| 106 | 208305_at | Hs.2905 | progesterone receptor | NM_000926.1 | 0.145722 | 0.687258 |
| 107 | 209623_at | Hs.167531 | methylcrotonoyl-Coenzyme A carboxylase 2 (beta) | AW439494_RC | -0.61293 | 0.369239 |
| 108 | 218450_at | Hs.108675 | heme-binding protein | NM_015987.1 | -0.07982 | 0.486745 |
| 109 | 204343_at | Hs.26630 | ATP-binding cassette, sub-family A (ABC1), member 3 | NM_001089.1 | -0.36256 | 0.648789 |
| 110 | 219051_x_at | Hs.124915 | hypothetical protein MGC2601 | NM_024042.1 | -0.43578 | 0.112222 |
| 111 | 205471_s_at | Hs.63931 | dachshund (Drosophila) homolog | AW772082_RC | -0.43168 | -0.26408 |
| 112 | 203439_s_at | Hs.155223 | stanniocalcin 2 | BC000658.1 | -0.28836 | 0.67174 |
| 113 | 204863_s_at | Hs.82065 | interleukin 6 signal transducer (gp130, oncostatin M receptor) | BE856546_RC | 0.259289 | 0.691633 |
| 114 | 203289_s_at | Hs.19699 | Conserved gene telomeric to alpha globin cluster | BE791629 | -0.18036 | 0.122646 |
| 115 | 221765_at | Hs.23703 | ESTs | Al378044_RC | -0.0539 | 0.714017 |
| 116 | 219001_s_at | Hs.317589 | hypothetical protein MGC10765 | NM_024345.1 | -0.28755 | 0.64098 |
| 117 | 220581_at | Hs.287738 | hypothetical protein FLJ23305 | NM_025059.1 | -0.13763 | 0.781039 |

(continued)

| Table S3. SAM-133 Gene List | | | | | | |
|---|---|---|---|---|---|---|
| **122 Genes Positively Correlated to ER+ Status** | | | | | **S2N Ratio** | |
| 118 | 211596_s_at | | Homo sapiens mRNA for membrane glycoprotein LIG-1, complete cds. | AB050468.1 | | |
| 119 | 205645_at | Hs.80667 | RALBP1 associated Eps domain containing 2 | NM_004726.1 | -0.29164 | 0.308819 |
| 120 | 219663_s_at | Hs.157527 | hypothetical protein MGC4659 | NM_025268.1 | 0.059072 | -0.06016 |
| 121 | 205380_at | Hs.15456 | PDZ domain containing 1 | NM_002614.1 | 0.094959 | 0.486972 |
| 122 | 201508_at | Hs.1516 | insulin-like growth factor-binding protein 4 | NM_001552.1 | 0.102433 | 0.237825 |

| 11 Genes Negatively Correlated to ER+ Status | | | | | | |
|---|---|---|---|---|---|---|
| 1 | 215729_s_at | Hs.9030 | TONDU | BE542323 | 0.729732 | -0.40161 |
| 2 | 201983_s_at | Hs.77432 | epidermal growth factor receptor (avian erythroblastic leukemia viral (v-erb-b) oncogene homolog) | AW157070_RC | 0.183968 | -0.10873 |
| 3 | 204914_s_at | Hs.32964 | SRY (sex determining region Y)-box 11 | AW157202_RC | -0.3552 | -0.61822 |
| 4 | 204913_s_at | Hs.32964 | SRY (sex determining region Y)-box 11 | A1360875_RC | -0.54222 | -0.6594 |
| 5 | 205646_s_at | Hs.89506 | paired box gene 6 (aniridia, keratitis) | NM_000280.1 | 0.667994 | -0.15217 |
| 6 | 207030_s_at | Hs.10526 | cysteine and glycine-rich protein 2 | NM_001321.1 | 0.526203 | -0.44193 |
| 7 | 204915_s at | Hs.32964 | SRY (sex determining region Y)-box 11 | AB028641.1 | -0.4419 | -0.47414 |
| 8 | 203021_at | Hs.251754 | secretory leukocyte protease inhibitor | NM_003064.1 (antileukoproteinase) | -0.08293 | -1.00559 |
| 9 | 209800_at | Hs.115947 | keratin 16 (focal non-epidermolytic palmoplantar keratoderma) | AF061812.1 | 0.573263 | -0.29962 |
| 10 | 203234_at | Hs.77573 | uridine phosphorylase | NM_003364.1 | 0.30456 | 0.307505 |

(continued)

| Table S3. SAM-133 Gene List | | | | | | |
|---|---|---|---|---|---|---|
| 122 Genes Positively Correlated to ER+ Status | | | | | S2N Ratio | |
| 11 | 201984_s_at | Hs.77432 | epidermal growth factor receptor (avian erythroblastic leukemia viral (v-erb-b) oncogene homolog) | NM_005228.1 | 0.416409 | 0.086073 |

**Top 54 ER discriminating genes that are negatively correlated to ER+ status**

Due to the limited number of ER negative genes, we decreased the threshold of SAM to derive 54 genes with FDR of 0%. These negative genes were used in Figure 2c) and d).

**Table S4 : Comparing the Global Expression Profiles of 'High' and 'Low-Confidence' Tumors**

SAM was used to identify differentially regulated genes between a) ER+ 'High' and 'Low' Confidence tumors, and b) ER- 'High' and 'Low' Confidence tumors. For the ER+ comparison, 50 genes were identified as up-regulated in ER+/Low and 39 are down-regulated in comparison to ER+/High tumors. For the ER- comparison, 50 genes were identified as up-regulated in ER-/Low, and no genes were identified as being downregulated in comparison to ER-/High tumors.

**Table S4. Top-ranked genes differently expressed in Low/High confidence samples**

**a) ER+/Low vs. ER+/High**

| Genes Up-regulated in ER+/Low | UniGene | Rank | Chromosome |
|---|---|---|---|
| chloride channel, calcium activated, family member 2 | Hs.241551 | 1 | |
| ESTs, Weakly similar to hypothetical protein H.sapiens | Hs.106642 | 2 | |
| v-myc avian myelocytomatosis viral related oncogene, neuroblastoma derived | Hs.25960 | 3 | |
| phenylethanolamine N-methyltransferase | Hs.1892 | 4 | 17q21-q22 |
| Alu-binding protein with zinc finger domain | Hs.289104 | 5 | |
| fibroblast growth factor receptor 4 | Hs.165950 | 6 | |
| KIAA0300 protein | Hs.173035 | 7 | |
| growth factor receptor-bound protein 7 | Hs.86859 | 8 | 17q21.1 |
| myosin, heavy polypeptide 4, skeletal muscle | Hs.272207 | 9 | |
| apomucin | Hs.103707 | 10 | |
| proline oxidase homolog | Hs.274550 | 11 | |
| S100 calcium-binding protein A8 (calgranulin A) | Hs.100000 | 12 | |
| glycine C-acetyltransferase (2-amino-3-ketobutyrate coenzyme A ligase) | Hs.54609 | 13 | |
| phospholamban | Hs.85050 | 14 | |
| CGI-96 protein | Hs.239934 | 15 | |
| leptin (murine obesity homolog) | Hs.194236 | 16 | |
| hypothetical protein FLJ14146 | Hs.103395 | 17 | |
| kynurenine 3-monooxygenase (kynurenine 3-hydroxylase) | Hs.107318 | 18 | |
| inhibin, beta B (activin AB beta polypeptide) | Hs.1735 | 19 | |
| hydroxysteroid (17-beta) dehydrogenase 2 | Hs.155109 | 20 | |
| fatty acid binding protein 7, brain | Hs.26770 | 21 | |
| orosomucoid 2 | Hs.278388 | 22 | |
| secretory leukocyte protease inhibitor (antileukoproteinase) | Hs.251754 | 23 | |

| | | | |
|---|---|---|---|
| actin, gamma 2, smooth muscle, enteric | Hs.78045 | 24 | |
| Homo sapiens mRNA; cDNA DKFZp564G112 (from clone DKEp564G112) | Hs.51515 | 25 | |
| peptidylarginine deiminase type III | Hs.149195 | 26 | |
| myosin, heavy polypeptide 11, smooth muscle | Hs.78344 | 27 | |
| S100 calcium-binding protein A9 (calgranulin B) | Hs.112405 | 28 | |
| Homo sapiens clone 23809 mRNA sequence | Hs.6932 | 29 | |
| integrin, beta 6 | Hs.123125 | 30 | |
| lipopolysaccharide-binding protein | Hs.154078 | 31 | |
| glutamate receptor, ionotrophic, AMPA 3 | Hs.100014 | 32 | |
| Homo sapiens PAC clone RP5-1093017 from 7q11.23-q21 | Hs.193606 | 33 | |
| KIAA1102 protein | Hs.202949 | 34 | |
| transmembrane 4 superfamily member 3 | Hs.84072 | 35 | |
| v-erb-b2 avian erythroblastic leukemia viral oncogene homolog 2 (neuroglioblastoma derived oncogene homolog) | Hs.323910 | 36 | 17q11.2-q12 |
| protein phosphatase 1. regulatory (inhibitor) subunit 1A | Hs.76780 | 37 | |
| HGC6.1.1 protein | Hs.225962 | 38 | |
| mucin and cadherin-like | Hs.165619 | 39 | |
| homeo box A9 | Hs.127428 | 40 | |
| 4-hydroxyphenylpyruvate dioxygenase | Hs.2899 | 41 | |
| lactotransferrin | Hs.105938 | 42 | |
| KIAA1069 protein | Hs.193143 | 43 | |
| folate hydrolase (prostate-specific membrane antigen) 1 | Hs.1915 | 44 | |
| argininosuccinate synthetase | Hs.160786 | 45 | |
| keratin 7 | Hs.23881 | 46 | |
| angiotensin receptor 2 | Hs.3110 | 47 | |
| calmodulin-like skin protein | Hs.180142 | 48 | |
| electron-transfer-flavoprotein, alpha polypeptide (glutaric aciduria II) | Hs.169919 | 49 | |
| S100 calcium-binding protein A7 (psoriasin 1) | Hs.112408 | 50 | |

| | | |
|---|---|---|
| Genes Down-regulated in ER+/Low | | |
| phorbol-12-myristate-13-acetate-induced protein 1 | Hs.96 | 1 |
| dynein, axonemal, light intermediate polypeptide | Hs.33846 | 2 |
| cytochrome P450, subfamily IIB (phenobarbital-inducible), polypeptide 6 | Hs.1360 | 3 |
| estrogen receptor 1 | Hs.1657 | 4 |
| artemin | Hs.194689 | 5 |
| carcinoembryonic antigen-related cell adhesion molecule 1 (biliary glycoprotein) | Hs.50964 | 6 |
| ESTs | Hs.23703 | 7 |
| KIAA0575 gene product | Hs.193914 | 8 |
| retinoic acid receptor, alpha | Hs.250505 | 9 |
| annexin A9 | Hs.279928 | 10 |
| Cas-$B_F$ M (murine) ectropic retroviral transforming sequence c | Hs.156637 | 11 |
| GATA-binding protein 3 | Hs.169946 | 12 |
| hypothetical protein FLJ12650 | Hs.4243 | 13 |
| arsenate resistance protein ARS2 | Hs.111801 | 14 |
| huntingtin interacting protein 2 | Hs.155485 | 15 |
| hypothetical protein FLJ13134 | Hs.99603 | 16 |
| zinc finger protein 165 | Hs.55481 | 17 |

| | | | |
|---|---|---|---|
| Homo sapiens cDNA: FLJ21695 fis, clone COL09653 | Hs.306803 | 18 | |
| insulin-like growth factor 1 receptor | Hs.239176 | 19 | |
| hepsin (transmembrane protease, serine 1) | Hs.823 | 20 | |
| two pore potassium channel KT3.3 | Hs.203845 | 21 | |
| UDP-glucose ceramide glucosyltransferase | Hs.152601 | 22 | |
| Human cytochrome P450-IIB (hIIB3) mRNA, complete cds | Hs.330780 | 23 | |
| sema domain, immunoglobulin domain (Ig), short basic domain, secreted, (semaphorin) 3F | Hs.32981 | 24 | |
| microtubule-associated protein tau | Hs.101174 | 25 | |
| phosphatidylserine-specific phospholipase A1alpha | Hs.17752 | 26 | |
| Similar to hypothetical protein PRO2831 [Homo sapiens], mRNA sequence | Hs.406646 | 27 | |
| cytochrome c oxidase subunit Vlc | Hs.74649 | 28 | |
| adenylate cyclase 9 | Hs.20196 | 29 | |
| Homo sapiens cytokine-like nuclear factor n-pac mRNA, complete cds | Hs.331584 | 30 | |
| Human DNA sequence from clone RP1-6315 on chromosome 6q25.1-26. Contains the 3 part of a novel gene and an exon of the ESR1 gene for estrogen receptor 1 (NR3A1, estradiol receptor), ESTs, STSs and GSSs | Hs.272288 | 31 | |
| calsyntenin-2 | Hs.12079 | 32 | |
| interleukin 6 signal transducer (gp130, oncostatin M receptor) | Hs.82065 | 33 | |
| A kinase (PRKA) anchor protein 10 | Hs.75456 | 34 | |
| N-acetyltransferase 1 (arylamine N-acetyltransferase) | Hs.155956 | 35 | |
| hypothetical protein FLJ13687 | Hs.278850 | 36 | |
| cystatin SA | Hs.247955 | 37 | |
| heat shock 27kD protein 1 | Hs.76067 | 38 | |
| synaptojanin 2 | Hs.61289 | 39 | |

| b) ER-/Low vs. ER-/High | | | |
|---|---|---|---|
| **Genes Up-regulated in ER-/Low** | **UniGene** | **Rank** | **Chromosome** |
| UDP-N-acetyl-alpha-D-galactosamine:polypeptide N-acetylgalactosaminyltransferase 6 (GalNAc-T6) | Hs.151678 | 1 | |
| aldehyde dehydrogenase 4 family, member A1 | Hs.77448 | 2 | |
| chromosome 6 open reading frame 29 | Hs.334514 | 3 | |
| melanoma antigen, family D, 2 | Hs.4943 | 4 | |
| phenylethanolamine N-methyltransferase | Hs.1892 | 5 | 17q21-q22 |
| tripartite motif-containing 3 | Hs.321576 | 6 | |
| hypothetical gene MGC9753 | Hs.91668 | 7 | |
| ATP-binding cassette, sub-family C (CFTR/MRP), member 6 | Hs.274260 | 8 | |
| SH3 domain binding glutamic acid-rich protein like | Hs.14368 | 9 | |
| growth factor receptor-bound protein 7 | Hs.86859 | 10 | 17q21.1 |
| 3-hydroxy-3-methylglutaryl-Coenzyme A synthase 2 (mitochondrial) | Hs.59889 | 11 | |
| fibroblast growth factor receptor 4 | Hs.165950 | 12 | |
| fatty acid synthase | Hs.83190 | 13 | |
| mucin 1, transmembrane | Hs.89603 | 14 | |
| phafin 2 | Hs.29724 | 15 | |
| carnitine acetyltransferase | Hs.12068 | 16 | |
| hypothetical protein FLJ20151 | Hs.279916 | 17 | |
| GATA binding protein 3 | Hs.169946 | 18 | |
| WW domain-containing protein 1 | Hs.355977 | 19 | |

(continued)

| | | | |
|---|---|---|---|
| transcription factor AP-2 beta (activating enhancer binding protein 2 beta) | Hs.33102 | 20 | |
| KIAA0882 protein | Hs.90419 | 21 | |
| tetraspan 1 | Hs.38972 | 22 | |
| peroxisomal biogenesis factor 11A | Hs.31034 | 23 | |
| solute carrier family 4, sodium bicarbonate cotransporter, member 8 | Hs.132136 | 24 | |
| hypothetical gene MGC9753 | Hs.91668 | 25 | |
| forkhead box A1 | Hs.70604 | 26 | |
| aquaporin 3 | Hs.234642 | 27 | |
| v-erb-b2 erythroblastic leukemia viral oncogene homolog 2, neuro/ glioblastoma derived oncogene homolog (avian) | Hs.323910 | 28 | 17q11.2-q12 |
| inositol 1,4,5-triphosphate receptor, type 1 | Hs.198443 | 29 | |
| hypothetical protein PRO1489 | Hs.197922 | 30 | |
| aldehyde dehydrogenase 3 family, member B2 | Hs.87539 | 31 | |
| Hypothetical protein [Homo sapiens], mRNA sequence | Hs.381412 | 32 | |
| dual specificity phosphatase 6 | Hs.180383 | 33 | |
| carbonic anhydrase XII | Hs.5338 | 34 | |
| NAD(P)H dehydrogenase, quinone 1 | Hs.406515 | 35 | |
| mannosidase, alpha, class 1C, member 1 | Hs.8910 | 36 | |
| KIAA0703 gene product | Hs.6168 | 37 | |
| stearoyl-CoA desaturase (delta-9-desalurase) | Hs.119597 | 38 | |
| fructose-1,6-bisphosphatase 1 | Hs.574 | 39 | |
| arylsulfatase D | Hs.326525 | 40 | |
| X-box binding protein 1 | Hs.149923 | 41 | |
| methylcrotonoyl-Coenzyme A carboxylase 2 (beta) | Hs.167531 | 42 | |
| synaptosomal-associated protein, 23kDa | Hs.184376 | 43 | |
| kraken-like | Hs.301947 | 44 | |
| anterior gradient 2 homolog (Xenepus laevis) | Hs.91011 | 45 | |
| hypothetical protein FLJ20174 | Hs.114556 | 46 | |
| chaperonin containing TCP1, subunit 2 (beta) | Hs.432970 | 47 | |
| immunoglobulin heavy constant gamma 3 (G3m marker) | Hs.300697 | 48 | |
| transmembrane 4 superfamily member 3 | Hs.84072 | 49 | |
| sorbitol dehydrogenase | Hs.878 | 50 | |

**Use of DRAGON-ERE Finder (DEREF) to Identify**

**Putative EREs in Gene Promoters**

The DEREF algorithm was used to define potential EREs in the promoters of genes belonging to various categories (see http://sdmc.lit.org.sg/ERE-V2/index for a description of the underlying methodology of DEREF). The manuscript of ref. 14 can be accessed via http://www.omniarray.com/ERClassification.html. The estrogen-induced SAGE data set was derived from (http://143.111.133.249/ggeg/, see ref. 13), using the thresholds of 3 hr fold increase >=2 and 3 hr p value < 0.005. 65 SAGE Tags were selected. These 65 SAGE Tags matched 68 genes that are furthered subject to ERE analysis. The gene set of the top 100 genes negatively correlated to ER status was derived using SAM. Table S6a depicts the results.

**Table S6a:** The ERE prediction on various data sets: E2-induced SAGE data set, genes negatively correlated to ER+, and the SAM-133 gene set.

| Data set | Non-ERE | Low | High | ERE Hit with high confidence | 'N/A' |
|---|---|---|---|---|---|
| SAGE E2-induced | 21 | 15 | 21 | 41.18% | 11 |

(continued)

| Data set | Non-ERE | Low | High | ERE Hit with high confidence | 'N/A' |
|---|---|---|---|---|---|
| ER-negative genes | 50 | 22 | 6 | 7.69% | 22 |
| SAM-133 | 15 | 15 | 17 | 36.17% | 23 |

**Table S6b.** Predicted ERE patterns by DEREF for genes listed in Table 2 of the main text.

| ERE pattern for Table 2 | | |
|---|---|---|
| **Gene Name** | **Rank** | **ERE pattern** |
| **12 ERE with high confidence out of 50 genes perturbed in ER+** | | |
| annexin A9 | 4 | PP 2783 CA-GGGCA-CCC-CAGCC-TG new CCTGTTGGGGCACATACCAGCAGGGCACCCCAGCCT GCACCCCAGAGGGGGTCCCAG 21 |
| N-acetyltransferase 1 (arylamine N-acetyltransferase) | 5 | PP 150 AA-GGTTA-CAA-TAACC-AA new CCACCTTCAAATCATACTACAAGGTTACAATAACCAA AACAGCGTGGTACTGATACA 21 |
| retinoic acid receptor, alpha | 7 | PP 2149 GA-GGTCC-CTC-TGCCC-CT new TGAAGTTGATCTGTTGTATTGAGGTCCCTCTGCCCCT ATATTTATCCTAAATGGTAT 21 |
| B-cell CLL/lymphoma 2 | 11 | PP 647 CA-GGGCA-CAG-TGGCT-CA new GACAAAATAAAGATGTCAGGCAGGGCACAGTGGCTC ATGTCTGTAATCCCAGCACTT 21 |
| RNB6 | 13 | PP 1920 TT-GGTCA-GGC-TGGTC-TC known AAAGACAGGGTTTCACCATGTTGGTCAGGCTGGTCT CGAACTTCTGACCTCAGGTGA 21 |
| regulator of G-protein signalling 11 | 21 | PP 847 CG-GGTCA-CTG-CAACC-TC new GGAGTGCAATGGTGCAATCTCGGGTCACTGCAACCT CCGCCTCCTGGGTTCAAGCGA 21 |
| UDP-glucose ceramide glucosyltransferase | 25 | PP 466 TG-AGTCA-CCG-TGCCC-AG new AAGTGCTGGGATTACAGGCGTGAGTCACCGTGCCCA GCCAATGGCTTGTGGTTTTCT 21 |
| ATP-binding cassette, sub-family A (ABC1), member 3 | 33 | PP 1363 CA-GGGCA-CAG-TGGCT-CA new GCACAGAGATAAAACCTCGGCAGGGCACAGTGGCTC ACGCCTGTAATCCCCACACTT 21 |
| carbonic anhydrase XII | 34 | PP 1376 TA-GGCCA-AAC-TAACC-TT new TCCTTATTCATTCCTGGGCATAGGCCAAACTAACCTT AGAAAGGAATTCAGTTTATG 21 |
| serine (or cysteine) proteinase inhibitor, clade A (alpha-1 antiproteinase, antitrypsin), member 3 | 40 | PP 2408 TT-GGTCG-GAC-TGGTC-TT new AGAGACAGGGTTTCACCTTGTTGGTCGGACTGGTCT TGAACTCCTGACCTCGTGATC 21 |
| adenylate cyclase 9 | 44 | PP 710 TT-GGTCA-GGC-TGGTC-TC known AGAGATGGGGTTTCTCCGTGTTGGTCAGGCTGGTCT CGAACTCCCGACCTCAGGTGA 21 |
| heme binding protein 1 | 46 | PP 1738 GA-GGTCC-GGG-TGGCC-GC new AAAGAGCAGAGGCGCCCGTAGAGGTCCGGGTGGCC GCTGCTGTTAACATCCATCACT 21 |

(continued)

| 3 ERE with high confidence out of 50 genes perturbed in ER- | | |
|---|---|---|
| LAG1 longevity assurance homolog 2 (S. cerevisiae) | 13 | PP 3662 CA-GGCCA-GGG-CAACC-CC new CCCAAGCCACAGGACGCGTCCAGGCCAGGGCAACC CCGCGGGCCGCTGCCAGGGTGG 21 |
| fructose-1,6-bisphosphatase 1 | 15 | PP 776 TT-GGTCA-GGC-TGGTC-TC known AGAGACGGGGTTTCTCCATGTTGGTCAGGCTGGTCT CGAGCTCCCAACCTCAGGTGA 21 |
| hypothetical protein MGC2601 | 33 | PP 966 CT-GGTCA-GGC-TGGTC-TT new AGAGACGAGGTTTCTCCATGCTGGTCAGGCTGGTCT TGAACTCCCGACCTCAGGTGA 21 |

**Table S7: Weighted Voting parameters for mean ($\mu$) and standard deviation ($\sigma$) of expression data SAM-133 geneset**

| SEQ ID | Gene Name | ER- | | ER+ | |
|---|---|---|---|---|---|
| | | mean | SD | mean | SD |
| 0_at | X-box binding protein 1 | 0.786506 | 0.716285 | 4.265411 | 1.422852 |
| 8_at | insulin-like growth factor-binding protein 4 | -0.34357 | 1.388805 | 2.57045 | 0.925761 |
| 4_at | cytochrome c oxidase subunit Vlc | -1.58027 | 1.870693 | 1.927193 | 1.237708 |
| 5_s_at | CGI-49 protein | 3.371655 | 1.153737 | 5.720964 | 0.582412 |
| 3_s_at | epidermal growth factor receptor (avian erythroblastic leukemia viral (v-erb-b) oncogene homolog) | -0.23687 | 1.75591 | 2.753161 | 0.803569 |
| 4_s_at | epidermal growth factor receptor (avian erythroblastic leukemia viral (v-erb-b) (oncogene oncogene homolog) | -1.44281 | 0.960058 | 2.42027 | 2.337701 |
| 8_at | LIV-1 protein, estrogen regulated | 1.312524 | 1.221556 | 3.870357 | 0.929939 |
| 9_s_at | LIV-1 protein, estrogen regulated | 1.734565 | 1.093064 | 4.085214 | 0.81537 |
| 6_at | serine (or cysteine) proteinase inhibitor, clade A (alpha-1 antiproteinase, antitrypsin), member 3 | 2.023548 | 1.032196 | 4.420661 | 0.934515 |

(continued)

| SEQ ID | Gene Name | ER- | | ER+ | |
|---|---|---|---|---|---|
| | | mean | SD | mean | SD |
| 2_x_at | solute carrier family 7 (cationic amino acid transporter, $y^+$ system), member 8 | 1.981605 | 1.049118 | 4.149982 | 0.712426 |
| 6_at | polymerase (DNA-directed), delta 4 | 0.786499 | 1.029001 | 3.014232 | 0.865812 |
| 1_at | secretory leukocyte protease inhibitor (antileukoproteinase) | 0.355523 | 0.675879 | 3.16287 | 1.761351 |
| 1_at | sema domain, immunoglobulin domain (Ig), short basic domain, secreted, (semaphorin) 3B | 1.825558 | 0.726706 | 4.052804 | 1.145816 |
| 8_at | retinoic acid induced 3 | -2.75146 | 0.887259 | -0.09227 | 1.606679 |
| 4_at | uridine phosphorylase cluster | -2.68964 | 1.552946 | 0.243702 | 1.641435 |
| 9_s_at | Conserved gene telomeric to alpha globin | 3.20195 | 0.718557 | 5.197518 | 0.987453 |
| 8_at | stanniocalcin 2 | -1.29648 | 1.055361 | 0.795528 | 0.993152 |
| 9_s_at | stanniocalcin 2 | -1.57332 | 1.345545 | 0.998514 | 1.454402 |
| 1_s_at | adipose specific 2 | 0.233895 | 0.988328 | 2.283714 | 1.060332 |
| 7_at | insulin-like growth factor 1 receptor | 0.141016 | 0,610073 | 2.127288 | 1.174363 |
| 8_at | insulin-like growth factor 1 receptor | 2.29995 | 0.509475 | 3.833107 | 0.788714 |
| 5_at | B-cell CLLlymphoma 2 | -1.10751 | 1.324287 | 1.15701 | 1.355875 |
| 9_s_at | retinoic acid receptor, alpha | -1.58118 | 1.167735 | 0.537334 | 1.268906 |
| 8_x_at | microtubule-associated protein tau | 0.359852 | 0.516477 | 1.888305 | 0.821962 |
| 9_s at | microtubule-associated protein tau | -2.59884 | 0.565755 | -0.00962 | 2.145673 |
| 3_at | carbonic anhydrase XII | 1.190756 | 3.229512 | 4.402 | 1.181501 |
| .1_at | monoamine oxidase B | -3.13061 | 1.085626 | -0.75919 | 1.755041 |

(continued)

| SEQ ID | Gene Name | ER- | | ER+ | |
|---|---|---|---|---|---|
| | | mean | SD | mean | SD |
| .3_at | ATP-binding cassette, sub-family A (ABC1), member 3 | -0.29571 | 1.843682 | 2.228971 | 1.512369 |
| 7_at | adenylate cyclase 9 | -2.34613 | 1.534418 | -0.05573 | 1.429526 |
| 8_s_at | hypothetical protein FLJ20151 | -3.52135 | 1.303031 | -0.87495 | 2.10528 |
| 3_at | trefoil factor 3 (intestinal) | -0.37083 | 1.33889 | 1.50405 | 0.899477 |
| 2_s_at | KIAA0590 gene product | -0.9475 | 1.745737 | 1.257564 | 1.170708 |
| 8_at | v-myb avian myeloblastosis viral oncogene homolog | 1.288571 | 1.107004 | 3.060625 | 0.97928 |
| 2_s_at | non-metastatic cells 3, protein expressed in | -1.44821 | 0.786716 | 0.388854 | 1.271171 |
| 3_s_at | interleukin 6 signal transducer (gp130, oncostatin M receptor) | -0.10956 | 1.179102 | 1.970259 | 1.431009 |
| 1_s_at | UDP-glucose ceramide glucosyltransferase | -1.39262 | 1.195462 | 1.156751 | 2.153286 |
| 3_s_at | SRY (sex determining region Y)-box 11 | -2.53383 | 1.536914 | -0.16571 | 1.727001 |
| 4_s_at | SRY (sex determining region Y)-box 11 | -1.8799 | 1.273909 | 0.144791 | 1.375233 |
| 5_s_at | SRY (sex determining region Y)-box 11 | 0.484505 | 1.125341 | 2.823356 | 1.941558 |
| 4s_at | hepsin (transmembrane protease, serine 1) | 0.462278 | 0.985428 | 2.501289 | 1.570414 |
| 9_at | trefoil factor 1 (breast cancer, estrogen-inducible sequence expressed in) | -1.98675 | 1.39922 | -0.14861 | 0.959657 |
| 1_at | cysteine-rich protein 1 (intestinal) | 0.366598 | 1.124549 | 1.87895 | 0.590829 |
| 6_at | dynein, axonemal, light intermediate polypeptide | -2.39302 | 0.959482 | -0.48343 | 1.433455 |
| 5_at | estrogen receptor 1 | -1.62943 | 1.558096 | 0.486988 | 1.459551 |

(continued)

| SEQ ID | Gene Name | ER- | | ER+ | |
|---|---|---|---|---|---|
| | | mean | SD | mean | SD |
| 4_at | guanidinoacetate N-methyltransferase | 0.719039 | 0.547264 | 2.096279 | 0.868384 |
| 0_at | PDZ domain containing 1 | -0.92507 | 1.254295 | 1.252606 | 1.789471 |
| 1_s_at | dachshund (Drosophila) homolog | 1.676963 | 0.591793 | 3.169036 | 1.05951 |
| 5_at | RALBP1 associated Eps domain containing 2 | -0.63258 | 1.838056 | 2.053427 | 2.368533 |
| 6_s_at | paired box gene 6 (aniridia, keratitis) | -0.06075 | 0.836545 | 1.524428 | 1.119938 |
| 6_s_at | GDNF family receptor alpha 1 | 3.8834 | 1.041947 | 5.212661 | 0.43379 |
| 4_s_at | lymphoid nuclear protein related to AF4 | -1.3702 | 1.00987 | 0.420671 | 1.393757 |
| 8_s_at | fatty-acid-Coenzyme A ligase, very long-chain 1 | 0.5008 | 0.790296 | 2.069968 | 1.166292 |
| 2_at | KIAA0575 gene product | 2.848348 | 1.291904 | 4.670661 | 1.303459 |
| 7_at | regulator of G-protein signalling 11 | -1.36697 | 1.337414 | 0.179662 | 0.681822 |
| 1_s_at | microtubule-associated protein tau | -3.3514 | 1.637863 | -1.01214 | 2.020108 |
| 9_x_at | aldo-keto reductase family 7, member A3 (aflatoxin aldehyde reductase) | 0.948475 | 0.99349 | 2.289914 | 0.621401 |
| 4_s_at | cytochrome P450, subfamily IIB (phenobarbital-inducible), polypeptide 6 | -0.71324 | 1.775643 | 1.082716 | 0.869708 |
| 207030_s_at | cysteine and glycine-rich protein 2 | -2.03214 | 1.126525 | -0.19338 | 1.540646 |
| 207038_at | solute carrier family 16 (monocarboxylic acid transporters), member 6 | 0.374876 | 0.580637 | 1.790818 | 1.094049 |
| 207414_s_at | paired basic amino acid cleaving system 4 | 0.341324 | 1.065353 | 2.062852 | 1.376036 |
| 207847_s_at | mucin 1, transmembrane | 0.247008 | 1.354516 | 2.257601 | 1.737215 |

(continued)

| SEQ ID | Gene Name | ER- | | ER+ | |
|---|---|---|---|---|---|
| | | mean | SD | mean | SD |
| 208305_at | progesterone receptor | -1.24605 | 0.974745 | 0.384022 | 1.29497 |
| 208451_s_at | complement component 4B | -4.78762 | 1.049086 | -2.66361 | 2.080728 |
| 208682_s_at | hepatocellular carcinoma associated protein; breast cancer associated gene 1 | -1.959 | 0.821013 | -0.3239 | 1.382716 |
| 208788_at | homolog of yeast long chain polyunsaturated fatty acid elongation enzyme 2 | 0.152008 | 0.660975 | 1.523099 | 1.038038 |
| 209173_at | anterior gradient 2 (Xenepus laevis) homolog | -4.28803 | 0.661578 | -2.56017 | 1.677193 |
| 209339_at | seven in absentia (Drosophila) homolog 2 | 1.270858 | 1.066389 | 2.646046 | 0.849767 |
| 209443_at | serine (or cysteine) proteinase inhibitor, clade A (alpha-1 antiproteinase, antitrypsin), member 5 | 4.667825 | 0.671724 | 5.873446 | 0.804606 |
| 209459_s_at | NPD009 protein | 1.072112 | 1.457092 | 2.973341 | 1.645057 |
| 209460_at | NPD009 protein | -0.96002 | 1.349904 | 0.607753 | 1.04472 |
| 209581_at | similar to rat HREV107 | -0.56188 | 0.872894 | 0.668399 | 0.727131 |
| 209602_s_at | GATA-binding protein 3 | 2.019065 | 1.056594 | 3.416464 | 0.940078 |
| 209603_at | GATA-binding protein 3 | 1.985985 | 0.863569 | 3.186089 | 0.674166 |
| 209604_s_at | GATA-binding protein 3 | 2.395052 | 1.790175 | 4.34208 | 1.519527 |
| 209623_at | methylcrotonoyl-Coenzyme A carboxylase 2 (beta) | -1.00419 | 1.154041 | 0.445889 | 1.017354 |
| 209696_at | fructose-1,6-bisphosphatase 1 | -1.68104 | 0.963742 | -0.1215 | 1.377052 |
| 209800_at | keratin 16 (focal non-epidermolytic palmoplantar keratoderma) | 2.324715 | 1.562155 | 4.012295 | 1.229197 |
| 210085_s_at | annexin A9 | 2.4829 | 1.125042 | 4.043161 | 1.290489 |

(continued)

| SEQ ID | Gene Name | ER- | | ER+ | |
|---|---|---|---|---|---|
| | | mean | SD | mean | SD |
| 210272_at | Human cytochrome P450-IIB (hIIB3) mRNA, complete cds | 1.01495 | 0.91653 | 2.191543 | 0.64021 |
| 210480_s_at | myosin VI | -0.14392 | 1.616287 | 1.455335 | 1.006298 |
| 210652_s_at | DEME-6 protein | 1.251577 | 0.889677 | 2.556116 | 0.970199 |
| 210735_s_at | carbonic anhydrase XII | 1.213425 | 2.03426 | 3.084783 | 1.272118 |
| 211000_s_at | interleukin 6 signal transducer (gp130, oncostatin M receptor) | -3.02427 | 1.43442 | -1.18813 | 1.697067 |
| 211233_x_at | estrogen receptor 1 | -0.0459 | 1.740133 | 1.544577 | 0.867934 |
| 211234_x_at | estrogen receptor 1 | 0.044649 | 1.763802 | 1.765441 | 1.206805 |
| 211235_s_at | estrogen receptor 1 | -2.24335 | 1.765844 | -0.48324 | 1.306074 |
| 211323_s_at | inositol 1,4,5-triphosphate receptor, type 1 | 2.749775 | 0.789763 | 3.855643 | 0.652063 |
| 211596_s_at | Homo sapiens mRNA for membrane glycoprotein LIG-1, complete cds. | 0.451307 | 1.03825 | 1.691284 | 0.751559 |
| 211712_s_at | Homo sapiens, clone MGC:1925, RNA, complete cds. | 0.615955 | 1.516076 | 2.069047 | 0.790366 |
| 212195_at | Homo sapiens mRNA; cDNA DKFZp564F053 (from clone DKFZp564F053) | 0.66476 | 0.873729 | 1.797193 | 0.663081 |
| 212196_at | Homo sapiens mRNA; cDNA DKFZp564F053 (from clone DKFZp564F053) | 1.370605 | 0.637597 | 2.49272 | 0.820267 |
| 212496_s_at | KIAA0876 protein | 2.9339 | 0.874367 | 4.097768 | 0.756001 |
| 212637_s_at | Homo sapiens mRNA; cDNA DKFZp434D2111 (from clone DKFZp434D2111) | -1.88266 | 1.081913 | -0.63578 | 0.780821 |
| 212638_s_at | Homo sapiens mRNA; cDNA DKFZp434D2111 (from clone DKFZp434D2111) | 2.261515 | 1.394089 | 3.785398 | 1.192581 |
| 212956_at | KIAA0882 protein | -2.7829 | 1.397052 | -0.86347 | 2.046812 |

(continued)

| SEQ ID | Gene Name | ER- | | ER+ | |
|---|---|---|---|---|---|
| | | mean | SD | mean | SD |
| 212960_at | KIAA0882 protein | -0.50333 | 1.45485 | 0.947772 | 1.02444 |
| 213201_s_at | troponin T1, skeletal, slow | -1.9544 | 1.210569 | -0.40381 | 1.441706 |
| 213412_at | tight junction protein 3 (zona occludens 3) B, member 2 (Fe65-like) | 2.951875 | 0.714379 | 4.007446 | 0.711117 |
| 213419_at | amyloid beta (A4) precursor protein-binding, family clone DKFZp434E082) | -2.21361 | 1.478023 | -0.51415 | 1.591816 |
| 213712_at | Homo sapiens mRNA; cDNA DKFZp434E082 (from | 0.270749 | 0.847277 | 1.499404 | 1.020576 |
| 214053_at | Homo sapiens clone 23736 mRNA sequence subunit | -0.39205 | 1.186238 | 0.845048 | 0.820314 |
| 214164_x_at | adaptor-related protein complex 1, gamma 1 | -1.08541 | 1.111223 | 0.178117 | 0.95879 |
| 214428_x_at | complement component 4A | 0.533406 | 0.838849 | 1.642348 | 0.807099 |
| 214440_at | N-acetyltransferase 1 (arylamine N-acetyltransferase) | -0.99962 | 0.684062 | 0.154358 | 0.999297 |
| 215304_at | Human clone 23948 mRNA sequence | 2.4353 | 0.529481 | 3.488893 | 0.879103 |
| 215552_s_at | Human DNA sequence from clone RP1-6315 on chromosome 6q25.1-26. Contains the 3 part of a novel gene and an exon of the ESR1 gene for estrogen receptor 1 (NR3A1, estradiol receptor), ESTs, STSs and GSSs | -4.0518 | 1.024367 | -2.20072 | 2.254477 |
| 215616_s_at | KIAA0876 protein | 2.582125 | 0.659442 | 3.570411 | 0.700552 |
| 215729_s_at | TONDU subunit | 1.641575 | 0.849076 | 2.756482 | 0.863148 |
| 215867_x_at | adaptor-related protein complex 1, gamma 1 system), member 8 | -0.42352 | 0.884606 | 0.727052 | 0.926142 |

(continued)

| SEQ ID | Gene Name | ER- | | ER+ | |
|---|---|---|---|---|---|
| | | mean | SD | mean | SD |
| 216092_s_at | solute carrier family 7 (cationic amino acid transporter, y+ COL09653 | 0.063651 | 1.352604 | 1.366287 | 0.918248 |
| 216109_at | Homo sapiens cDNA: FLJ21695 fis, clone reductase family 7, member A3 (aflatoxin aldehyde reductase) | -1.17386 | 1.143511 | 0.232514 | 1.345207 |
| 216381_x_at | aldo-keto reductase family 7, member A3 (attataxin aldehyde reductase) | 0.46636 | 0.383625 | 1.657506 | 1.251032 |
| 217190_x_at | Estrogen receptor {exon 6} human, tamoxifen-resistant breast tumor 17, Genomic Mutant, 187 nt | 0.899139 | 0.533766 | 2.030393 | 1.097631 |
| 217838_s_at | RNB6 | -1.31066 | 0.930532 | -0.16453 | 0.933916 |
| 218195_at | hypothetical protein FLJ12910 | 0.847629 | 0.786234 | 2.077682 | 1.202885 |
| 218450_at | heme-binding protein I | 0.080843 | 0.82158 | 1.234993 | 1.027254 |
| 218502_s_at | trichorhinophalangeal syndrome | -1.57325 | 1.012703 | -0.27651 | 1.276184 |
| 218806_s_at | vav 3 oncogene | 1.662298 | 0.790643 | 2.689179 | 0.799202 |
| 218976_at | J domain containing protein 1 | -1.84709 | 1.306292 | -0.43267 | 1.374615 |
| 219001_s_at | hypothetical protein MGC10765 | -2.18314 | 1.146729 | -0.93169 | 1.100879 |
| 219051_x_at | hypothetical protein MGC2601 | -1.64776 | 1.079359 | -0.04531 | 1.917545 |
| 219197_s_at | CEGP1 protein | 3.017955 | 0.866409 | 4.110571 | 0.929583 |
| 219414_at | calsyntenin-2 | | | | |
| 219663_s_at | hypothetical protein MGC4659 | | | | |
| 219682_s_at | TBX3-iso protein | -2.31967 | 2.774285 | -5.24093 | 1.743328 |
| 219919_s_at | hypothetical protein FLJ10928 | 1.5957 | 1.348698 | -0.22476 | 1.003375 |
| 220329_s_at | hypothetical protein FLJ20627 | 1.476165 | 1.643622 | -0.81183 | 1.617203 |
| 220581_at | hypothetical protein FLJ23305 | 0.707923 | 1.691725 | -1.11592 | 1.188481 |

(continued)

| SEQ ID | Gene Name | ER- | | ER+ | |
|---|---|---|---|---|---|
| | | mean | SD | mean | SD |
| 220744_s_at | WD repeat domain 10 | -1.15664 | 1.569856 | -2.79242 | 0.859538 |
| 221765_at | ESTs | 1.266316 | 0.936218 | -0.08462 | 0.892242 |
| 222212_s_at | tumor metastasis-suppressor | 0.105187 | 1.541242 | -1.65582 | 1.335109 |
| 222314_x_at | ESTs | 2.914925 | 1.476344 | 1.290308 | 1.093452 |
| 41660_at | Cluster Incl. AL031588: dJ1163J1.1 (ortholog of mouse transmembrane receptor Celsr1 (KIAA0279 LIKE EGF-like domain containing protein similar to rat MEG | -1.50101 | 2.986928 | -3.88453 | 1.411412 |
| | | -0.50993 | 0.923661 | -1.93244 | 1.140847 |
| | | 0.987597 | 0.893199 | -0.11725 | 0.498882 |

Table S8: Gene Expression data for Genes of Table A4 (common-13 genes)

| | | | | | | | | | | | |
|---|---|---|---|---|---|---|---|---|---|---|---|
| UID | NAME | 2000683T+neg | | 2000775T+neg | | 2000804T+neg | | 980346T+pos | | 980383T+neg | |
| 990082T+neg | | 980177T+neg | | 980178T+neg | | 980403T+neg | | 980434T+neg | | 990075T+neg | |
| 990113T+neg | | 990107T+neg | | 980203T+neg | | 980208T+pos | | 980220T+pos | | 980221T+neg | |
| 990115T+pos | | 990375T+neg | | 980404T+neg | | 980409T+neg | | 990123T+neg | | 2000422T+neg | |
| 2000787T-LA | | 2000818T-LA | | 20020021T-LA | | 20020051T-LA | | 20020056T-LA | | 980197T+pos | |
| 980215T+neg | | 980217T+neg | | 980261T+neg | | 980391T+neg | | 2000768T+pos | | 2000779T+neg | |
| 2000948T+neg | | 20020160T-LA | | 2000401T-LA | | 20020071T-LA | | 2000215T-normal-like | | | |
| 2000220T-LA | | 980333T-LA | | 980058T-LA | | 980278T-LA | | 980288T-ERBB2 | | 2000597T-LA | |
| 2000609T-LA | | 2000272T-LA | | 2000274T-normal-like | | | 980285T-Basal | | 2000593T-Basal | | |
| 2000638T-Basal | | 2000641T-ERBB2 | | 2000675T-ERBB2 | | 2000287T-ERBB2 | | 2000320T-Basal | | | |
| 2000880T-Basal | | 2000731T-Basal | | 980353T-neg | | 2000829T-pos | | 980373T-pos | | 2000500T-neg | |
| 2000759T-pos | | 980238T-pos | | 980395T-pos | | 980396T-pos | | 980411T-neg | | 980441T-neg | |
| 990262T-neg | | 980216T-neg | | 980194T-pos | | 980247T-pos | | 980338T-neg | | 990174T-neg | |
| 990299T-neg | | 2000210T-ERBB2 | | 980315T-LA | | 980335T-ERBB2 | | 980193T-Basal | | | |
| 980256T-Basal | | 980214T+pos | | 990148T+pos | | 2000209T+pos | | 990223T+pos | | | |
| 2000104T-ERBB2 | | 2000651T-normal-like | | | 2000237T-ERBB2 | | 2000652T-ERBB2 | | 2000376T-ERBB2 | | |
| 2000399T-ERBB2 | | 20020090T-ERBB2 | | 2000709T-ERBB2 | | 2000813T-pos | | 980380T-pos | | 990134T-pos | |
| 2000171T-ERBB2 | | | | | | | | | | | |
| Confidence | | | High | High | High | High | High | High | High | High | High |
| High | High | High | High | High | High | High | High | High | High | High | High |
| High | High | High | High | High | High | High | High | High | High | High | High |
| High | High | High | High | High | High | High | High | High | High | High | High |
| High | High | High | High | High | High | High | High | High | High | High | High |
| High | High | High | High | High | High | High | High | High | High | High | High |
| High | High | High | High | High | High | High | High | High | High | High | Low |
| Low | Low | Low | Low | Low | Low | Low | Low | Low | Low | Low | Low |
| Low | Low | Low | | | | | | | | | |
| 201525_at | | apolipoprotein D | | | 2.749 | 7.332 | 2.111 | 2.803 | 1.752 | 1.958 | 1.75 |
| 2.712 | 4.541 | 3.009 | 3.613 | 4.291 | 1.486 | 4.204 | 2.849 | 3.388 | 3.262 | 3.603 | |
| 3.097 | 7.419 | 5.491 | 4.873 | 1.444 | 2.954 | 1.296 | 3.352 | 2.856 | 2.266 | 5.145 | |
| 4.695 | 4.072 | 6.963 | 4.804 | 2.886 | 0.7888 | 3.226 | 0.3389 | 1.921 | 2.803 | 4.261 | |
| 4.993 | 4.251 | 0.785 | 6.066 | 4.539 | 2.019 | 5.235 | 1.808 | 4.592 | 0.09904 | 2.77 | 2.85 |
| 3.059 | 3.353 | 1.229 | 1.679 | 1.879 | 2.77 | 0.9126 | 4.246 | 6.957 | 3.753 | 7.109 | 4.31 |

(continued)

| | | | | | | | | | | |
|---|---|---|---|---|---|---|---|---|---|---|
| 1.624 | 2.986 | 2.603 | 0.984 | 4.797 | 0.5836 | 5.433 | 2.722 | 1.66 | 3.161 | 2.94 |
| 0.3395 | 1.008 | 4.023 | 2.417 | 4.21 | 4.833 | 5.118 | 0.7322 | 7.893 | 5.443 | 5.369 |
| 1.104 | 6.198 | 2.819 | 3.773 | 1.536 | 1.673 | 6.562 | 4.973 | 6.796 | 6.121 | |
| 202991_at | | START domain containing 3 | | | | 0.1623 | 0.7959 | -0.3925 | 3.014 | 0.4513 |
| 0.2522 | 0.3208 | -0.2599 | 0.5714 | -0.5644 | 0.5246 | 0.8061 | 0.6035 | -0.3416 | 2.886 | 0.8943 |
| -0.6905 | 2.991 | 0.6204 | 0.4511 | -0.4408 | -0.2534 | 0.07863 | 1.517 | 0.6792 | 0.6636 | 0.2455 |
| -0.1443 | 2.871 | -0.3209 | -0.05486 | | 1.605 | 0.1314 | 2.252 | 0.002929 | | 0.9972 |
| 0.08306 | 2.623 | 0.4914 | 0.4794 | -0.02506 | | 0.1142 | 0.3137 | 0.5399 | 3.005 | 0.2001 |
| 2.758 | 0.1815 | 0.1945 | -0.05305 | | 0.6643 | 0.5267 | 2.002 | 0.462 | 3.014 | 0.2885 |
| 0.1389 | -0.05295 | | -1.923 | 1.882 | 0.5175 | 0.09324 | 1.667 | 3.328 | 2.384 | 3.651 |
| 1.299 | 0.1444 | 0.158 | 1.234 | 2.21 | 0.1798 | -0.1465 | 0.411 | 0.5087 | 3.457 | 1.745 |
| 3.551 | -0.2846 | 0.158 | 2.62 | 3.53 | 3.728 | 3.149 | 0.2238 | -0.9861 | -0.3033 | 3.286 |
| -0.07757 | | 2.736 | 3.579 | 2.466 | 1.495 | 2.523 | 3.703 | 3.77 | | |
| 203628_art | | Human insulin-like growth factor 1 receptor mRNA, 3' sequence, mRNA sequence | | | | | | | | |
| 2.795 | 2.381 | 5.773 | 1.45 | 3.568 | 3.288 | 2.631 | 2.062 | 2.515 | 4.693 | 2 |
| 2.984 | 3.098 | 4.667 | 2.513 | 2.232 | 2.442 | 0.5148 | 2.452 | 3.675 | 4.111 | 2.55 |
| 3.705 | 1.115 | 1.538 | 1.731 | 2.76 | 3.559 | 2.259 | 1.855 | 0.6405 | 3.657 | 4.928 |
| 2.664 | 6.732 | 6.752 | 0.5081 | 2.53 | 1.503 | 1.872 | 4.124 | 1.466 | 3.48 | 2.903 |
| 0.2213 | 3.556 | 1.22 | 1.193 | 3.206 | -0.1502 | 0.07299 | 0.3962 | 0.5347 | 0.7098 | 0.06693 |
| 0.09198 | 0.3905 | -0.02844 | | -0.009415 | | 1.025 | 0.7389 | 2.194 | -0.4784 | 1.723 |
| 0.222 | 0.05793 | 0.573 | 3.054 | 1.338 | 0.6058 | 1.426 | 1.54 | 0.9868 | 0.84 | 0.1264 |
| 0.2324 | -0.258 | 1.21 | -0.8171 | 1.998 | 1.449 | -0.1467 | 0.3772 | 1.21 | -0.4615 | 1.451 |
| 0.1205 | -0.1947 | -0.9146 | 1.441 | -0.8475 | 0.04923 | 0.4557 | -2.688 | 0.2235 | 0.5537 | |
| 205307_s_at | | kynurenine 3-monooxygenase (kynurenine 3-hydroxylase) | | | | | | | -0.117 | -1.011 |
| -2.489 | -0.9037 | -1.085 | -1.12 | -1.219 | -1.735 | -1.829 | -1.721 | -1.433 | -0.02038 | |
| 1.167 | -1.694 | -1.571 | 1.055 | -2.743 | 0.03987 | 0.01731 | 0.1225 | 0.1203 | -1.484 | -0.591 |
| -1.35 | -0.2275 | 0.7435 | -1.218 | -0.4883 | -0.8609 | -0.7848 | -0.2848 | -1.499 | -0.3403 | -1.388 |
| -0.9036 | -0.3888 | -0.4186 | -1.082 | -1.261 | -1.201 | -0.1329 | -1.222 | -1.679 | -0.2855 | 0.5551 |
| -1.587 | -0.1132 | -1.485 | -1.13 | -0.7033 | -0.7773 | 0.7705 | 0.008025 | | -0.2992 | 0.06924 |
| -0.3291 | -2.038 | -1.017 | -3.967 | -0.4769 | 0.8039 | -1.589 | -0.7423 | -0.4919 | -1.328 | 0.2971 |
| -1.549 | -0.7277 | 1.643 | -1.604 | 0.5154 | -0.09918 | | -0.6515 | -0.8327 | -0.986 | -0.04337 |

(continued)

| | | | | | | | | | | | |
|---|---|---|---|---|---|---|---|---|---|---|---|
| -0.95 | -0.273 | -0.3601 | -2.266 | 1.182 | 0.7985 | -0.8065 | 1.063 | 2.302 | -0.6945 | -1.219 | |
| 0.9502 | -0.894 | 0.7855 | -1.668 | 0.1515 | -0.3956 | -1.677 | 0.22 | 1.595 | | | |
| 210761_s_at | | growth factor receptor-bound protein 7 | | | | | 0.4452 | 1.205 | 1.412 | 2.858 | |
| 1.493 | 1.508 | 0.3961 | 0.7703 | 1.033 | 0.922 | 0.4947 | 1.016 | 1.668 | 1.669 | 2.906 | |
| 1.568 | 0.889 | 3.42 | 1.335 | 0.6151 | 0.7453 | 0.6185 | 1.248 | 1.748 | 2.238 | 0.6557 | |
| 0.7697 | 1.296 | 4.588 | 0.7527 | 0.5559 | 0.7794 | 0.9863 | 1.981 | 1.503 | 0.3864 | 0.5489 | |
| 3.704 | 0.7039 | 1.561 | 0.9271 | 0.6039 | 0.9461 | 1.471 | 3.699 | 1.334 | 1.981 | 0.6054 | |
| 0.5662 | 1.051 | 1.677 | 1.507 | 3.042 | 1.307 | 4.472 | 1.189 | 0.7615 | 0.228 | 0.6253 | |
| 3.214 | 1.966 | 0.6688 | 2.263 | 3.093 | 2.839 | 1.988 | 1.721 | 1.684 | 0.6625 | 1.159 | 2.94 |
| 1.063 | 0.1599 | 1.04 | 0.2849 | 3.697 | 2.31 | 3.887 | 0.6321 | 0.7463 | 3.728 | 5.268 | |
| 3.912 | 3.666 | 1.984 | 0.7088 | 0.5511 | 3.982 | 5.042 | 4.321 | 4.339 | 4.248 | 2.174 | |
| 3.317 | 4.032 | 4.736 | | | | | | | | | |
| 210930_s_at | | v-erb-b2 erythroblastic leukemia viral oncogene homolog 2, | | | | | | | | | |
| neuro/glioblastoma derived oncogene homolog (avian) | | | | | | | -0.8461 | -2.708 | -0.9694 | 0.3187 | |
| -1.475 | -1.568 | 0.3559 | -1.343 | -2.559 | -0.9886 | -1.727 | -1.466 | -0.1998 | -0.8977 | 0.3377 | |
| -0.3748 | -1.943 | 1.36 | -1.455 | -1.361 | -1.218 | -1.374 | -0.4494 | 1.16 | 0.7238 | -0.4209 | |
| -2.201 | -0.4352 | 1.833 | -1.829 | -0.6478 | -4.138 | -0.5983 | 0.6215 | -1.066 | -1.07 | -0.332 | |
| 1.556 | -0.5345 | -0.8175 | -0.2384 | -1.649 | -0.837 | 0.487 | 1.322 | -0.7451 | 0.7285 | -0.9136 | |
| -1.812 | -3.225 | -0.1626 | -1.19 | 1.542 | -0.4326 | 1.705 | 0.2116 | -0.2503 | -1.408 | -1.292 | |
| 1.544 | -0.8231 | -1.735 | 0.4762 | 0.09548 | -0.7243 | -0.7869 | -1.927 | -1.524 | -2.637 | -4.457 | |
| -0.278 | -2.773 | -2.013 | -1.611 | -2.056 | 1.532 | 0.08922 | 2.774 | -0.2269 | -1.08 | 1.078 | 2.7 |
| 1.397 | 1.554 | -1.5 | -0.9627 | -0.8952 | 2.069 | 1.728 | 3.212 | 3.121 | 3.149 | 1.108 | |
| -0.7891 | 0.9288 | 2.864 | | | | | | | | | |
| 211657_at | | carcinoembryonic antigen-related cell adhesion molecule 6 (non-specific | | | | | | | | | |
| cross reacting antigen) | | | 3.887 | 1.127 | 5.069 | 1.162 | 4.256 | 2.372 | 0.06854 | 2.496 | |
| 0.534 | 1.805 | 0.6949 | 4.237 | 3.755 | -0.05911 | | 1.471 | 1.388 | 1.548 | 1.032 | |
| 4.176 | 0.407 | 3.742 | 3.638 | 4.006 | 3.88 | 5.988 | 1.433 | 0.1368 | 2.179 | 3.537 | |
| 0.7946 | 0.4718 | 3.327 | -0.02141 | | 1.842 | 0.3149 | 5.084 | 0.3826 | 1.889 | -0.9834 | |
| 2.416 | 0.3955 | 0.08346 | 1.603 | 2.92 | 3.158 | 0.7611 | 5.397 | -0.485 | 0.3396 | 0.1982 | |
| 0.2382 | 1.376 | 4.494 | 0.6605 | 4.674 | 4.38 | -0.2242 | 0.2056 | -0.3151 | 3.863 | 0.983 | |
| 0.8939 | 1.474 | 0.5326 | 3.265 | -0.034 | -0.8774 | -0.5614 | 2.687 | 5.257 | 4.683 | 0.7389 | |
| 0.7168 | 0.8051 | 4.189 | 4.894 | 4.905 | 1.134 | 0.431 | 0.5341 | 3.92 | 5.643 | 4.536 | |

(continued)

| | | | | | | | | | | |
|---|---|---|---|---|---|---|---|---|---|---|
| 4.869 | 3.96 | 0.6223 | 5.275 | 4.33 | 3.687 | 4.673 | 0.2819 | 1.224 | 2.126 | 5.62 |
| 3.871 | 0.6072 | | | | | | | | | |
| 213557_at | | ESTs, Weakly similar to ubiquitously transcribed tetratricopeptide repeat | | | | | | | | |
| gene, Y̅ chromosome; Ubiquitously transcribed TPR gene on Y chromosome [Homo sapiens] | | | | | | | | | | |
| [H.sapiens] | | 1.252 | 1.184 | 0.5043 | 3.153 | 1.387 | 1.868 | 0.5293 | -0.2155 | 0.3275 |
| 0.5276 | 1.395 | 1.851 | 1.543 | 0.5434 | 2.397 | 1.591 | 0.1861 | 1.623 | 1.723 | 0.7596 |
| 0.5377 | 0.3335 | 1.596 | 2.154 | 1.513 | 1.603 | 0.1632 | 1.181 | 3.969 | 0.5737 | 1.136 |
| 2.645 | 0.6143 | 2.339 | 0.2645 | 0.7221 | 0.6219 | 3.499 | 0.5513 | 1.099 | 0.9166 | 1.378 |
| 0.6302 | 0.9299 | 3.592 | 0.9732 | 3.427 | 0.7249 | 0.7654 | 0.586 | 1.397 | -1.58 | 3.088 |
| 0.7145 | 4.663 | 0.5107 | 1.368 | 1.251 | 0.8759 | 1.862 | 2.072 | 1.048 | 0.8533 | 3.836 |
| 2.693 | 4.055 | 1.126 | 0.493 | 0.3712 | 1.462 | 1.211 | 0.621 | 1.516 | 0.4326 | 1.09 2.63 |
| 2.419 | 0.667 | 0.5337 | 0.3296 | 3.749 | 3.494 | 3.834 | 3.956 | 1.295 | -0.3071 | 0.5377 |
| 0.8307 | 1.086 | 2.534 | 3.733 | 3.321 | 2.127 | 0.05067 | 3.98 | 4.461 | | |
| | | | | | | | | | | |
| 214451_at | | transcription factor AP-2 beta (activating enhancer binding protein 2 beta) | | | | | | | | |
| -3.097 | 2.467 | -3.372 | 3.439 | 0.1365 | -1.298 | 2.39 | 1.441 | 2.839 | 2.516 | -1.258 |
| -2.597 | -0.5943 | 1.978 | -0.9813 | -1.202 | 1.496 | 3.43 | 3.001 | -1.562 | 2.541 | -4.519 |
| 2.889 | 0.6659 | 1.661 | -2.472 | 1.623 | 3.059 | -2.935 | 3.575 | 1.469 | -4.59 | 3.603 |
| 3.517 | -3.813 | -0.1878 | 4.003 | -0.4031 | 0.88 | 2.51 | -4.28 | 2.753 | 1.234 | -4.588 |
| 3.173 | -4.705 | 1.066 | -1.809 | 1.967 | -2.498 | 1.153 | 0.279 | 2.117 | 3.623 | -0.005383 |
| 1.745 | -4.141 | -1.479 | -1.257 | 1.798 | 4.45 | -1.547 | 2.506 | 3.646 | -3.226 | -0.913 |
| -3.058 | -3.123 | 3.658 | -1.289 | 3.548 | -0.2634 | -1.531 | -4.923 | 2.247 | 1.723 | -2.025 |
| 3.197 | -2.015 | -0.7008 | 4.068 | 3.333 | -1.154 | 4.028 | 3.88 | 0.3311 | 3.34 | 2.444 |
| 2.631 | 3.682 | 3.38 | 3.92 | 3.618 | 4.305 | 3.96 | 4.973 | | | |
| | | | | | | | | | | |
| 215465_at | | ATP-binding cassette, sub-family A (ABC1), member 12 | | | | | | | -5.53 | -0.2993 |
| -2.982 | -1.196 | -1.515 | -1.129 | 1.018 | -2.386 | -0.3181 | -1.932 | -1.838 | 0.7215 | -1.211 |
| -1.273 | -1.483 | -0.995 | -1.928 | -1.288 | -1.39 | -0.7415 | -0.23 | -2.464 | -1.478 | -0.2715 |
| -1.114 | -2.064 | 1.22 | -2.498 | -0.9399 | -2.507 | -0.4786 | -2.321 | -0.5358 | -2.004 | -2.388 |
| -2.234 | 0.078 | -1.043 | 1.185 | -1.93 | -1.992 | -2.169 | -2.156 | -2.18 | 0.381 | -4.889 |
| 1.702 | -1.345 | -1.946 | -1.149 | -0.7878 | -0.6671 | -1.429 | -0.559 | -1.242 | -2.897 | -2.329 |
| -1.631 | -2.476 | -0.6065 | 0.4199 | -2.905 | -0.8082 | -1.942 | -1.804 | -1.404 | -1.384 | -3.471 |
| 0.2961 | -0.6596 | -0.5091 | -2.246 | -2.386 | -2.697 | -1.245 | 0.4357 | -0.7417 | -0.01172 | |
| -1.168 | -2.224 | -0.5227 | 1.617 | -0.04832 | | 0.4729 | -0.4882 | -2.002 | -0.5482 | 1.449 |

(continued)

| | | | | | | | | | | |
|---|---|---|---|---|---|---|---|---|---|---|
| -1.664 | 0.7275 | 0.8683 | -2.091 | 0.14 | 0.4634 | 1.916 | 0.7919 | | | |
| 219429_at | | fatty acid hydroxylase | | | -1.539 | -0.2486 | -0.06329 | | -0.606 | -1.426 |
| -1.273 | 0.05695 | 0.4841 | 0.3636 | -0.7702 | -1.403 | -0.7 | -1.611 | -0.5367 | 0.6557 | -0.5048 |
| -0.9159 | 0.8194 | -1.687 | -1.037 | -0.6167 | -0.1531 | -1.306 | 0.1918 | -0.531 | 0.2454 | 0.7654 |
| -1.344 | 0.7986 | 0.2327 | -0.9519 | -0.8758 | -1.052 | -0.6758 | 0.8207 | -0.1432 | -0.4994 | -0.0002446 |
| -0.2944 | -1.152 | -0.2746 | -1.314 | 0.3005 | -0.5842 | 0.218 | -0.5254 | -0.7197 | -0.6967 | -0.2 |
| -0.8899 | -0.2978 | 0.2625 | 1.562 | -1.044 | 1.383 | -0.5091 | -0.3997 | -0.8286 | -3.217 | -0.2482 |
| 0.5994 | 0.06282 | 0.06886 | 0.1471 | 0.9134 | 0.1739 | 0.6888 | -1.575 | 0.3812 | -0.6085 | 0.7442 |
| -0.7528 | -0.5949 | -0.4236 | -0.7073 | 1.218 | -0.4363 | 1.209 | 0.3444 | -0.969 | 0.2863 | 0.9532 |
| 0.7178 | 1.296 | 0.6456 | -0.4466 | 1.152 | 0.4512 | 1.933 | 1.497 | -0.3116 | 0.1834 | 0.142 |
| 1.228 | 1.876 | 1.35 | | | | | | | | |
| | | hypothetical protein FLJ22671 | | | | -0.585 | -1.416 | -0.7662 | 2.221 | -0.3646 |
| 220149_at -0.8895 | -0.6838 | -0.5557 | -0.4347 | -0.4597 | -0.07175 | | -0.09613 | | -0.4148 | -0.781 |
| -1.112 | -0.482 | -1.328 | -0.6111 | -2.445 | -1.028 | -0.6113 | -0.08989 | | -1.397 | -0.5025 |
| -0.3443 | -1.424 | -0.3695 | -0.8427 | 0.4616 | -1.052 | -1.163 | -0.9368 | -0.3882 | 0.7431 | -0.04467 |
| -0.4188 | -0.7193 | 2.204 | -1.393 | -0.7435 | -1.423 | -0.5707 | -0.4196 | -0.6552 | 2.686 | -0.6905 |
| 4.914 | -0.3156 | -0.9062 | -0.1168 | 0.2261 | 0.1723 | 0.386 | 1.191 | 2.885 | -0.7671 | -2.42 |
| -0.2398 | -1.799 | 2.044 | 0.8819 | -0.3224 | 3.604 | 1.023 | 3.736 | 2.807 | -0.5473 | -1.357 |
| 0.3665 | -0.2828 | -0.246 | -0.01971 | | 0.4476 | -0.5921 | -0.2366 | 1.906 | -0.3266 | 2.079 |
| 0.2249 | -0.5295 | 0.08667 | 2.691 | 1.636 | 1.349 | -0.3243 | -1.536 | 1.435 | 4.099 | -0.8161 |
| 1.734 | 2.641 | 1.301 | 1.355 | -1.242 | 1.708 | 3.096 | | | | |
| | | aquaporin 3 | | 0.4769 | -0.2623 | -0.7927 | 1.948 | 0.03186 | 2.194 | 0.6044 |
| 39248_at 2.335 | -0.1663 | 0.4244 | 1.476 | 3.025 | 0.6734 | 2.102 | 3.241 | -0.5173 | 0.8267 | 3.789 |
| 2.556 | -0.07496 | | 2.804 | 1.786 | -1.024 | 0.4586 | 2.795 | 0.6762 | 0.07351 | 0.3396 |
| 0.4198 | 0.7147 | 1.677 | 2.114 | -0.1301 | 0.06363 | 3.336 | 3.314 | 0.1946 | 1.919 | -0.1613 |
| 0.8785 | -0.1946 | -0.1926 | -1.876 | 3.881 | 0.3148 | -1.082 | -0.852 | 0.0508 | 0.3455 | -0.9268 |
| 0.2052 | 0.2611 | 0.8294 | 2.1 | 1.987 | 3.696 | 0.8302 | 1.104 | -1.175 | 3.041 | 0.07521 |
| 3.434 | 3.543 | 0.13 | 1.305 | 0.1424 | 2.271 | 1.841 | 0.7022 | 4.044 | 4.959 | 0.2898 |
| 0.4821 | 1.642 | 0.9258 | 1.169 | -0.382 | -0.8969 | 0.8155 | 1.156 | 3.712 | 2.333 | 1.722 |
| 1.466 | 3.247 | 1.128 | 1.167 | 3.68 | 4.088 | 4.324 | -0.5153 | 2.505 | 5.002 | 0.05894 |
| 5.292 | 0.9251 | | | | | | | | | |

**Table S9: Weighted Voting parameters for mean (μ) and standard deviation (σ) of expression data for Table A4 (common-13) geneset**

| Probe_ID | Gene Name | Full Length Ref. Sequences | Unigene | High-Confidence | | Low-confidence | |
|---|---|---|---|---|---|---|---|
| | | | | mean | SD | mean | SD |
| Upregulated in Low Confidence Tumours | | | | | | | |
| 201525_at | apolipoprotein D | NM_001647 | Hs.75736 | 3.213993 | 1.711066 | 4.43395 | 2.23157 |
| 202991_at | START domain containing 3 | NM_006804 | Hs.77628 | 0.838735 | 1.186229 | 2.215114 | 1.621765 |
| 205307_s_at | kynurenine 3-monooxygenase (kynurenine 3-hydroxylase) | NM_003679 | Hs.107318 | -0.75339 | 0.924201 | 0.105819 | 1.199695 |
| 210761_s_at | growth factor receptor-bound protein 7 v-erb-b2 erythroblastic leukemia viral oncogene homolog 2, | NM_005310 | Hs.86859 | 1.512564 | 1.051211 | 3.500556 | 1.421506 |
| 210930_s_at | neuro/ glioblastoma derived oncogene homolog (avian) carcinoembryonic antigen-related cell adhesion molecule 6 | NM_004448 | Hs.323910 | -0.71309 | 1.339254 | 1.297613 | 1.591897 |
| 211657_at | (non-specific cross reacting antigen) | NM_002483 | Hs.73848 | 1.948209 | 1.842322 | 3.452838 | 1.859184 |
| | ESTs, Weakly similar to ubiquitously transcribed tetratricopeptide repeat gene, Y chromosome; Ubiquitously transcribed TPR gene on Y chromosome [Homo sapiens] | | | | | | |
| 213557_at | [H.sapiens] | --- | Hs.14691 | 1.359728 | 1.098941 | 2.417623 | 1.605763 |

(continued)

| Probe_ID | Gene Name | Full Length Ref. Sequences | Unigene | High-Confidence | | Low-confidence | |
|---|---|---|---|---|---|---|---|
| | | | | mean | SD | mean | SD |
| Upregulated in Low Confidence Tumours | | | | | | | |
| 214451_at | transcription factor AP-2 beta (activating enhancer binding protein 2 beta) | NM_003221 | Hs.33102 | 0.234429 | 2.657284 | 3.171194 | 1.547226 |
| 215465_at | ATP-binding cassette, sub-family A (ABC1), member 12 | NM_015657 | Hs.134585 | -1.35669 | 1.237705 | 0.067599 | 1.228661 |
| 219429_at | fatty acid hydroxylase | --- | Hs.249163 | -0.32527 | 0.827988 | 0.809581 | 0.722212 |
| 220149_at | hypothetical protein FLJ22671 | NM_024861 | Hs.193745 | -0.05674 | 1.363225 | 1.200829 | 1.596251 |
| 39248_at | aquaporin 3 | NM_004925 | Hs.234642 | 1.076674 | 1.458035 | 2.508421 | 1.755277 |
| | | | | | | | |
| Up-regulated in High Confidence tumours | | | | | | | |
| 203628_at | Human insulin-like growth factor 1 receptor mRNA, 3' sequence, mRNA sequence | --- | Hs. 405998 | 1.956068 | 1.625758 | 0.129864 | 1.072433 |

**Table A1:** SAM (Significance Analysis of Microarrays) : At a FDR (False-discovery rate) of <15%, a total of 86 up-regulated and 2 down-regulated genes in low-confidence tumors were identified. Using this gene set, the LOOCV assay produced a classification accuracy of 84%.

| Genes up-regulated in Low-confidence tumors | | | | |
|---|---|---|---|---|
| Name Gene | Score(d) | q-value (%) | Unigene | Full Length Ref. Sequences |
| 206793_at | 4.1852709 | 1.3837984 | Hs.1892 | NM_002686 // phenylethanolamine N-methyltransferase |
| 211237_s_at | 4.071839 | 1.3837984 | Hs.165950 | NM_002011 // fibroblast growth factor receptor 4 isoform 1 precursor /// NM_022963 // fibroblast growth factor receptor 4 isoform 2 precursor |

(continued)

| Genes up-regulated in Low-confidence tumors | | | | |
|---|---|---|---|---|
| Name Gene | Score(d) | q-value (%) | Unigene | Full Length Ref. Sequences |
| 210761_s_at | 3.9001438 | 1.3837984 | Hs.86859 | NM_005310 // growth factor receptor-bound protein 7 |
| 206164_at | 3.8109161 | 1.3837984 | Hs.241551 | NM_006536 // calcium activated chloride channel 2 |
| 204913_s_at | 3.486716 | 1.3837984 | Hs.32964 | NM_003108 // SRY (sex determining region Y)-box 11 derived |
| 210930_s_at | 3.4544924 | 1.3837984 | Hs.323910 | NM_004448 // v-erb-b2 erythroblastic leukemia viral oncogene homolog 2, neuro/glioblastoma oncogene homolog |
| 204910_s_at | 3.3311974 | 1.3837984 | Hs.321576 | NM_006458 // tripartite motif-containing 3 isoform alpha /// NM_033278 // tripartite motif-containing 3 isoform beta /// NM_033279 // tripartite motif-containing 3 isoform gamma |
| 214451_at | 3.2935388 | 1.3837984 | Hs.33102 | NM_003221 // transcription factor AP-2 beta (activating enhancer binding protein 2 beta) |
| 217562_at | 3.2344498 | 1.3837984 | Hs.106642 | --- |
| 217276_x_at | 3.0703975 | 1.3837984 | Hs.301947 | NM_014509 // kraken-like |
| 215686_x_at | 3.0323791 | 1.3837984 | --- | --- |
| 215559_at | 3.0225718 | 1.3837984 | Hs.274260 | NM_001171 // ATP-binding cassette, sub-family C, member 6 |

(continued)

| Genes up-regulated in Low-confidence tumors | | | | |
|---|---|---|---|---|
| Name Gene | Score(d) | q-value (%) | Unigene | Full Length Ref. Sequences |
| 206827_s_at | 2.9342047 | 1.3837984 | Hs.302740 | NM_014274 // transient receptor potential cation channel, subfamily V, member 6 /// NM_ 018646 // transient receptor potential cation channel, subfamily V, member 6 |
| 208893_s_at | 2.9089684 | 1.3837984 | Hs. 180383 | NM_001946 // dual specificity phosphatase 6 isoform a /// NM_022652 // dual specificity phosphatase 6 isoform b |
| 203619_s_at | 2.8107802 | 1.3837984 | Hs.182859 | --- |
| 203824_at | 2.7813798 | 1.3837984 | Hs.84072 | NM_004616 // transmembrane 4 superfamily member 3 |
| 221811_at | 2.747613 | 1.3837984 | Hs.91668 | --- |
| 216202_s_at | | 1.3837984 | Hs.59403 | NM_004863 // serine palmitoyltransferase, long chain base subunit 2 |
| 209757_s_at | 2.7319622<br>2.7152502 | 1.3837984 | Hs.25960 | NM_005378 // v-myc myelocytomatosis viral related oncogene, neuroblastoma derived |
| 219429_at | 2.665359 | 1.3837984 | Hs.249163 | --- |
| 215465_at | 2.628031 | 1.3837984 | Hs.134585 | NM_015657 // ATP-binding cassette, sub-family A, member 12 isoform b /// NM_ 173076 // ATP-binding cassette, sub-family A, member 12 isoform a |
| 214203_s_at | 2.6018018 | 1.3837984 | Hs.343874 | NM_005974 // /// NM_ 016335 // proline dehydrogenase (oxidase) 1 |

(continued)

| Genes up-regulated in Low-confidence tumors | | | | |
|---|---|---|---|---|
| Name Gene | Score(d) | q-value (%) | Unigene | Full Length Ref. Sequences |
| 202942_at | 2.5652724 | 1.3837984 | Hs.74047 | NM_001985 // electron-transfer-flavoprotein, beta polypeptide |
| 205478_at | 2.545305 | 1.3837984 | Hs.76780 | NM_006741 // protein phosphatase 1, regulatory (inhibitor) subunit 1A |
| 203722_at | 2.5390254 | 1.3837984 | Hs.77448 | NM_003748 // aldehyde dehydrogenase 4A1 precursor /// NM_ 170726 // aldehyde dehydrogenase 4A1 precursor |
| 202991_at | 2.5022628 | 1.3837984 | Hs.77628 | NM_006804 // steroidogenic acute regulatory protein related |
| 205104_at | 2.4827654 | 1.3837984 | Hs.323833 | NM_014723 // syntaphilin |
| 215659_at | 2.4619073 | 1.3837984 | Hs.306777 | --- |
| 220622_at | 2.407245 | 1.3837984 | Hs.114005 | NM_024727 // hypothetical protein FLJ23259 |
| 208083_s_at | 2.3715062 | 1.3837984 | Hs.57664 | NM_000888 // integrin, beta 6 |
| 206043_s_at | 2.3543638 | 1.3837984 | Hs.6168 | NM_014861 // KIAA0703 gene product |
| 221345_at | 2.3351396 | 1.3837984 | Hs.248056 | NM_005306 // G protein-coupled receptor 43 |
| 39248_at | 2.3213986 | 1.3837984 | Hs.234642 | NM_004925 // aquaporin 3 |
| 205766_at | 2.3057935 | 1.3837984 | Hs.343603 | NM 003673 // telethonin |
| 211682_x_at | 2.2991204 | 1.3837984 | Hs.137585 | NM_053039 // UDP glycosyltransferase 2 family, polypeptide B28 |
| 210571_s_at | 2.2806771 | 1.3837984 | Hs.24697 | XR_000114 // |
| 219233_s_at | 2.2752973 | 1.3837984 | Hs.19054 | NM_018530 // hypothetical protein PRO2521 |

(continued)

| Genes up-regulated in Low-confidence tumors | | | | |
|---|---|---|---|---|
| Name Gene | Score(d) | q-value (%) | Unigene | Full Length Ref. Sequences |
| 204818_at | 2.2720676 | 1.3837984 | Hs.155109 | NM_0021533 // hydroxysteroid (17-beta) dehydrogenase 2 |
| 211828_s_at | 2.2270979 | 1.3837984 | Hs.170204 | --- |
| 205916_at | 2.2142817 | 1.3837984 | Hs.112408 | NM_002963 // S100 calcium-binding protein A7 |
| 209522_s_at | 2.2117774 | 1.3837984 | Hs.12068 | NM_000755 // carnitine acetyltransferase precursor, isoform 1 /// NM_004003 // carnitine acetyltransferase isoform 2 /// NM_ 144782 // carnitine acetyltransferase precursor, isoform 3 |
| 209016_s_at | 2.2112214 | 1.3837984 | Hs.23881 | --- |
| 209505_at | 2.2006627 | 1.3837984 | Hs.374991 | --- |
| 200831_s_at | 2.1927228 | 1.3837984 | Hs.119597 | NM_005063 // stearoyl-CoA desaturase (delta-9-desaturase) |
| 207802_at | 2.1832898 | 1.3837984 | Hs.54431 | NM_006061 // specific granule protein (28 kDa) |
| 216633_s_at | 2.1766477 | 1.3837984 | Hs.193143 | --- |
| 214614_at | 2.1670563 | 1.3837984 | Hs.37035 | NM_005515 // homeo box HB9 |
| 204607_at | 2.1402505 | 1.3837984 | Hs.59889 | NM_005518 // 3-hydroxy-3-methylglutaryl-Coenzyme A synthase 2 (mitochondrial) |
| 220149_at | 2.1400852 | 1.3837984 | Hs.193745 | NM_024861 // hypothetical protein FLJ22671 |
| 219756_s_at | 2.1391208 | 1.3837984 | Hs.267038 | NM_02492 // premature ovarian failure 1B |
| 213674_x_at | 2.1351759 | 1.3837984 | HS.300697 | --- |

(continued)

| Genes up-regulated in Low-confidence tumors | | | | |
|---|---|---|---|---|
| Name Gene | Score(d) | q-value (%) | Unigene | Full Length Ref. Sequences |
| 211657_at | 2.1231572 | 1.3837984 | Hs.73848 | NM_002483 // carcinoembryonic antigen-related cell adhesion molecule 6 (non-specific cross reacting antigen) |
| 204941_s_at | 2.1178907 | 1.3837984 | Hs.87539 | NM_000695 // aldehyde dehydrogenase 3B2 |
| 214133_at | 2.0836401 | 3.5733527 | Hs.99918 | --- |
| 210663_s_at | 2.0766057 | 3.5733527 | Hs.169139 | NM_003937 // kynureninase (L-kynurenine hydrolase) |
| 220414_at | 2.0543228 | 3.5733527 | Hs.180142 | NM_017422 // calmodulin-like skin protein |
| 205808_at | 2.0365629 | 3.5733527 | Hs.283664 | NM_004318 // aspartate beta-hydroxylase isoform a /// NM_020164 // aspartate beta-hydroxylase isoform e /// NM_032466 // aspartate beta-hydroxylase isoform c /// NM_032467 // aspartate beta-hydroxylase isoform d /// NM_032468 // aspartate beta-hydroxylase isoform b |
| 203365_s_at | 2.0185514 | 3.5733527 | Hs.80343 | NM 002428 // matrix metalloproteinase 15 preproprotein |
| 206509_at | 2.0114514 | 3.5733527 | Hs.99949 | NM_002652// prolactin-induced protein |
| 213557_at | 1.9942427 | 3.5733527 | Hs.14691 | --- |
| 214971_s_at | 1.9917977 | 3.5733527 | Hs.2554 | NM_003032 // sialyltransferase 1 isoform a /// NM_ 173216 // sialyltransferase 1 isoform a /// NM_ 173217 // sialyltransferase 1 isoform b |

(continued)

| Genes up-regulated in Low-confidence tumors | | | | |
|---|---|---|---|---|
| Name Gene | Score(d) | q-value (%) | Unigene | Full Length Ref. Sequences |
| 211899_s_at | 1.9768615 | 4.5901604 | Hs.8375 | NM_004295 // TNF receptor-associated factor 4 isoform 1 /// NM_145751 // TNF receptor-associated factor 4 isoform 2 |
| 220615_s_at | 1.9216703 | 4.5901604 | Hs.100895 | NM_018099 // hypothetical protein FLJ10462 |
| 206915_at | 1.8471141 | 7.400989 | Hs.355454 | NM_002509 // NK2 transcription factor related, locus 2 |
| 201388_at | 1.8446012 | 7.400989 | Hs.9736 | NM_002809 // proteasome 26S non-ATPase subunit 3 |
| 205307_s_at | 1.8282052 | 7.400989 | Hs.107318 | NM_003679 // kynurenine 3-monooxygenase (kynurenine 3-hydroxylase) |
| 209616_s_at | 1.8059335 | 7.400989 | Hs.76688 | NM_001266 // carboxylesterase 1 (monocyte/ macrophage serine esterase 1) |
| 205910_s_at | 1.7828285 | 7.400989 | Hs.406160 | NM_001807 // carboxyl ester lipase precursor |
| 201525_at | 1.7490382 | 7.400989 | Hs.75736 | NM_001647 // apolipoprotein D precursor |
| 201729_s_at | 1.7197176 | 9.106286 | Hs.151761 | --- |
| 204304_s_at | 1.6603865 | 9.106286 | Hs.112360 | NM_006017 // prominin-like 1 |
| 220225_at | 1.6559087 | 9.106286 | Hs.196927 | NM_016358 // iroquois homeobox protein 4 |
| 209560_s_at | 1.6357376 | 10.248328 | Hs.169228 | NM_003836 // delta-like homolog |

(continued)

| Genes up-regulated in Low-confidence tumors | | | | |
|---|---|---|---|---|
| Name Gene | Score(d) | q-value (%) | Unigene | Full Length Ref. Sequences |
| 207131_x_at | 1.6311017 | 10.248328 | Hs.401847 | NM_005265 // gamma-glutamyltransferase 1 /// NM_013421 // gamma-glutamyltransferase 1 precursor /// NM_013430 // gamma-glutamyltransferase 1 |
| 220972_s_at | 1.6233436 | 10.248328 | Hs.307010 | NM_030975 // keratin associated protein 9.9 |
| 209641_s_at | 1.6169812 | 10.248328 | Hs.90786 | NM_003786 // ATP-binding cassette, sub-family C, member 3 isoform MRP3 /// NM_020037 // ATP-binding cassette, sub-family C, member 3 isoform MRP3A /// NM_020038 // ATP-binding cassette, sub-family C, member 3 isoform MRP3B |
| 211588_s_at | 1.6135313 | 10.248328 | Hs.381618 | --- |
| 201946_s_at | 1.5784917 | 10.248328 | Hs.432970 | NM_006431 // chaperonin containing TCP1, subunit 2 (beta) acid protein 7, brain |
| 205029_s_at | 1.5779091 | 10.248328 | Hs.26770 | NM_001446 // fatty acid binding protein 7, brain |
| 201942_s_at | 1.5530281 | 11.432502 | Hs.5057 | NM_001304 // carboxypeptidase precursor |
| 213913_s_at | 1.5514129 | 11.432502 | Hs.11912 | --- |
| 207102_at | 1.5436816 | 11.432502 | Hs.201667 | NM_005989 // aldo-keto reductase family 1, member D1 |
| 214624_at | 1.5133976 | 11.432502 | Hs.159309 | NM_007000 // uroplakin 1A /// NM_032896 // second type |

(continued)

| Genes up-regulated in Low-confidence tumors | | | | |
|---|---|---|---|---|
| Name Gene | Score(d) | q-value (%) | Unigene | Full Length Ref. Sequences |
| 206714_at | 1.5040028 | 11.432502 | Hs.111256 | NM_001141 // arachidonate 15-lipoxygenase, 3, subfamily A, polypeptide 5 |
| 205765_at | 1.4589879 | 12.831585 | Hs.104117 | NM_000777 // cytochrome P450, family |
| 213043_s_at | 1.4469888 | 12.831585 | Hs.23106 | NM_014815 // thyroid hormone receptor-associated protein |
| | | | | |
| Genes up-regulated in High-confidence tumours | | | | |
| Gene Name | Score(d) | q-value (%) | | |
| 204286_5_at | -3.429773 | 1.3837984 | Hs.96 | NM_021127 // phorbol-12-myristate-13-acetate-induced protein 1 |
| 203628_at | -2.907564 | 1.3837984 | Hs.405998 | --- |

**Table A2: GR (Gene Ranking by SVM) : A total of 251 genes were identified with the ability to classify the HC or LC status of a tumor, with a classification accuracy of 86%. The genes are ranked by their discriminative strength, which is calculated by gene-specific misclassification rate. The Gene Rank--SVM package is provided by GeneData™ (Basel, Switzerland)**

| **Probe ID** | **Gene Description** | **Unigene ID** |
|---|---|---|
| 205225_at | estrogen receptor 1 | Hs.1657 |
| 206165_s_at | chloride channel, calcium activated, family member 2 | Hs.241551 |
| 202917_s_at | S100 calcium binding protein A8 (calgranulin A) | Hs.100000 |
| 210761_s_at | growth factor receptor-bound protein 7 | Hs.86859 |
| 202376_at | serine (or cysteine) proteinase inhibitor, clade A (alpha-1 antiproteinase, antitrypsin), member 3 | Hs.234726 |
| 211657_at | carcinoembryonic antigen-related cell adhesion molecule 6 (non-specific cross reacting antigen) | Hs.73848 |
| 206509_at | prolactin-induced protein | Hs.99949 |
| 201650_at | keratin 19 | Hs.182265 |
| 204734_at | keratin 15 | Hs.80342 |

(continued)

| Probe ID | Gene Description | Unigene ID |
|---|---|---|
| 203627_at | Human insulin-like growth factor 1 receptor mRNA, 3' sequence, mRNA sequence | Hs.405998 |
| 39248_at | aquaporin 3 | Hs.234642 |
| 209603_at | GATA binding protein 3 | Hs.169946 |
| 204508_s_at | hypothetical protein FLJ20151 | Hs.279916 |
| 215470_at | Homo sapiens cDNA FLJ36630 fis, clone TRACH2018278, mRNA sequence | Hs.14658 |
| 203749_s_at | retinoic acid receptor, alpha | Hs.361071 |
| 210930_s_at | v-erb-b2 erythroblastic leukemia viral oncogene homolog 2, neuro/glioblestoma derived oncogene homolog (avian) | Hs.323910 |
| 219233_s_at | hypothetical protein PRO2521 | Hs.19054 |
| 204475_at | matrix metalloproteinase 1 (interstitial collagenase) | Hs.83169 |
| 203875_at | SWI/SNF related, matrix associated, actin dependent regulator of chromatin, subfamily a, member 1 | Hs.152292 |
| 211699_x_at | hemoglobin, alpha 1 | Hs.272572 |
| 205239_at | amphiregulin (schwannoma-derived growth factor) | Hs.270833 |
| 205009_at | trefoil factor 1 (breast cancer, estrogen-inducible sequence expressed in) | Hs.350470 |
| 221811_at | hypothetical gene MGC9753 | Hs.91668 |
| 218541_s_at | chromosome 8 open reading frame 4 | Hs.283683 |
| 203628_at | Human insulin-like growth factor 1 receptor mRNA, 3' sequence, mRNA sequence | Hs.405998 |
| 209301_at | carbonic anhydrase II | Hs.155097 |
| 219263_at | hypothetical protein FLJ23516 | Hs.9238 |
| 203917_at | coxsackie virus and adenovirus receptor | Hs.79187 |
| 203980_at | fatty acid binding protein 4, adipocyte | Hs.391561 |
| 207076_s_at | argininosuccinate synthetase | Hs.160786 |
| 203408_s_at | AT-rich binding protein 1 (binds to nuclear matrix/scaffold-associating DNA's) | Hs.74592 |
| 203060_s_at | special sequence 3'-phosphoadenosine 5'-phosphosulfate synthase 2 | Hs.274230 |
| 63825_at | Similar to hypothetical protein PR02831 [Homo sapiens], mRNA sequence | Hs.406646 |
| 222303_at | ESTs | Hs.292477 |
| 211959_at | Unknown (protein for IMAGE:4183312) [Homo sapiens], mRNA sequence | Hs.380833 |
| 217776_at | retinol dehydrogenase 11 (all-trans and 9-cis) | Hs.179817 |

(continued)

| Probe ID | Gene Description | Unigene ID |
|---|---|---|
| 204863_s_at | interleukin 6 signal transducer (gp130, oncostatin M receptor) | Hs.82065 |
| 202887_s_at | HIF-1 responsive RTP801 | Hs.111244 |
| 201841_s_at | heat shock 27kDa protein 1 | Hs.76067 |
| 207847_s_at | mucin 1, transmembrane | Hs.89603 |
| 215294_s_at | SWI/SNF related, matrix associated, actin dependent regulator of chromatin, subfamily a member 1 | Hs.152292 |
| 218677_at | S100 calcium binding protein A14 | Hs.288998 |
| 201931_at | electron-transfer-flavoprotein, alpha polypeptide (glutaric aciduria II) | Hs.169919 |
| 202991_at | START domain containing 3 | Hs.77628 |
| 210633_x_at | keratin 10 (epidermolytic hyperkeratosis; keratosis palmaris et plantaris) | Hs.99936 |
| 203571_s_at | adipose specific 2 | Hs.74120 |
| 220625_s_at | E74-like factor 5 (ets domain transcription factor) | Hs.11713 |
| 205567_at | carbohydrate (keratan sulfate Gal-6) sulfotransferase 1 | Hs.104576 |
| 212202_s_ats | DKFZP564G2022 protein | Hs.16492 |
| 202888_s_at | alanyl (membrane) aminopeptidase (aminopeptidase N, aminopeptidase M, microsomal aminopeptidase, CD13, p150) | Hs.1239 |
| 207023_x_at | keratin 10 (epidermolytic hyperkeratosis; keratosis palmaris et plantaris) | Hs.99936 |
| 204913_s_at | SRY (sex determining region Y)-box 11 | Hs.32964 |
| 204404_at | solute carrier family 12 (sodium/potassium/ chloride transporters), member 2 | Hs.110736 |
| 211719_x_at | fibronectin 1 | Hs.287820 |
| 216510_x_at | immunoglobulin heavy constant mu | Hs.153261 |
| 218772_x_at | hypothetical protein FLJ10493 | Hs.279610 |
| 201951_at | activated leukocyte cell adhesion molecule | Hs.10247 |
| 209250_at | degenerative spermatocyte homolog, lipid desaturase (Drosophila) | Hs.185973 |
| 214745_at | KIAA1069 protein | Hs.193143 |
| 201946_s_at | chaperonin containing TCP1, subunit 2 (beta) | Hs.432970 |
| 205916_at | S100 calcium binding protein A7 (psoriasin 1) | Hs.112408 |
| 212736_at | hypothetical gene BC008967 | Hs.6349 |
| 213438_at | Homo sapiens cDNA FLJ34019 fis, clone FCBBF2002898, mRNA sequence | Hs.7309 |

(continued)

| Probe ID | Gene Description | Unigene ID |
|---|---|---|
| 205518_s_at | cytidine monophosphate-N-acetylneuraminic acid hydroxylase (CMP-N-acetylneuraminate monooxygenase) | Hs.24697 |
| 221728_x_at | Homo sapiens cDNA FLJ30298 fis, clone BRACE2003172, mRNA sequence | Hs.351546 |
| 205943_at | tryptophan 2,3-dioxygenase | Hs.183671 |
| 207431_s_at | degenerative spermatocyte homolog, lipid desaturase (Drosophila) | Hs.185973 |
| 209267_s_at | BCG-induced gene in monocytes, clone 103 | Hs.284205 |
| 204018_x_at | hemoglobin, alpha 1 | Hs.272572 |
| 212204_at | DKFZP564G2022 protein | Hs.16492 |
| 202310_s_at | collagen, type I, alpha 1 | Hs.172928 |
| 201998_at | sialyltransferase 1 (beta-galactoside alpha-2,6-sialytransferase) | Hs.2554 |
| 208792_s_at | clusterin (complement lysis inhibitor, SP-40,40, sulfated glycoprotein 2, testosterone-repressed prostate message 2, apolipoprotein J) | Hs.75106 |
| 204731_at | transforming growth factor, beta receptor III (betaglycan, 300kDa) | Hs.342874 |
| 204881_s_at | UDP-glucose ceramide glucosyltransferase | Hs.432605 |
| 205242_at | chemokine (C-X-C motif) ligand 13 (B-cell chemoattractant) | Hs.100431 |
| 200601_at | actinin, alpha 4 | Hs.182485 |
| 202037_s_at | secreted frizzled-related protein 1 | Hs.7306 |
| 219795_at | solute carrier family 6 (neurotransmitter transporter), member 14 | Hs.162211 |
| 217028_at | chemokine (C-X-C motif) receptor 4 | Hs.89414 |
| 205066_s_at | ectonucleotide pyrophosphatase/ phosphodiesterase 1 | Hs.11951 |
| 202357_s_at | B-factor, properdin | Hs.69771 |
| 202743_at | phosphoinositide-3-kinase, regulatory subunit, polypeptide 3 (p55, gamma) | Hs.372548 |
| 203874_s_at | SWI/SNF related, matrix associated, actin dependent regulator of chromatin, subfamily a, member 1 | Hs.152292 |
| 210072_at | chemokine (C-C motif) ligand 19 | Hs.50002 |
| 202990_at | phosphorylase, glycogen; liver (Hers disease, glycogen storage disease type VI) | Hs.771 |
| 206115_at | early growth response 3 | Hs.74088 |
| 205498_at | growth hormone receptor | Hs.125180 |

(continued)

| Probe ID | Gene Description | Unigene ID |
|---|---|---|
| 212789_at | KIAA0056 protein | Hs.13421 |
| 222155_s_at | putative G-protein coupled receptor GPCR41 1 | Hs.6459 |
| 218776_s_at | hypothetical protein FLJ23375 | Hs.285996 |
| 200820_at | proteasome (prosome, macropain) 26S subunit, non-ATPase, 8 | Hs.78466 |
| 203337_x_at | integrin cytoplasmic domain-associated protein 1 | Hs.173274 |
| 214218_s_at | Human XIST, coding sequence'a' mRNA (locus DXS399E), mRNA sequence | Hs.352403 |
| 201729_s_at | KIAA0100 gene product | Hs.151761 |
| 204285_s_at | phorbol-12-myristate-13-acetate-induced protein 1 | Hs.96 |
| 214451_at | transcription factor AP-2 beta (activating enhancer binding protein 2 beta) | Hs.33102 |
| 218313_s_at | UDP-N-acetyl-alpha-D-galactosamine: polypeptide N-acetylgalactosaminyltransferase 7 (GaINAc-T7) | Hs.246315 |
| 217838_s_at | RNB6 | Hs.241471 |
| 209189_at | v-fos FBJ murine osteosarcoma viral oncogene homolog | Hs.25647 |
| 201131_s_at | cadherin 1, type 1, E-cadherin (epithelial) | Hs.194657 |
| 203058_s_at | 3'-phosphoadenosine 5'-phosphosulfate synthase 2 | Hs.274230 |
| 213557_at | ESTs, Weakly similar to ubiquitously transcribed tetratricopeptide repeat gene, Y chromosome; Ubiquitously transcribed TPR gene on Y chromosome [Homo sapiens] [H.sapiens] | Hs.14691 |
| 215465_at | ATP-binding cassette, sub-family A (ABC1), member 12 | Hs.134585 |
| 213693_s_at | mucin 1, transmembrane | Hs.89603 |
| 202218_s_at | fatty acid desaturase 2 | Hs.184641 |
| 207175_at | adipose most abundant gene transcript 1 | Hs.80485 |
| 205798_at | interleukin 7 receptor | Hs.362807 |
| 200916_at | transgelin 2 | Hs.406504 |
| 216623_x_at | trinucleotide repeat containing 9 | Hs.110826 |
| 211776_s_at | erythrocyte membrane protein band 4.1-like 3 | Hs.103839 |
| 204472_at | GTP binding protein overexpressed in skeletal muscle | Hs.79022 |
| 220149_at | hypothetical protein FLJ22671 | Hs.193745 |

(continued)

| Probe ID | Gene Description | Unigene ID |
|---|---|---|
| 219517_at | hypothetical protein FLJ22637 | Hs.296178 |
| 208653_s_at | CD164 antigen, sialomucin | Hs.43910 |
| 202457_s_at | protein phosphatase 3 (formerly 2B), catalytic subunit, alpha isoform (calcineurin A alpha) | Hs.272458 |
| 222108_at | --- | --- |
| 200648_s_at | glutamate-ammonia ligase (glutamine synthase) | Hs.170171 |
| 203287_at | ladinin 1 | Hs.18141 |
| 219429_at | fatty acid hydroxylase | Hs.249163 |
| 212934_at | Homo sapiens cDNA FLJ30096 fis, clone BNGH41000045, mRNA sequence | Hs.155572 |
| 205307_s_at | kynurenine 3-monooxygenase (kynurenine 3-hydroxylase) | Hs.107318 |
| 212686_at | KIAA1157 protein | Hs.21894 |
| 204623_at | trefoil factor 3 (intestinal) | Hs.82961 |
| 209459_s_at | NPD009 protein | Hs.283675 |
| 203827_at | hypothetical protein FLJ10055 | Hs.9398 |
| 201952_at | activated leukocyte cell adhesion molecule | Hs.10247 |
| 202047_s_at | chromobox homolog 6 | Hs.107374 |
| 206036_s_at | v-rel reticuloendotheliosis viral oncogene homolog (avian) | Hs.44313 |
| 205048_s_at | phosphoserine phosphatase-like | Hs.369508 |
| 211527_x_at | vascular endothelial growth factor | Hs.73793 |
| 202660_at | minor histocompatibility antigen HA-1 | Hs.196914 |
| 210495_x_at | fibronectin 1 | Hs.287820 |
| 216442_x_at | fibronectin 1 | Hs.287820 |
| 212865_s_at | collagen, type XIV, alpha 1 (undulin) | Hs.403836 |
| 221765_at | UDP-glucose ceramide glucosyltransferase | Hs.432605 |
| 210538_s_at | baculoviral IAP repeat-containing 3 | Hs.127799 |
| 204151_x_at | aldo-keto reductase family 1, member C1 (dlhydrodiol dehydrogenase 1; 20-alpha (3-alpha)-hydroxysteroid dehydrogenase) | Hs.306098 |
| 213836_s_at | hypothetical protein FLJ10055 | Hs.9398 |
| 202724_s_at | forkhead box O1A (rhabdomyosarcoma) | Hs.170133 |
| 202404_s_at | collagen, type I, alpha 2 | Hs.179573 |
| 202871_at | TNF receptor-associated factor 4 | Hs.8375 |
| 204455_at | bullous pemphigoid antigen 1, 230/240kDa | Hs.198689 |
| 203640_at | muscleblind-like protein MBLL39 | Hs.283609 |
| 823_at | chemokine (C-X3-C motif) ligand 1 | Hs.80420 |

(continued)

| Probe ID | Gene Description | Unigene ID |
|---|---|---|
| 214203_s_at | proline dehydrogenase (oxidase) 1 | Hs.343874 |
| 201963_at | fatty-acid-Coenzyme A ligase, long-chain 2 | Hs. 154890 |
| 221730_at | collagen, type V, alpha 2 | Hs.82985 |
| 217047_s_at | family with sequence similarity 13, member A1 | Hs.177664 |
| 203814_s_at | NAD(P)H dehydrogenase, quinone 2 | Hs.73956 |
| 202581_at | heat shock 70kDa protein 1 B | Hs.274402 |
| 218640_s_at | phafin 2 | Hs.29724 |
| 201752_s_at | adducin 3 (gamma) | Hs.324470 |
| 221558_s_at | lymphoid enhancer-binding factor 1 | Hs.44865 |
| 211798_x_at | immunoglobulin lambda joining 3 | Hs.102950 |
| 218400_at | 2'-5'-oligoadenylate synthetase 3, 100kDa | Hs.56009 |
| 203549_s_at | lipoprotein lipase | Hs.180878 |
| 201525_at | apolipoprotein D | Hs.75736 |
| 203207_s_at | likely ortholog of chicken chondrocyte protein with a poly-proline region | Hs.170198 |
| 201397_at | phosphoglycerate dehydrogenase | Hs.3343 |
| 217996_at | pleckstrin homology-like domain, family A, member 1 | Hs.82101 |
| 211479_s_at | 5-hydroxytryptamine (serotonin) receptor 2C | Hs.46362 |
| 213287_s_at | keratin 10 (epidermolytic hyperkeratosis; keratosis palmaris et plantaris) | Hs.99936 |
| 221517_s_at | cofactor required for Sp1 transcriptional activation, subunit 6, 77kDa | Hs.22630 |
| 212775_at | KIAA0657 protein | Hs.6654 |
| 217791_s_at | pyrroline-5-carboxylate synthetase (glutamate gamma-semialdehyde synthetase) | Hs.114366 |
| 215250_at | Homo sapiens cDNA FLJ12140 fis, clone MAMMA1000340, mRNA sequence | Hs.287491 |
| 208733_at | RAB2, member RAS oncogene family | Hs.78305 |
| 219629_at | hypothetical protein FLJ20635 | Hs.265018 |
| 205542_at | six transmembrane epithelial antigen of the prostate | Hs.61635 |
| 208682_s_at | melanoma antigen, family D, 2 | Hs.4943 |
| 218729_at | latexin protein | Hs.109276 |
| 205376_at | inositol polyphosphate-4-phosphatase, type II, 105kDa | Hs.153687 |
| 203953_s_at | claudin 3 | Hs.25640 |
| 206916_x_at | tyrosine aminotransferase | Hs.161640 |

(continued)

| Probe ID | Gene Description | Unigene ID |
|---|---|---|
| 212196_at | Homo sapiens mRNA; cDNA DKFZp564F053 (from clone DKFZp564F053), mRNA sequence | Hs.71968 |
| 211000_s_at | interleukin 6 signal transducer (gp130, oncostatin M receptor) | Hs.82065 |
| 212254_s_at | bullous pemphigoid antigen 1, 230/240kDa | Hs.198689 |
| 204914_s_at | SRY (sex determining region Y)-box 11 | Hs.32964 |
| 221505_at | leucine-rich acidic nuclear protein like | Hs.71331 |
| 208498_s_at | amylase, alpha 1A; salivary | Hs.274376 |
| 201694_s_at | early growth response 1 | Hs.326035 |
| 201936_s_at | eukaryotic translation initiation factor 4 gamma, 3 | Hs.25732 |
| 203090_at | stromal cell-derived factor 2 | Hs.118684 |
| 37117_at | Rho GTPase activating protein 8 | Hs.102336 |
| 202770_s_at | cyclin G2 | Hs.429880 |
| 209522_s_at | carnitine acetyltransferase | Hs.12068 |
| 212451_at | KIAA0256 gene product | Hs.118978 |
| 201839_s_at | tumor-associated calcium signal transducer 1 | Hs.692 |
| 218309_at | hypothetical protein PRO1489 | Hs.197922 |
| 212450_at | KIAA0256 gene product | Hs.118978 |
| 221589_s_at | aldehyde dehydrogenase 6 family, member A1 | Hs.293970 |
| 217281_x_at | immunoglobulin heavy constant gamma 3 (G3m marker) | Hs.300697 |
| 217388_s_at | kynureninase (L-kynurenine hydrolase) | Hs.169139 |
| 203336_s_at | integrin cytoplasmic domain-associated protein 1 | Hs.173274 |
| 217704_x_at | --- | -- |
| 201563_at | sorbitol dehydrogenase | Hs.878 |
| 208151_x_at | DEAD/H (Asp-Glu-Ala-Asp/His) box polypeptide 17, 72kDa | Hs.349121 |
| 217880_at | cell division cycle 27 | Hs.406631 |
| 213229_at | Dicer1, Dcr-1 homolog (Drosophila) | Hs.87889 |
| 219768_at | hypothetical protein FLJ22418 | Hs.36563 |
| 200602_at | amyloid beta (A4) precursor protein (protease nexin-II, Alzheimer disease) | Hs.177486 |
| 201082_s_at | dynactin 1 (p150, glued homolog, Drosophila) | Hs.74617 |
| 214774_x_at | trinucleotide repeat containing 9 | Hs.110826 |
| 208654_s_at | CD164 antigen, sialomucin | Hs.43910 |

(continued)

| Probe ID | Gene Description | Unigene ID |
|---|---|---|
| 202018_s_at | lactotransferrin | Hs.105938 |
| 212915_at | likely ortholog of mouse semaF cytoplasmic domain associated protein 3 | Hs.177635 |
| 202196_s_at | dickkopf homolog 3 (Xenopus laevis) | Hs.4909 |
| 221024_s_at | solute carrier family 2 (facilitated glucose transporter), member 10 | Hs.305971 |
| 211702_s_at | ubiquitin specific protease | Hs.155787 |
| 205110_s_at | fibroblast growth factor 13 | Hs.6540 |
| 219956_at | UDP-N-acetyl-alpha-D-galactosamine: polypeptide N-acetylgalactosaminyltransferase 6 (GalNAc-T6) | Hs.151678 |
| 202687_s_at | tumor necrosis factor (ligand) superfamily, member 10 | Hs.83429 |
| 205882_x_at | adducin 3 (gamma) | Hs.324470 |
| 203476_at | trophoblast glycoprotein | Hs.82128 |
| 208991_at | Homo sapiens cDNA FLJ35646 fis, clone SPLEN2012743, mRNA sequence | Hs.381933 |
| 204866_at | KIAA0215 gene product | Hs.82292 |
| 208180_s_at | H4 histone family, member H | Hs.421737 |
| 219410_at | hypothetical protein FLJ10134 | Hs.104800 |
| 209290_s_at | nuclear factor I/B | Hs.33287 |
| 202718_at | insulin-like growth factor binding protein 2, 36kDa | Hs.433326 |
| 205862_at | GREB1 protein | Hs.193914 |
| 203895_at | Homo sapiens mRNA; cDNA DKFZp434E235 (from clone DKFZp434E235), mRNA sequence | Hs.348724 |
| 212171_x_at | vascular endothelial growth factor | Hs.73793 |
| 217762_s_at | RAB31, member RAS oncogene family | Hs.223025 |
| 208891_at | dual specificity phosphatase 6 | Hs.180383 |
| 221543_s_at | chromosome 8 open reading frame 2 | Hs.125849 |
| 218834_s_at | hypothetical protein FLJ20539 | Hs.118552 |
| 201852_x_at | collagen, type III, alpha 1 (Ehlers-Danlos syndrome type IV, autosomal dominant) | Hs.119571 |
| 211965_at | zinc finger protein 36, C3H type-like 1 | Hs.85155 |
| 202015_x_at | methionyl aminopeptidase 2 | Hs.78935 |
| 203348_s_at | ets variant gene 5 (ets-related molecule) | Hs.43697 |
| 202783 at | nicotinamide nucleotide transhydrogenase | Hs.18136 |

(continued)

| Probe ID | Gene Description | Unigene ID |
|---|---|---|
| 202403_s_at | collagen, type I, alpha 2 | Hs.179573 |
| 214440_at | N-acetyltransferase 1 (arylamine N-acetyltransferase) | Hs.155956 |
| 211748_x_at | prostaglandin D2 synthase 21kDa (brain) | Hs.8272 |
| 215073_s_at | Homo sapiens, clone IMAGE:5287010, mRNA, mRNA sequence | Hs.288869 |
| 215806_x_at | T cell receptor gamma constant 2 | Hs.274509 |
| 205158_at | ribonuclease, RNase A family, 4 | Hs.283749 |
| 221841 s_at | Homo sapiens cDNA FLJ38575 fis, clone HCHON2007046, mRNA sequence | Hs.376206 |
| 214858_at | Homo sapiens clone 24566 mRNA sequence | Hs.133342 |
| 212464_s_at | fibronectin 1 | Hs.287820 |
| 206510 at | sine oculis homeobox homolog 2 (Drosophila) | Hs.101937 |
| 216246_at | ribosomal protein S20 | Hs.173717 |
| 200923_at | lectin, galactoside-binding, soluble, 3 binding protein | Hs.79339 |
| 221989_at | ribosomal protein L10 | Hs.29797 |
| 211284_s_at | granulin | Hs.180577 |
| 209173 at | anterior gradient 2 homolog (Xenepus laevis) | Hs.91011 |
| 200924_s_at | solute carrier family 3 (activators of dibasic and neutral amino acid transport), member 2 | Hs.79748 |
| 212859_x_at | -- | --- |
| 213109_at | KIAA0551 protein | Hs.170204 |

**Table A3: WT (Wilcoxon Test) : At a P-value of <0.05 and a >=2-fold change cutoff, a total of 38 genes were identified. This 38 gene set delivered a LOOCV accuracy of 80%. The genes are ranked by their significance (P-value).**

| Probe | Gene Description | Unigene |
|---|---|---|
| 210761_s_at | growth factor receptor-bound protein 7 | Hs.86859 |
| 201931_at | electron-transfer-flavoprotein, alpha polypeptide (glutaric aciduria II) | Hs.169919 |
| 219429_at | fatty acid hydroxylase | Hs.249163 |
| 204285_s_at | phorbol-12-myristate-13-acetate-induced protein 1 | Hs.96 |
| 209603_at | GATA binding protein 3 | Hs.169946 |

(continued)

| Probe | Gene Description | Unigene |
|---|---|---|
| 206165_s_at | chloride channel, calcium activated, family member 2 | Hs.241551 |
| 216836_s_at | v-erb-b2 erythroblastic leukemia viral oncogene homolog 2, neuro/ glioblastoma derived oncogene homolog (avian) | Hs.323910 |
| 203627_at | Human insulin-like growth factor 1 receptor mRNA, 3' sequence, mRNA sequence | Hs.405998 |
| 205225_at | estrogen receptor 1 | Hs.1657 |
| 215465_at | ATP-binding cassette, sub-family A (ABC1), member 12 | Hs.134585 |
| 203628_at | Human insulin-like growth factor 1 receptor mRNA, 3' sequence, mRNA sequence | Hs.405998 |
| 202991_at | START domain containing 3 | Hs.77628 |
| 208891_at | dual specificity phosphatase 6 | Hs.180383 |
| 214451_at | transcription factor AP-2 beta (activating enhancer binding protein 2 beta) | Hs.33102 |
| 204508_s_at | hypothetical protein FLJ20151 | Hs.279916 |
| 202376_at | serine (or cysteine) proteinase inhibitor, clade A (alpha-1 antiproteinase, antitrypsin), member 3 | Hs.234726 |
| 200832_s_at | stearoyl-CoA desaturase (delta-9-desaturase) | Hs.119597 |
| 205307_s_at | kynurenine 3-monooxygenase (kynurenine 3-hydroxylase) | Hs.107318 |
| 203060_s_at | 3'-phosphoadenosine 5'-phosphosulfate synthase 2 | Hs.274230 |
| 201963_at | fatty-acid-Coenzyme A ligase, long-chain 2 | Hs.154890 |
| 209602_s_at | GATA binding protein 3 | Hs.169946 |
| 211138_s_at | kynurenine 3-monooxygenase (kynurenine 3-hydroxylase) | Hs.107318 |
| 39248_at | aquaporin 3 | Hs.234642 |
| 220149_at | hypothetical protein FLJ22671 | Hs.193745 |
| 55616_at | hypothetical gene MGC9753 | Hs.91668 |
| 205306_x_at | kynurenine 3-monooxygenase (kynurenine 3-hydroxylase) | Hs.107318 |
| 205862_at | GREB1 protein | Hs.193914 |
| 217388_s_at | kynureninase (L-kynurenine hydrolase) | Hs.169139 |

(continued)

| Probe | Gene Description | Unigene |
|---|---|---|
| 204942_s_at | aldehyde dehydrogenase 3 family, member B2 | Hs.87539 |
| 202218_s_at | fatty acid desaturase 2 | Hs.184641 |
| 213557_at | ESTs, Weakly similar to ubiquitously transcribed tetratricopeptide repeat gene, Y chromosome; Ubiquitously transcribed TPR gene on Y chromosome [Homo sapiens] [H.sapiens] | Hs.14691 |
| 211657_at | carcinoembryonic antigen-related cell adhesion molecule 6 (non-specific cross reacting antigen) | Hs.73848 |
| 214598_at | claudin 8 | Hs.162209 |
| 218532_s_at | hypothetical protein FLJ20152 | Hs.82273 |
| 202917_s_at | S100 calcium binding protein A8 (calgranulin A) | Hs.100000 |
| 208792_s_at | clusterin (complement lysis inhibitor, SP-40,40, sulfated glycoprotein 2, testosterone-repressed prostate message 2, apolipoprotein J) | Hs.75106 |
| 215659_at | Homo sapiens cDNA: FLJ21521 fis, clone COL05880, mRNA sequence | Hs.306777 |
| 201525_at | apolipoprotein D | Hs.75736 |

**Table A4: 13 'common' genes among the three gene sets (SAM-88, GR-251, WT-38) were then identified. This 13 member gene achieved a classification accuracy of 84% by LOOCV. In essence, these 13 'common genes' are robust significant markers and can archive comparable performance as other 'complete' marker sets.**

| Probe_ID | Unigene | Full Length Ref. Sequences | Location |
|---|---|---|---|
| 39248_at | Hs.234642 | NM 004925 // aquaporin 3 | Chr:9p13 |
| 201525_at | Hs.75736 | NM 001647 // apolipoprotein D precursor | Chr:3q26.2-qter |
| 202991_at | Hs.77628 | NM 006804 // steroidogenic acute regulatory protein related | Chr:17q11-q12 |
| 203628_at | Hs.405998 | --- | --- |
| 205307_s_at | Hs.107318 | NM 003679 // kynurenine 3-monooxygenase (kynurenine 3-hydroxylase) | Chr:1q42-q44 |

(continued)

| Probe_ID | Unigene | Full Length Ref. Sequences | Location |
|---|---|---|---|
| 210761_s_at | Hs.86859 | NM 005310 // growth factor receptor-bound protein 7 cell adhesion molecule 6 (non-specific cross reacting | Chr-17q21.1 |
| 211657_at | Hs.73848 | NM_002483 // carcinoembryonic antigen-related antigen) | Chr:19q13.2 |
| 213557_at | Hs.14691 | --- | --- |
| 214451_at | Hs.33102 | NM 003221 // transcription factor AP-2 beta (activating enhancer binding protein member 12 isoform b /// NM_ 173076 // ATP-binding 2 beta) | Chr:6p12 |
| 215465_at | Hs.134585 | NM_015657 // ATP-binding cassette, sub-family A, cassette, sub-family A, member 12 isoform a | Chr:2q35 |
| 219429_at | Hs.249163 | --- | Chr:16q23 |
| 220149_at | Hs.193745 | NM 024861 // hypothetical protein FLJ22671 derived | Chr:2q37.3 |
| 210930_s_at | Hs.323910 | NM_004448 // v-erb-b2 erythroblastic leukemia viral oncogene homolog 2, neuro/glioblastoma oncogene homolog | Chr: 17q11.2-q12 |

**Table L1: Look-up ID table for SAM-133 Genes**

**SAM-133**

| Rank | Probe_ID | Unigene | GenBank |
|---|---|---|---|
| 1 | 205225_at | Hs.1657 | NM_000125.1 |
| 2 | 209603_at | Hs.169946 | AI796169 |
| 3 | 204508_s_at | Hs.279916 | BC001012.1 |
| 4 | 209604_s_at | Hs.169946 | BC003070.1 |
| 5 | 209602_s_at | Hs.169946 | AI796169 |
| 6 | 206754_s_at | Hs.1360 | NM_000767.2 |
| 7 | 203963_at | Hs.5338 | NM_001218.2 |
| 8 | 214164_x_at | Hs.5344 | BF752277 |
| 9 | 212956_at | Hs.90419 | AI348094 |
| 10 | 215867_x_at | Hs.5344 | AL050025.1 |
| 11 | 210735_s_at | Hs.5338 | BC000278.1 |
| 12 | 214440_at | Hs.155956 | NM_000662.1 |
| 13 | 202089_s_at | Hs.79136 | NM_012319.2 |
| 14 | 210085_s_at | Hs.279928 | AF230929.1 |

(continued)

| SAM-133 | | | |
|---|---|---|---|
| **Rank** | **Probe_ID** | **Unigene** | **GenBank** |
| 15 | 205862_at | Hs.193914 | NM_014668.1 |
| 16 | 202088_at | Hs.79136 | AI635449 |
| 17 | 211712_s_at | | BC005830.1 |
| 18 | 206401_s_at | Hs.101174 | J03778.1 |
| 19 | 215304_at | Hs.159264 | U79293.1 |
| 20 | 218195_at | Hs.15929 | NM_024573.1 |
| 21 | 212195_at | Hs.71968 | AL049265.1 |
| 22 | 203928_x_at | Hs.101174 | AI870749 |
| 23 | 209460_at | Hs.283675 | AF237813.1 |
| 24 | 212960_at | Hs.90419 | BE646554 |
| 25 | 209443_at | Hs.76353 | J02639.1 |
| 26 | 209173_at | Hs.91011 | AF088867.1 |
| 27 | 203071_at | Hs.82222 | NM_004636.1 |
| 28 | 203571_s_at | Hs.74120 | NM_006829.1 |
| 29 | 205354_at | Hs.81131 | NM_000156.3 |
| 30 | 213712_at | Hs.30504 | BF508639 |
| 31 | 41660_at | | |
| 32 | 220744_s_at | Hs.70202 | NM_018262.1 |
| 33 | 204798_at | Hs.1334 | NM 005375.1 |
| 34 | 215552_s_at | Hs.272288 | A1073549 |
| 35 | 209339_at | Hs.20191 | U76248.1 |
| 36 | 210272_at | Hs.330780 | M29873.1 |
| 37 | 205186_at | Hs.33846 | NM_003462.2 |
| 38 | 207414_s_at | Hs.170414 | NM_002570.1 |
| 39 | 205009_at | Hs.1406 | NM_003225.1 |
| 40 | 203628_at | Hs.239176 | H05812 |
| 41 | 211323_s_at | Hs.198443 | L38019.1 |
| 42 | 201825_s_at | Hs.238126 | AL572542 |
| 43 | 211234_x_at | Hs.1657 | AF258449.1 |
| 44 | 209459_s_at | Hs.283675 | AF237813.1 |
| 45 | 212196_at | Hs.71968 | AW242916 |
| 46 | 203438_at | Hs.155223 | AI435828 |
| 47 | 217838_s_at | Hs.241471 | NM_016337.1 |
| 48 | 204041_at | Hs.82163 | NM_000898.1 |
| 49 | 203929_s_at | Hs.101174 | AI056359 |
| 50 | 200670_at | Hs.149923 | NM-005080.1 |
| 51 | 219414_at | Hs.12079 | NM_022131.1 |
| 52 | 203627_at | Hs.239176 | AI830698 |
| 53 | 208451_s_at | Hs.278625 | NM_000592.2 |
| 54 | 213419_at | Hs.324125 | U62325.1 |
| 55 | 205768_s_at | Hs.11729 | NM_003645.1 |
| 56 | 204862_s_at | Hs.81687 | NM_002513.1 |
| 57 | 210480_s_at | Hs.22564 | U90236.2 |
| 58 | 205696_s_at | Hs.105445 | NM_005264.1 |
| 59 | 203685_at | Hs.79241 | NM-000633.1 |
| 60 | 218976_at | Hs.260720 | NM_021800.1 |
| 61 | 219197_s_at | Hs.222399 | AI424243 |
| 62 | 202996_at | Hs.82520 | NM_021173.1 |
| 63 | 205734_s_at | Hs.38070 | AI990465 |

(continued)

| SAM-133 | | | |
|---|---|---|---|
| Rank | Probe_ID | Unigene | GenBank |
| 64 | 211235_s_at | Hs.1657 | AF258450.1 |
| 65 | 211000_s_at | Hs.82065 | AB015706.1 |
| 66 | 217190_x_at | Hs.247976 | S67777 |
| 67 | 202752_x_at | Hs.22891 | NM_012244.1 |
| 68 | 201754_at | Hs.74649 | NM_004374.1 |
| 69 | 204623_at | Hs.82961 | NM_003226,1 |
| 70 | 207038_at | Hs.114924 | NM_004694.1 |
| 71 | 212637_s_at | Hs.324275 | AU155187 |
| 72 | 208682_s_at | Hs.4943 | AF126181.1 |
| 73 | 218502_s_at | Hs.26102 | NM_014112.1 |
| 74 | 202376_at | Hs.234726 | NM_001085.2 |
| 75 | 215616_s_at | Hs.301011 | AB020683.1 |
| 76 | 211233_x_at | Hs.1657 | M12674.1 |
| 77 | 205081_at | Hs.17409 | M_001311.1 |
| 78 | 21442B_x_at | Hs.170250 | K02403.1 |
| 79 | 209696_at | Hs.574 | D26054.1 |
| 80 | 219682_s_at | Hs.332150 | NM_016569.1 |
| 81 | 212496_s_at | Hs.301011 | BE256900 |
| 82 | 203108_at | Hs.194691 | NM_003979.2 |
| 83 | 206107_at | Hs.65756 | NM_003834.1 |
| 84 | 218806_s_at | Hs.267659 | AF118887.1 |
| 85 | 209581_at | Hs.37189 | BC001387.1 |
| 86 | 213412_at | Hs.25527 | NM_014428.1 |
| 87 | 212638_s_at | Hs.324275 | BF131791 |
| 88 | 206469_x_at | Hs.284236 | NM_012067.1 |
| 89 | 210652_s_at | Hs.125783 | BC004399.1 |
| 90 | 216381_x_at | Hs.284236 | AL035413 |
| 91 | 216092_s_at | Hs.22891 | AL365347. |
| 92 | 208788_at | Hs.250175 | AL136939.1 |
| 93 | 204792_s_at | Hs.111862 | NM_014714.1 |
| 94 | 207847_s_at | Hs.89603 | NM_002456.1 |
| 95 | 213201_s_at | Hs.73980 | AJ011712 |
| 96 | 204497_at | Hs.20196 | AB011092.1 |
| 97 | 222314_x_at | Hs.205660 | AW970881 |
| 98 | 222212_s_at | Hs.285976 | AK001105.1 |
| 99 | 219919_s_at | Hs.279808 | NM_018276.1 |
| 100 | 214053_at | Hs.7888 | AW772192 |
| 101 | 204934_s_at | Hs.823 | NM_002151.1 |
| 102 | 216109_at | Hs.306803 | AK025348.1 |
| 103 | 203749_s_at | Hs.250505 | AI806984 |
| 104 | 220329_s_at | Hs.238270 | NM_017909.1 |
| 105 | 204881_s_at | Hs.152601 | NM_003358.1 |
| 106 | 208305_at | Hs.2905 | NM_000926.1 |
| 107 | 209623_at | Hs.167531 | AW439494 |
| 108 | 218450_at | Hs.108675 | NM_015987.1 |
| 109 | 204343_at | Hs.26630 | NM_001089.1 |
| 110 | 219051_x_at | Hs.124915 | NM_024042.1 |
| 111 | 205471_s_at | Hs.63931 | AW772082 |
| 112 | 203439_s_at | Hs.155223 | BC000658.1 |

(continued)

| SAM-133 | | | |
|---|---|---|---|
| Rank | Probe_ID | Unigene | GenBank |
| 113 | 204863_s_at | Hs.82065 | BE856546 |
| 114 | 203289_s_at | Hs.19699 | BE791629 |
| 115 | 221765_at | Hs.23703 | AI378044 |
| 116 | 219001_s_at | Hs.317589 | NM_024345.1 |
| 117 | 220581_at | Hs.287738 | NM_025059.1 |
| 118 | 211596_s_at | | AB050468.1 |
| 119 | 205645_at | Hs.80657 | NM_004726.1 |
| 120 | 219663_s_at | Hs.157527 | NM_025268,1 |
| 121 | 205380_at | Hs.15456 | NM_002614.1 |
| 122 | 201508_at | Hs.1516 | NM_001552.1 |
| 1 | 215729_s_at | Hs.9030 | BE542323 |
| 2 | 201983_s_at | Hs.77432 | AW157070 |
| 3 | 204914_s_at | Hs.32964 | AW157202 |
| 4 | 204913_s_at | Hs.32964 | AI360875 |
| 5 | 205646_5_at | Hs.89506 | NM_000280,1 |
| 6 | 207030_s_at | Hs.10526 | NM_001321.1 |
| 7 | 204915_s_at | Hs.32964 | AB028641.1 |
| 8 | 203021_at | Hs.251754 | NM_003064.1 |
| 9 | 209800_at | Hs.115947 | AF061812.1 |
| 10 | 203234_at | Hs.77573 | NM_003364.1 |
| 11 | 201984_s_at | Hs.77432 | Mum_005228.1 |

Table L2: Lookup table for Table 2 genes

**Table 2**

| Probe-ID | Unigene | GenBank |
|---|---|---|
| 205225_at | Hs.1657 | NM_000125.1 |
| 205186_at | Hs.406050 | NM_003462.2 |
| 201754_at | Hs.351875 | NM_004374.1 |
| 210085_5_at | Hs.279928 | AF230929.1 |
| 214440_at | Hs.155956 | NM_000662.1 |
| 206754_s_at | Hs.1360 | NM_000767.2 |
| 203749_s_at | Hs.361071 | AI806984 |
| 215552_s_at | Hs.239176 | AI073549 |
| 209443_at | Hs.76353 | J02639.1 |
| 216109_at | Hs.306803 | AK025348.1 |
| 203685_at | Hs.79241 | NM_000633.1 |
| 205862_at | Hs.193914 | NM_014668.1 |
| 217838_s_at | Hs.241471 | NM_016337.1 |
| 209603_at | Hs.169946 | AI796169 |
| 212195_at | Hs.71968 | AL049265.1 |
| 212637_s_at | Hs.355977 | AU155187 |
| 205696_s_at | Hs.105445 | NM_005264.1 |
| 210652_s_at | Hs.125783 | BC004399.1 |
| 205734_s_at | Hs.38070 | AI990465 |
| 211000_s_at | Hs.82065 | AB015706.1 |

(continued)

| Probe-ID | Unigene | GenBank |
|---|---|---|
| 206107_at | Hs.65756 | NM_003834.1 |
| 203628_at | Hs.405998 | H05812 |
| 204934_s_at | Hs.823. | NM_002151.1 |
| 203071_at | Hs.82222 | NM_004636.1 |
| 204881_s_at | Hs.432605 | NM_003358.1 |
| 210272_at | Hs.330780 | M29873.1 |
| 213201_s_at | Hs.73980 | AJ011712 |
| 206401_s_at | Hs.101174 | J03778.1 |
| 209339_at | Hs.20191 | U76248.1 |
| 208305_at | Hs.2905 | NM_000926.1 |
| 212956_at | Hs.90419 | AI348094 |
| 214164_x_at | Hs.279916 | BF752277 |
| 204343_at | Hs.26630 | NM_001089.1 |
| 203963_at | Hs.5338 | NM_001218.2 |
| 207038_at | Hs.114924 | NM_004694.1 |
| 218195_at | Hs.15929 | NM_024573.1 |
| 220329_s_at | Hs.238270 | NM_017909.1 |
| 218502_s_at | Hs.26102 | NM_014112.1 |
| 219414_at | Hs.12079 | NM_022131.1 |
| 202376_at | Hs.234726 | NM_001085.2 |
| 218806_s_at | Hs.267659 | AF11B887.1 |
| 202089_s_at | Hs.79136 | NM_012319.2 |
| 213712_at | Hs.432587 | BF508639 |
| 204497_at | Hs.20196 | AB011092.1 |
| 215616_s_at | Hs.301011 | AB020683.1 |
| 218450_at | Hs.294133 | NM_015987.1 |
| 203438_at | Hs.155223 | AI435828 |
| 208451_s_at | Hs.433721 | NM_000592.2 |
| 205768_s_at | Hs.11729 | NM_003645.1 |
| 219682_s_at | Hs.267182 | NM_016569.1 |
| | | |
| 204508_s_at | Hs.279916 | BC001012.1 |
| 203963_at | Hs.5338 | NM_001218.2 |
| 209603_at | Hs.169946 | AI796169 |
| 208788_at | Hs.250175 | AL136939.1 |
| 212637_s_at | Hs.355977 | AU155187 |
| 200670_at | Hs.149923 | NM_005080.1 |
| 203571_s_at | Hs.74120 | NM_006829.1 |
| 208682_s_at | Hs.4943 | AF126181.1 |
| 209173_at | Hs.91011 | AF088867.1 |
| 201754_at | Hs.351875 | NM_004374.1 |
| 206469_x_at | Hs.284236 | NM_012067.1 |
| 213412_at | Hs.25527 | NM_014428.1 |
| 222212_s_at | Hs.285976 | AK001105.1 |
| 211323_s_at | Hs.198443 | L38019.1 |
| 209696_at | Hs.574 | D26054.1 |
| 212956_at | Hs.90419 | AI348094 |
| 218195_at | Hs.15929 | NM_024573.1 |
| 202089_s_at | Hs.79136 | NM_012319.2 |
| 209623_at | Hs.167531 | AW439494 |

(continued)

| Probe-ID | Unigene | GenBank |
|---|---|---|
| 210272_at | Hs.330780 | M29873.1 |
| 204623_at | Hs.82961 | NM_003226.1 |
| 215304_at | Hs.159264 | U79293.1 |
| 214440_at | Hs.155956 | NM_000662.1 |
| 205862_at | Hs.193914 | NM_014668.1 |
| 203108_at | Hs.194691 | NM_003979.2 |
| 207038_at | Hs.114924 | NM_004694.1 |
| 205186_at | Hs.406050 | NM_003462.2 |
| 202752_x_at | Hs.22891 | NM_012244.1 |
| 220744_s_at | Hs.70202 | NM_018262.1 |
| 219414_at | Hs.12079 | NM_022131.1 |
| 204798_at | Hs.1334 | NM_005375.1 |
| 205009_at | Hs.350470 | NM_003225.1 |
| 219051_x_at | Hs.124915 | NM_024042.1 |
| 205471_s_at | Hs.63931 | AW772082 |
| 207847_s_at | Hs.89603 | NM_002456.1 |
| 208451_s_at | Hs.433721 | NM_000592.2 |
| 205081_at | Hs.423190 | NM_001311.1 |
| 209459_s_at | Hs.283675 | AF237813.1 |
| 203071_at | Hs.82222 | NM_004636.1 |
| 209581_at | Hs.37189 | BC001387.1 |
| 204343_at | Hs.26630 | NM_001089.1 |
| 206401_s_at | Hs.101174 | J03778.1 |
| 210480_s_at | Hs.385834 | U90236.2 |
| 201825_s_at | Hs.238126 | AL572542 |
| 203749_s_at | Hs.361071 | AI806984 |
| 218806_s_at | Hs.267659 | AF118887.1 |
| 210652_s_at | Hs.125783 | BC004399.1 |
| 205225_at | Hs.1657 | NM_000125.1 |
| 205768_s_at | Hs.11729 | NM_003645.1 |
| 219682_s_at | Hs.332150 | NM_016569.1 |

**Table L3: Look up table for Table S4 Genes**

| Unigene | GenBank |
|---|---|
| Hs.106642 | BF589529 |
| Hs.25960 | AF320053.1 |
| Hs.1892 | NM_002686.1 |
| Hs.289104 | NM_014274.1 |
| Hs.165950 | NM_002011.2 |
| Hs.173035 | AF338650.1 |
| Hs.86859 | AB008790.1 |
| Hs.272207 | NM_017533.1 |
| Hs.103707 | AW192795 |
| Hs.274550 | AA074145 |
| Hs.100000 | AW238654 |
| Hs.54609 | NM_014291.1 |
| Hs.85050 | NM_002667.1 |
| Hs.239934 | AL022316 |
| Hs.194236 | NM_000230.1 |

(continued)

| Unigene | GenBank |
|---|---|
| Hs.103395 | NM_024709.1 |
| Hs.107318 | NM_003679.1 |
| Hs.1735 | NM_002193.1 |
| Hs.155109 | NM_002153.1 |
| Hs.26770 | NM_001446.1 |
| Hs.278388 | NM_000608.1 |
| Hs.251754 | NM_003064.1 |
| Hs.378774 | NM_001615.2 |
| Hs.51515 | AA053967 |
| Hs.149195 | NM_016233.1 |
| Hs.78344 | AI889739 |
| Hs.112405 | NM_002965.2 |
| Hs.417091 | AF052117.1 |
| Hs.57664 | NM_000888.3 |
| Hs.154078 | NM_004139.1 |
| Hs.100014 | NM_007325.1 |
| Hs.193606 | AA343027 |
| Hs.202949 | AK027231.1 |
| Hs.84072 | NM_004616.1 |
| Hs.323910 | AF177761.2 |
| Hs.76780 | NM_006741.1 |
| Hs.225962 | NM_014354.1 |
| Hs.165619 | NM_017717.2 |
| Hs.127428 | AI246769 |
| Hs.2B99 | NM_002150.1 |
| Hs.105938 | NM_002343.1 |
| Hs.193143 | AK022610.1 |
| Hs.1915 | NM_004476.1 |
| Hs.160786 | NM_000050.1 |
| Hs.23881 | AI920979 |
| Hs.3110 | NM_000686.2 |
| Hs.180142 | NM_017422.2 |
| Hs.169919 | NM_000126.1 |
| Hs.112408 | NM_002963.2 |
| Hs.96 | NM_021127.1 |
| Hs.33846 | NM_003462.2 |
| Hs.1360 | NM_000767.2 |
| Hs.1657 | NM_000125.1 |
| Hs.194689 | AF120274.1 |
| Hs.50964 | NM_001712.1 |
| Hs.23703 | BF970427 |
| Hs.193914 | NM_014668.1 |
| Hs.250505 | AI806984 |
| Hs.279928 | AF230929.1 |
| Hs.156637 | NM_012116.1 |
| Hs.169946 | AI796169 |
| Hs.4243 | NM_024522.1 |
| Hs.111801 | NM_015908.1 |
| Hs.155485 | NM_005339.2 |

(continued)

| Unigene | GenBank |
|---|---|
| Hs.99603 | NM_024701.1 |
| Hs.55481 | NM_003447.1 |
| Hs.306803 | AK025348.1 |
| Hs.239176 | NM_000875.2 |
| Hs.823 | NM_002151.1 |
| Hs.203845 | NM_022358.1 |
| Hs.432605 | NM_003358.1 |
| Hs.330780 | M29873.1 |
| Hs.32981 | U38276 |
| Hs.101174 | NM_016835.1 |
| Hs.17752 | NM_015900.1 |
| Hs.406646 | Data not found |
| Hs.351875 | NM_004374,1 |
| Hs.20196 | AB011092.1 |
| Hs.331584 | AF326966.1 |
| Hs.272288 | AI073549 |
| Hs.12079 | NM_022131.1 |
| Hs.82065 | NM_002184.1 |
| Hs.372446 | NM_007202.1 |
| Hs.155956 | NM_000662.1 |
| Hs.278850 | NM_024935.1 |
| Hs.247955 | NM_001322.1 |
| Hs.76067 | NM_001540.2 |
| Hs.61289 | AL157424.1 |
| | |
| **UniGene** | |
| Hs.334514 | NM_032794 |
| Hs.4943 | NM_177433 |
| Hs.1892 | NM_002686 |
| Hs.321576 | NM_006458 |
| Hs.91668 | BF033007 |
| Hs.274260 | NM_001171 |
| Hs.14368 | NM_003022 |
| Hs.86859 | NM_005310 |
| Hs.59889 | NM_005518 |
| Hs.165950 | NM_002011 |
| Hs.83190 | NM_004104 |
| Hs.89603 | NM_002456 |
| Hs.29724 | NM_024613.1 |
| Hs.12068 | NM_000755 |
| Hs.279916 | NM_017689 |
| Hs.169946 | NM_002051 |
| Hs.355977 | NM_007013 |
| Hs.33102 | NM_003221 |
| Hs.90419 | XM_093895 |
| Hs.38972 | NM_005727 |
| Hs.31034 | NM_003847 |
| Hs.132136 | NM_004858 |
| Hs.91668 | BF033007 |

(continued)

| UniGene | |
|---|---|
| Hs.70604 | NM_004496 |
| Hs.234642 | NM_004925 |
| Hs.323910 | NM_004448 |
| Hs.198443 | NM_002222 |
| Hs.197922 | NM_018584.1 |
| Hs.87539 | NM_000695 |
| Hs.381412 | Data not found |
| Hs.180383 | NM_001946 |
| Hs.5338 | NM_001218 |
| Hs.406515 | NM_000903 |
| Hs.8910 | NM_020379 |
| Hs.6168 | NM_014861 |
| Hs.119597 | NM_005063 |
| Hs.574 | NM_000507 |
| Hs.326525 | NM_009589 |
| Hs149923 | NM_005080 |
| Hs.167531 | NM_022132 |
| Hs.184376 | NM_003825 |
| Hs.301947 | NM_014509 |
| Hs.91011 | NM_006408 |
| Hs.114556 | NM_017699 |
| Hs.432970 | NM_006431 |
| Hs.300697 | AK090461 |
| Hs.84072 | NM_004616 |
| Hs.878 | NM_003104 |

## Claims

**1.** A method for classifying a breast tumour sample as "low confidence" or "high confidence", the method comprising providing the expression profile of said breast tumour sample, wherein the expression profile comprises the expression level of the genes from Table S4a and/or the genes from Table S4b, and classifying the tumour as a high or low confidence tumour based on the expression profile.

**2.** A method according to claim 1 comprising determining the estrogen receptor (ER) status of the sample.

**3.** A method according to claim 1 or claim 2 comprising the steps of:

(a) obtaining expression products from a breast tumour sample obtained from a patient;
(b) determining the expression levels of a multi-gene classifier comprising the genes identified in Table S4a and/or the genes identified in Table S4b by contacting said expression products with binding members, each binding member being capable of specifically binding to an expression product of the multi-gene classifier; and
(c) identifying the presence of a low confidence breast tumour in said patient based on the expression levels.

**4.** A method according to claim 3 wherein the expression products are cDNA and the binding members are nucleic acid probes capable of specifically hybridising to the cDNA.

**5.** A method according to claim 3 wherein the expression products are RNA or mRNA and the binding members are nucleic acid primers capable of specifically hybridising to the RNA or mRNA and amplifying them in a PCR.

**6.** A method according to claim 3 wherein the expression products are polypeptides and the binding members are antibody binding domains capable of binding specifically to the polypeptides.

7. A method according to any one of claims 3 to 6 comprising comparing the binding profile of the expression products from the breast tumour sample under test with a database of other previously obtained profiles and/or a previously determined "standard" profile which is characteristic of the presence of low confidence tumour.

8. A method according to claim 7 wherein the comparison is performed by a computer programmed to report the statistical similarity between the profile under test and the standard profiles so that a classification may be made.

9. A method according to any one of claims 1 to 8 wherein the step of classifying the breast tumour sample comprises the use of Weighted Voting, Support Vector Machines and/or Hierarchical Clustering.

10. A method of producing a nucleic acid expression profile for a breast tumour sample comprising the steps of

(a) isolating expression products from said breast tumour sample;
(b) identifying the expression levels of a multi-gene classifier comprising the genes from Table S4a and/or Table S4b; and
(c) producing from the expression levels an expression profile for said breast tumour sample.

11. A method according to claim 10 comprising the steps of

(a) isolating expression products from a breast tumour sample;
(b) contacting said expression products with multi-gene classifier comprising binding members capable of specifically and independently binding to expression products of the genes of Table S4a and/or Table S4b so as to create a first expression profile of a tumour sample from the expression levels of said multi-gene classifier;
(c) comparing the expression profile with an expression profile characteristic of a high confidence tumour and/or a low confidence breast tumour.

12. Apparatus for classifying a breast tumour sample as "high confidence" or "low confidence", comprising a plurality of binding members attached to a solid support, each binding member being capable of specifically binding to expression product of a multi-gene classifier comprising the genes from Table S4a and/or Table S4b, and wherein the solid support houses binding members for no more than 500 different genes.

13. Apparatus according to claim 12 comprising a microarray wherein the binding members are nucleic acid sequences capable of specifically hybridising to RNA or mRNA expression products, or cDNA derived therefrom.

14. A kit for classifying a breast tumour sample as "high confidence" or "low confidence", said kit comprising a plurality of binding members, each binding member being capable of specifically binding to an expression product of one of a multi-gene classifier comprising the genes identified in Table S4a and/or Table S4b and a detection reagent, wherein the binding members are antibody binding domains.

15. A kit according to claim 14 further comprising one or more standard expression profiles retrievably held on a data carrier for comparison with expression profiles of a test sample.

16. A kit according to claim 15 wherein the one or more standard expression profiles are produced according to the method of claim 10 or claim 11.

**Patentansprüche**

1. Verfahren zur Klassifizierung einer Brusttumorprobe als "wenig vertrauenswürdig" oder "sehr vertrauenswürdig", wobei das Verfahren das Bereitstellen des Expressionsprofils der Brusttumorprobe, worin das Expressionsprofil das Expressionslevel der Gene aus Tabelle S4a und/oder der Gene aus Tabelle S4b umfasst, und das Klassifizieren des Tumors auf Basis des Expressionsprofils als sehr oder wenig vertrauenswürdigen Tumor umfasst.

2. Verfahren nach Anspruch 1, welches das Bestimmen des Östrogenrezeptor-(ÖR-) Status der Probe umfasst.

3. Verfahren nach Anspruch 1 oder Anspruch 2, folgende Schritte umfassend:

(a) das Erhalten von Expressionsprodukten aus einer von einem Patienten erhaltenen Brusttumorprobe;

(b) das Bestimmen der Expressionslevels eines Multigenklassifikators, der die in Tabelle S4a identifizierten Gene und/oder die in Tabelle S4b identifizierten Gene umfasst, indem die Expressionsprodukte mit Bindungselementen kontaktiert werden, wobei jedes Bindungselement in der Lage ist, spezifisch an ein Expressionsprodukt des Multigenklassifikators zu binden; und
(c) das Bestimmen der Gegenwart eines wenig vertrauenswürdigen Brusttumors im Patienten auf Basis der Expressionslevels.

**4.** Verfahren nach Anspruch 3, worin die Expressionsprodukte cDNA sind und die Bindungselemente Nucleinsäuresonden sind, die in der Lage sind, spezifisch an die cDNA zu hybridisieren.

**5.** Verfahren nach Anspruch 3, worin die Expressionsprodukte RNA oder mRNA sind und die Bindungselemente Nucleinsäureprimer sind, die in der Lage sind, spezifisch an die RNA oder mRNA zu hybridisieren und sie in einer PCR zu amplifizieren.

**6.** Verfahren nach Anspruch 3, worin die Expressionsprodukte Polypeptide sind und die Bindungselemente Antikörperbindungsdomänen sind, die in der Lage sind, spezifisch an die Polypeptide zu binden.

**7.** Verfahren nach einem der Ansprüche 3 bis 6, welches das Vergleichen der Bindungsprofile der Expressionsprodukte der zu testenden Brusttumorprobe mit einer Datenbank anderer, zuvor erhaltener Profile und/oder einem zuvor bestimmten "Standardprofil", das für die Gegenwart eines wenig vertrauenswürdigen Tumors charakteristisch ist, umfasst.

**8.** Verfahren nach Anspruch 7, worin der Vergleich von einem Computer durchgeführt wird, der programmiert ist, die statistische Ähnlichkeit zwischen dem Testprofil und den Standardprofilen auszugeben, so dass eine Klassifizierung erfolgen kann.

**9.** Verfahren nach einem der Ansprüche 1 bis 8, worin der Schritt des Klassifizierens der Brusttumorprobe den Einsatz von gewichtetem Votieren, Support Vector Machines und/oder hierarchischem Clustern umfasst.

**10.** Verfahren zur Anfertigung eines Nucleinsäure-Expressionsprofils für eine Brusttumorprobe, folgende Schritte umfassend:

(a) das Isolieren von Expressionsprodukten aus der Brusttumorprobe;
(b) das identifizieren der Expressionslevels eines Multigenklassifikators, der die Gene aus Tabelle S4a und/oder Tabelle S4b umfasst; und
(c) das Anfertigen eines Expressionsprofils für die Brusttumorprobe aus den Expressionslevels.

**11.** Verfahren nach Anspruch 10, folgende Schritte umfassend:

(a) das Isolieren von Expressionsprodukten aus einer Brusttumorprobe;
(b) das Kontaktieren der Expressionsprodukte mit Multigenklassifikatoren, die Bindungselemente umfassen, die in der Lage sind, spezifisch und unabhängig voneinander an Expressionsprodukte der Gene aus Tabelle S4a und/oder Tabelle S4b zu binden, um so ein erstes Expressionsprofil einer Tumorprobe aus den Expressionslevels des Multigenklassifikators zu anzufertigen;
(c) das Vergleichen des Expressionsprofils mit einem Expressionsprofil, das charakteristisch für einen sehr vertrauenswürdigen Tumor und/oder einen wenig vertrauenswürdigen Tumor ist.

**12.** Vorrichtung zur Klassifizierung einer Brusttumorprobe als "sehr vertrauenswürdig" oder "wenig vertrauenswürdig", die mehrere Bindungselemente an einen festen Träger gebunden umfasst, wobei jedes Bindungselement in der Lage ist, spezifisch an das Expressionsprodukt eines Multigenklassifikators zu binden, der die Gene aus Tabelle S4a und/oder Tabelle S4b umfasst, und worin der feste Träger Bindungselemente für nicht mehr als 500 unterschiedliche Gene beherbergt.

**13.** Vorrichtung nach Anspruch 12, die einen Mikroarray umfasst, worin die Bindungselemente Nucleinsäuresequenzen sind, die in der Lage sind, spezifisch an RNA- oder mRNA-Expressionsprodukte oder an davon abgeleitete cDNA zu hybridisieren.

**14.** Set zur Klassifizierung einer Brusttumorprobe als "sehr vertrauenswürdig" oder "wenig vertrauenswürdig", wobei das Set mehrere Bindungselemente, wobei jedes Bindungselement in der Lage ist, spezifisch an das Expressionsprodukt eines Multigenklassifikators, der die in Tabelle S4a und/oder Tabelle S4b identifizierten Gene umfasst, zu binden, sowie ein Detektionsreagens umfasst, worin die Bindungselemente Antikörperbindungsdomänen sind.

**15.** Set nach Anspruch 14, das weiters ein oder mehrere von einem Datenträger abrufbare Standardexpressionsprofile zum Vergleich mit den Expressionsprofilen einer Testprobe umfasst.

**16.** Set nach Anspruch 15, worin das eine oder die mehreren Standardexpressionsprofile nach einem Verfahren nach Anspruch 10 oder Anspruch 11 angefertigt ist bzw. sind.

**Revendications**

**1.** Procédé pour classer un échantillon de tumeur mammaire dans la catégorie "à faible probabilité de malignité" ou "à forte probabilité de malignité", le procédé comprenant la fourniture du profil d'expression dudit échantillon de tumeur mammaire, dans laquelle le profil d'expression comprend le niveau d'expression des gènes du tableau S4a et/ou des gènes du tableau S4b, et le classement de la tumeur comme tumeur à forte ou à faible probabilité de malignité sur la base du profil d'expression.

**2.** Procédé selon la revendication 1, comprenant la détermination de l'état de récepteur d'oestrogènes (ER) de l'échantillon.

**3.** Procédé selon la revendication 1 ou la revendication 2, comprenant les étapes consistant à:

(a) obtenir des produits d'expression à partir d'un échantillon de tumeur mammaire prélevé sur une patiente;
(b) déterminer les niveaux d'expression d'un classificateur à gènes multiples comprenant les gènes identifiés au tableau S4a et/ou les gènes identifiés au tableau S4b en mettant lesdits produits d'expression en contact avec des éléments de fixation, chaque élément de fixation étant capable de se fixer de manière spécifique à un produit d'expression du classificateur à gènes multiples; et
(c) identifier la présence d'une tumeur mammaire à faible probabilité de malignité chez ladite patiente sur la base des niveaux d'expression.

**4.** Procédé selon la revendication 3, dans lequel les produits d'expression sont de l'ADNc et les éléments de fixation sont des sondes d'acide nucléique capables de s'hybrider de manière spécifique avec l'ADNc.

**5.** Procédé selon la revendication 3, dans lequel les produits d'expression sont de l'ARN ou de l'ARNm et les éléments de fixation sont des amorces d'acide nucléique capables de s'hybrider de manière spécifique avec l'ARN ou l'ARNm et de les amplifier dans une réaction en chaîne de la polymérase.

**6.** Procédé selon la revendication 3, dans lequel les produits d'expression sont des polypeptides et les éléments de fixation sont des domaines de fixation des anticorps capables de se fixer de manière spécifique aux polypeptides.

**7.** Procédé selon l'une quelconque des revendications 3 à 6, comprenant la comparaison du profil de fixation des produits d'expression issus de l'échantillon de tumeur mammaire analysé avec une base de données d'autres profils précédemment obtenus et/ou avec un profil "standard" précédemment obtenu, caractéristique de la présence d'une tumeur à faible probabilité de malignité.

**8.** Procédé selon la revendication 7, dans lequel la comparaison est effectuée par un ordinateur programmé pour signaler la ressemblance statistique entre le profil analysé et les profils standard de manière à permettre un classement.

**9.** Procédé selon l'une quelconque des revendications 1 à 8, dans lequel l'étape de classement de l'échantillon de tumeur mammaire comprend l'utilisation d'une classification par votes pondérés, de machines à vecteurs de support et/ou d'une classification hiérarchique.

**10.** Procédé pour produire un profil d'expression d'acide nucléique pour un échantillon de tumeur mammaire, comprenant les étapes consistant à:

(a) isoler les produits d'expression dudit échantillon de tumeur mammaire;
(b) identifier les niveaux d'expression d'un classificateur à gènes multiples comprenant les gènes du tableau S4a et/ou du tableau S4b; et
(c) produire, à partir des niveaux d'expression, un profil d'expression dudit échantillon de tumeur mammaire.

**11.** Procédé selon la revendication 10, comprenant les étapes consistant à:

(a) isoler les produits d'expression d'un échantillon de tumeur mammaire;
(b) mettre lesdits produits d'expression en contact avec un classificateur à gènes multiples comprenant des éléments de fixation capables de se fixer de manière spécifique et indépendante aux produits d'expression des gènes du tableau S4a et/ou du tableau S4b de manière à créer un premier profil d'expression d'un échantillon de tumeur à partir des niveaux d'expression dudit classificateur à gènes multiples;
(c) comparer le profil d'expression avec un profil d'expression caractéristique d'une tumeur à forte probabilité de malignité et/ou d'une tumeur mammaire à faible probabilité de malignité.

**12.** Dispositif pour classer un échantillon de tumeur mammaire dans la catégorie "à faible probabilité de malignité" ou "à forte probabilité de malignité", comprenant une pluralité d'éléments de fixation fixés sur un support solide, chaque élément de fixation étant capable de se fixer de manière spécifique à un produit d'expression d'un classificateur à gènes multiples comprenant les gènes du tableau S4a et/ou du tableau S4b, et dans lequel le support solide reçoit les éléments de fixation d'au plus 500 gènes différents.

**13.** Dispositif selon la revendication 12, comprenant un microréseau dans lequel les éléments de fixation sont des séquences d'acide nucléique capables de s'hybrider de manière spécifique avec les produits d'expression de l'ARN ou de l'ARNm, ou de l'ADNc obtenu à partir de celles-ci.

**14.** Ensemble pour classer un échantillon de tumeur mammaire dans la catégorie "à forte probabilité de malignité" ou "à faible probabilité de malignité", ledit ensemble comprenant une pluralité d'éléments de fixation, chaque élément de fixation étant capable de se fixer de manière spécifique à un produit d'expression soit d'un classificateur à gènes multiples comprenant les gènes identifiés au tableau S4a et/ou au tableau S4b, soit d'un réactif de détection, dans lequel les éléments de fixation sont des domaines de fixation d'anticorps.

**15.** Ensemble selon la revendication 14, comprenant en outre un ou plusieurs profils d'expression standard contenus sur un support de données et pouvant être récupérés à partir de celui-ci, pour être comparés aux profils d'expression d'un échantillon d'analyse.

**16.** Ensemble selon la revendication 15, dans lequel lesdits un ou plusieurs profils d'expression standard sont produits selon le procédé de la revendication 10 ou de la revendication 11.

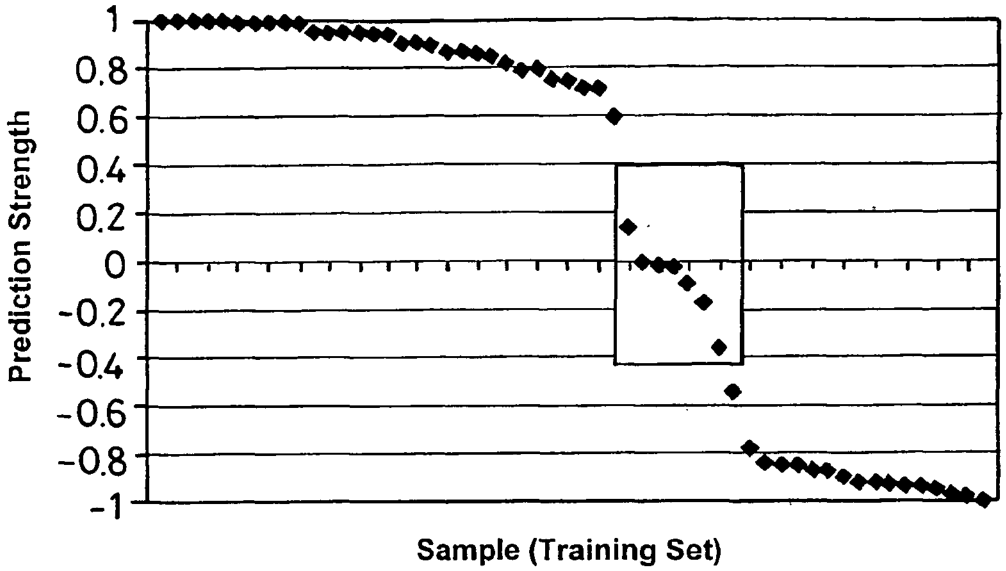
1
0.8
0.6
0.4
0.2
0
-0.2
-0.4
-0.6
-0.8
-1
Prediction Strength
Sample (Training Set)

*Fig. 1(a)*

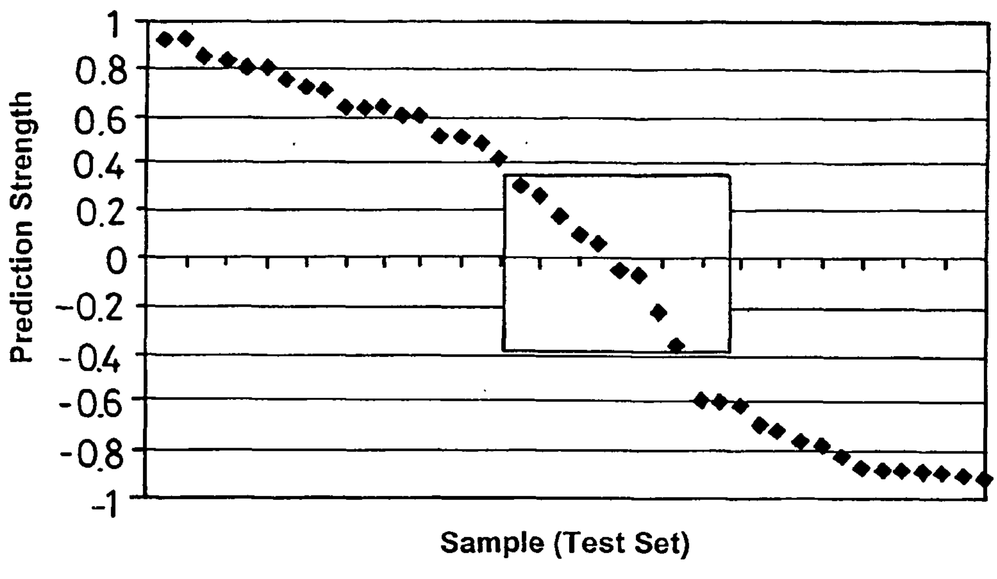
1
0.8
0.6
0.4
0.2
0
-0.2
-0.4
-0.6
-0.8
-1
Prediction Strength
Sample (Test Set)

*Fig. 1(b)*

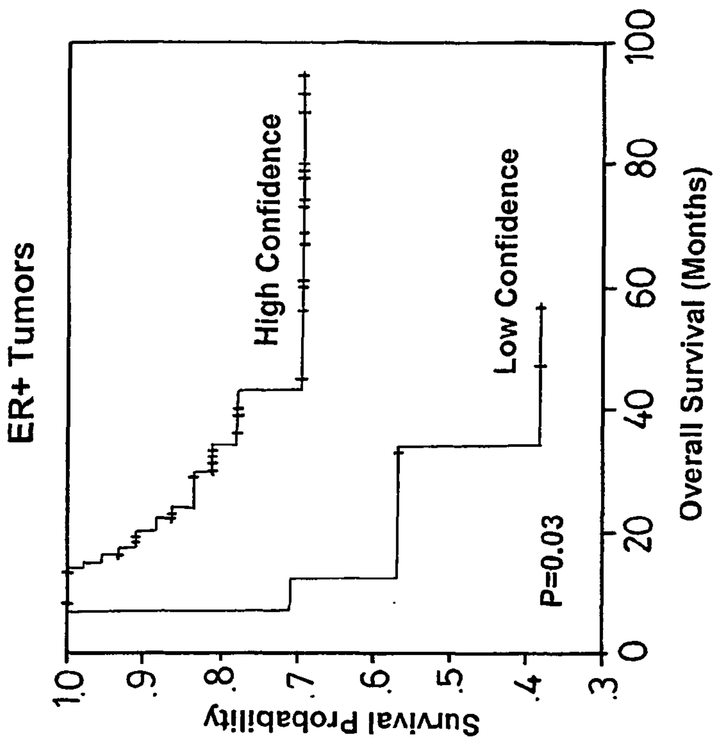
ER+ Tumors
Survival Probability
1.0
.9
.8
.7
.6
.5
.4
.3
0
20
40
60
80
100
Overall Survival (Months)
High Confidence
Low Confidence
P=0.03

Fig. 2(b)

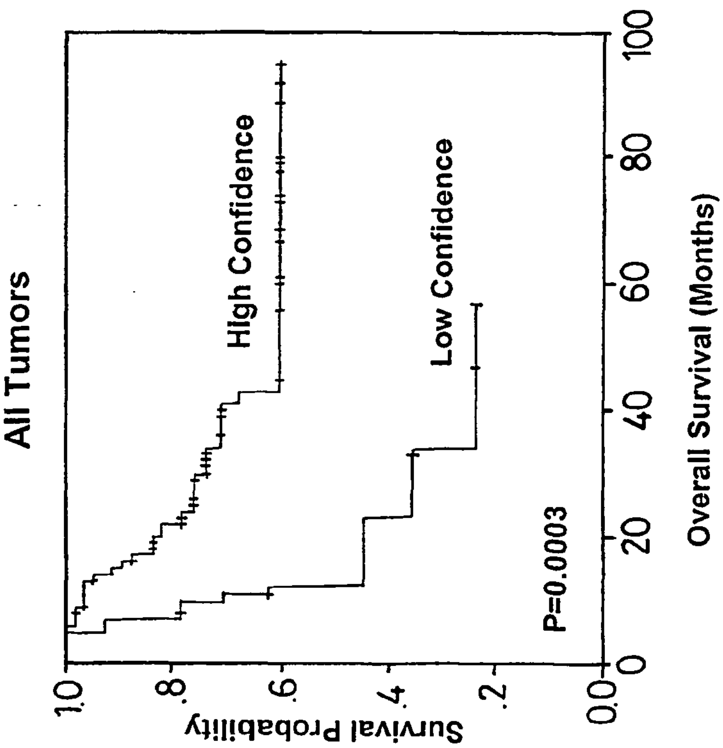
All Tumors
Survival Probability
1.0
.8
.6
.4
.2
0.0
0
20
40
60
80
100
Overall Survival (Months)
High Confidence
Low Confidence
P=0.0003

Fig. 2(a)

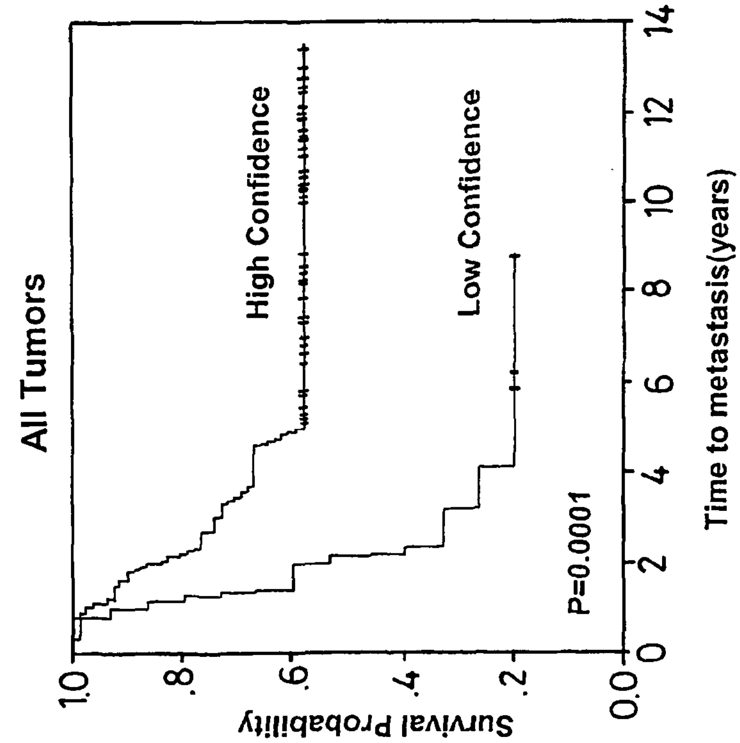
All Tumors
High Confidence
Low Confidence
P=0.0001
Survival Probability
1.0
.8
.6
.4
.2
0.0
0
2
4
6
8
10
12
14
Time to metastasis(years)

*Fig. 2(c)*

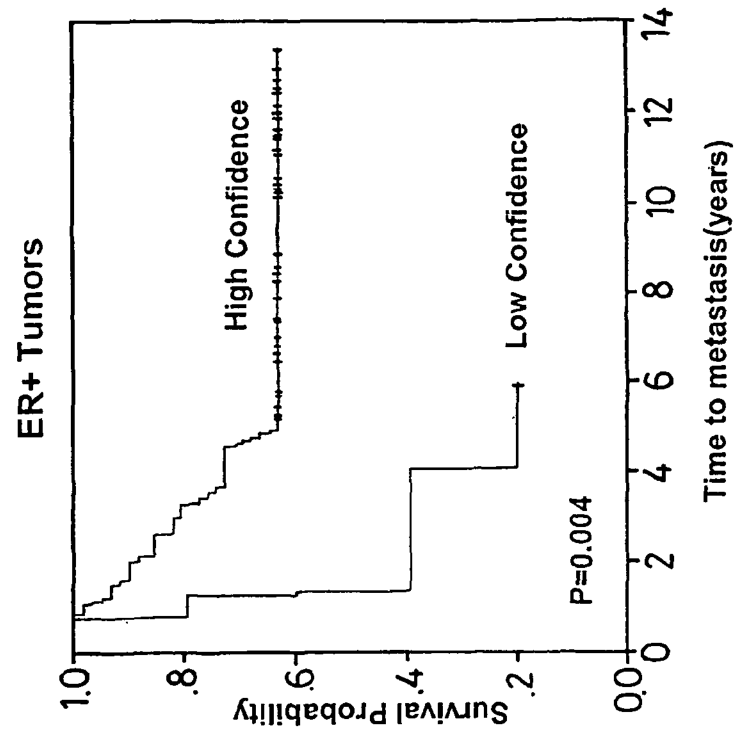
ER+ Tumors
High Confidence
Low Confidence
P=0.004
Survival Probability
1.0
.8
.6
.4
.2
0.0
0
2
4
6
8
10
12
14
Time to metastasis(years)

*Fig. 2(d)*

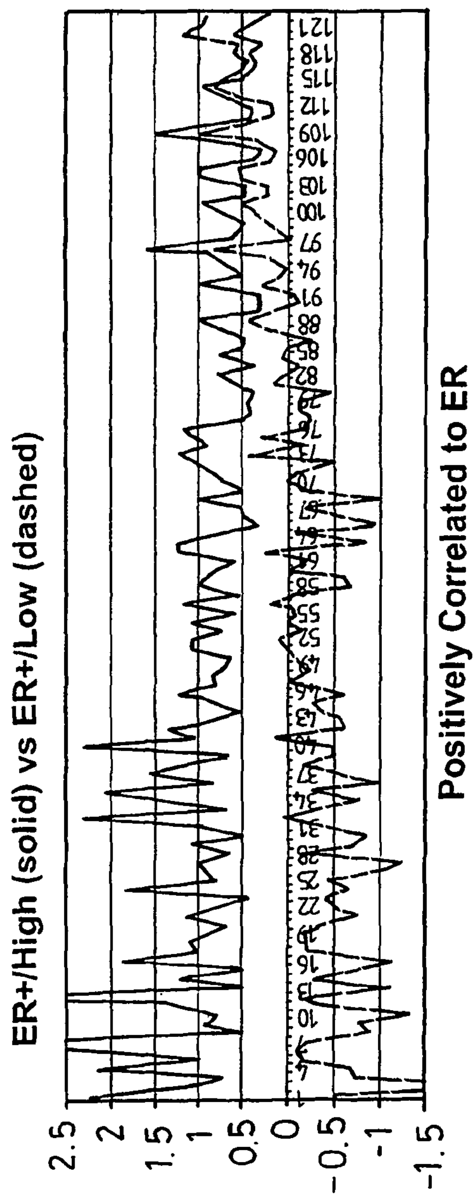


Positively Correlated to ER

*Fig. 3(a)*

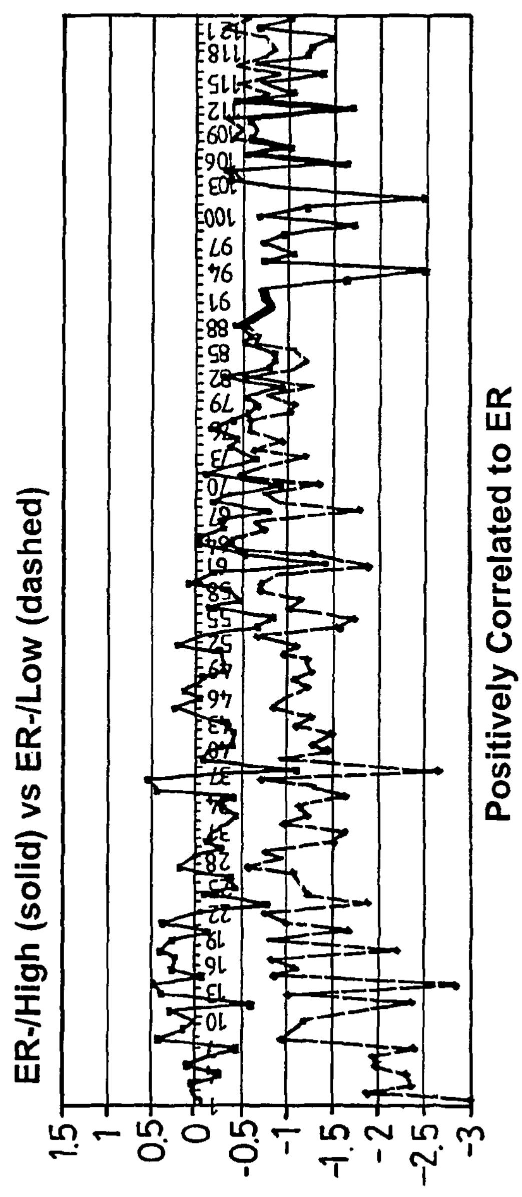


Positively Correlated to ER

*Fig. 3(b)*

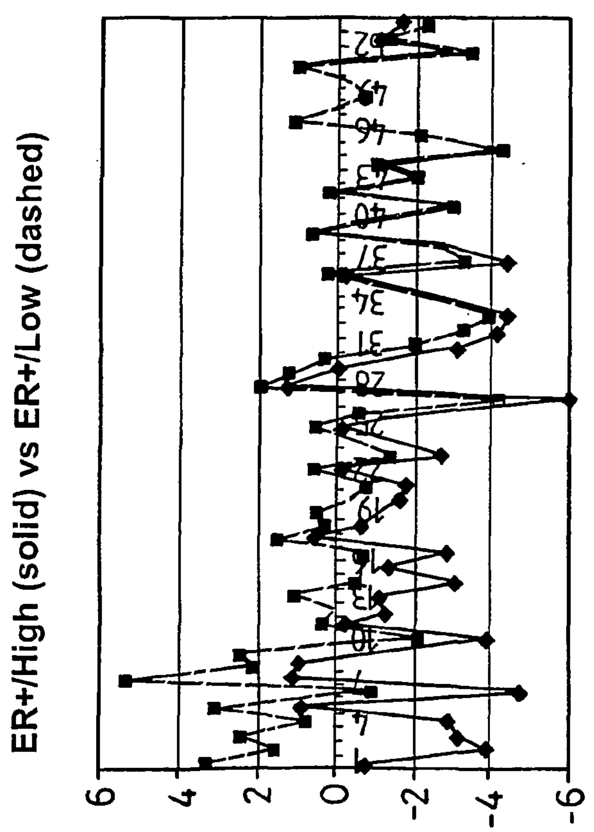


Negatively Correlated to ER

*Fig. 3(c)*

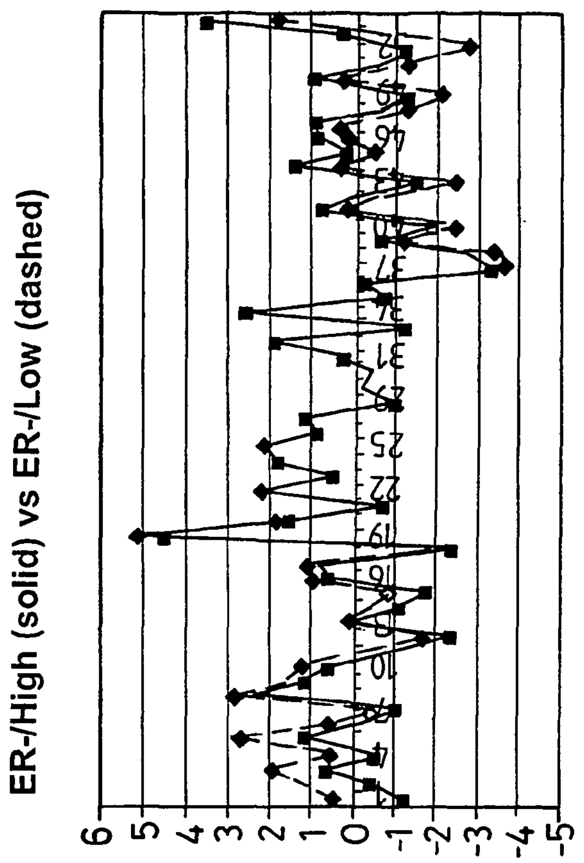


Negatively Correlated to ER

*Fig. 3(d)*

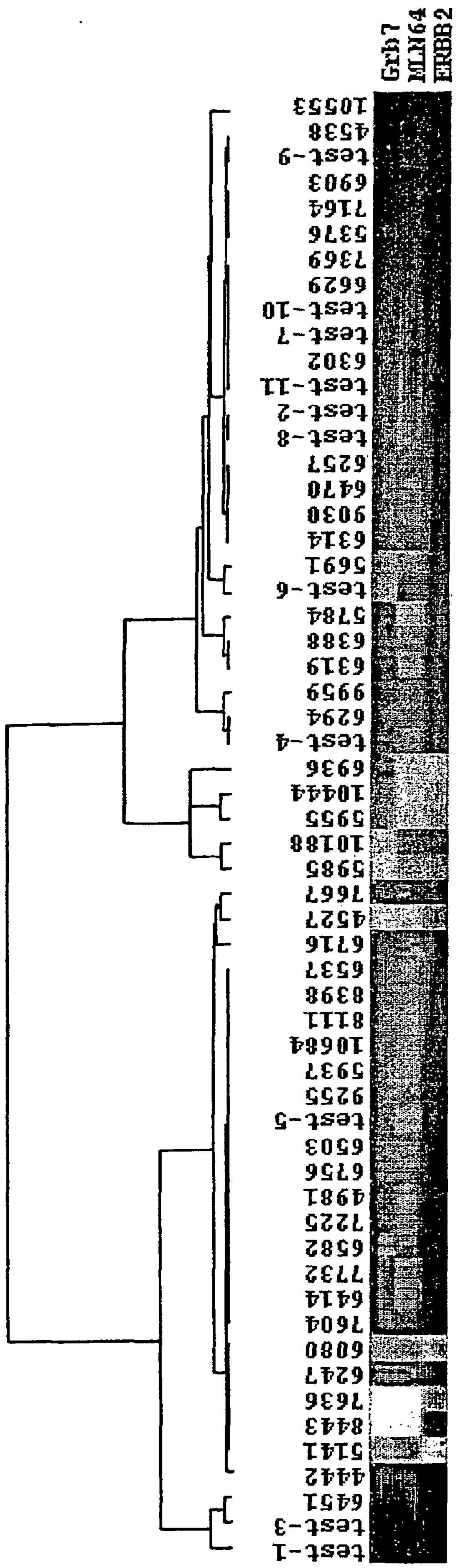
Grb7
MLN64
ERBB2
10553
4538
test-9
6903
7164
5376
7369
6629
test-10
test-7
6302
test-11
test-2
test-8
6257
6470
9030
6314
5691
test-6
5784
6388
6319
9959
6294
test-4
6936
10444
5955
10188
5985
7667
4527
6716
6537
8398
8111
10684
5937
9255
test-5
6503
6756
4981
7225
6582
7732
6414
7604
6080
6247
7636
8443
5141
4442
6451
test-3
test-1

*Fig. 4(a)*

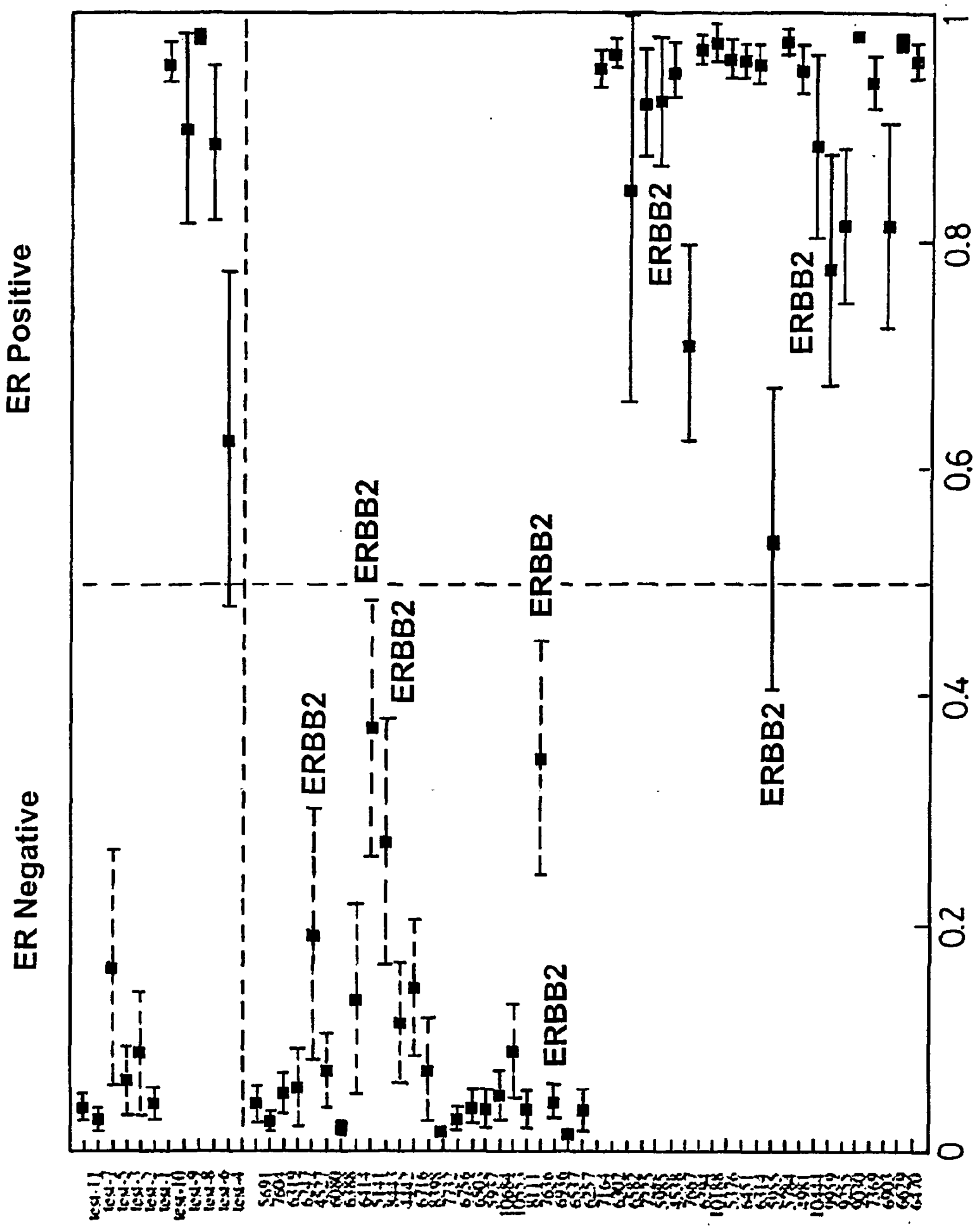
ER Negative
ER Positive
ERBB2
ERBB2
ERBB2
ERBB2
ERBB2
ERBB2
ERBB2
ERBB2
0
0.2
0.4
0.6
0.8
1
ANN committee vote

*Fig. 4(b)*

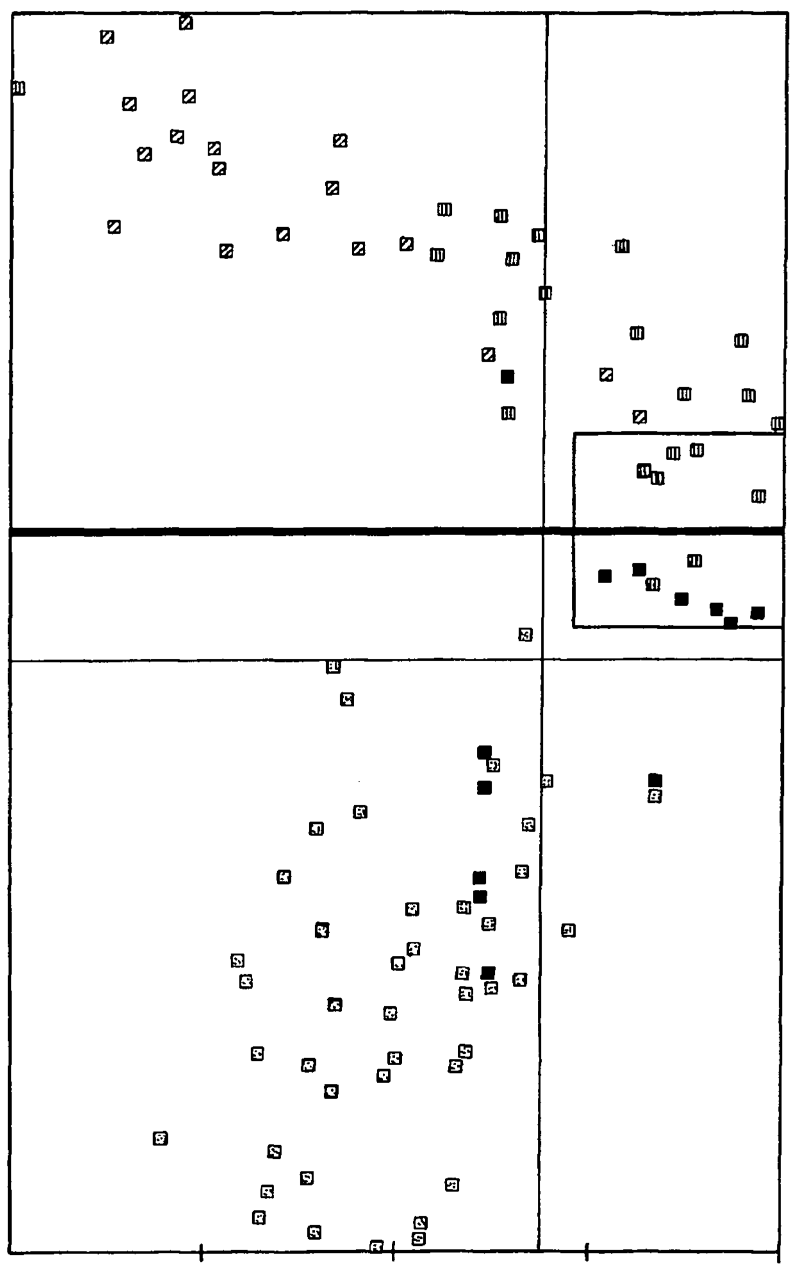


*Fig. 5*

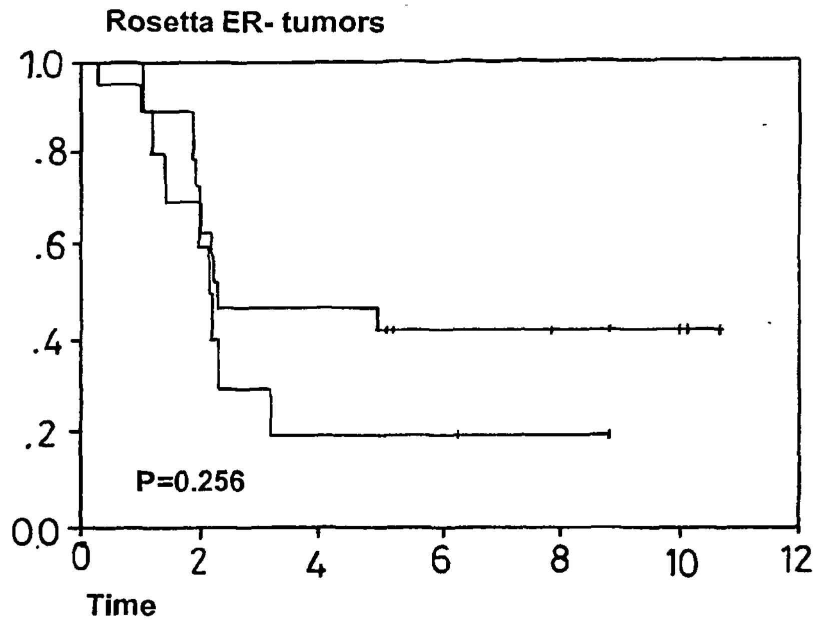
Rosetta ER- tumors
1.0
.8
.6
.4
.2
0.0
P=0.256
0
2
4
6
8
10
12
Time

*Fig. 6(a)*

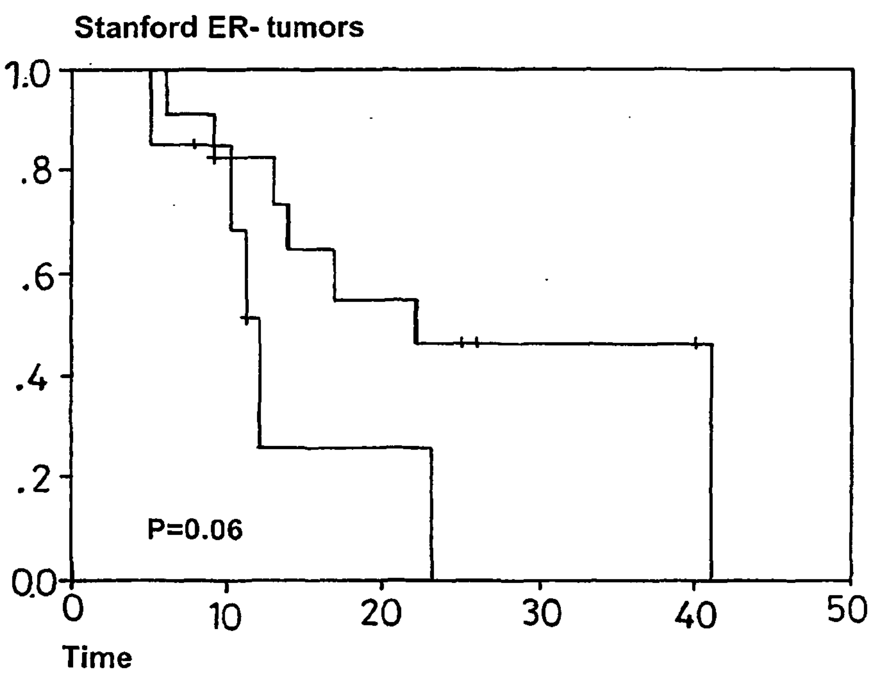
Stanford ER- tumors
1.0
.8
.6
.4
.2
0.0
P=0.06
0
10
20
30
40
50
Time

*Fig. 6(b)*

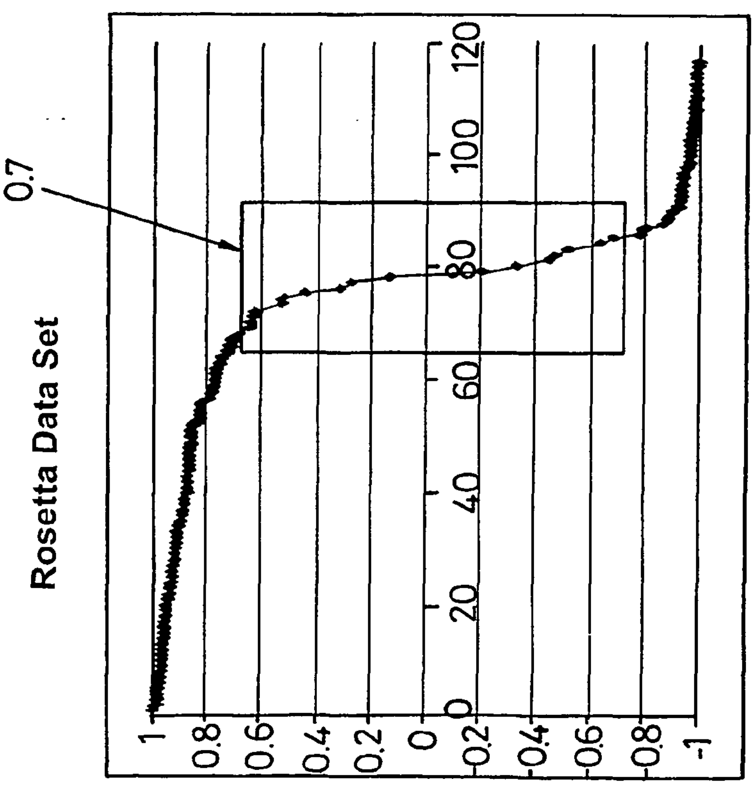
Rosetta Data Set
0.7
1
0.8
0.6
0.4
0.2
0
-0.2
-0.4
-0.6
-0.8
-1
0
20
40
60
80
100
120

*Fig. 7(a)*

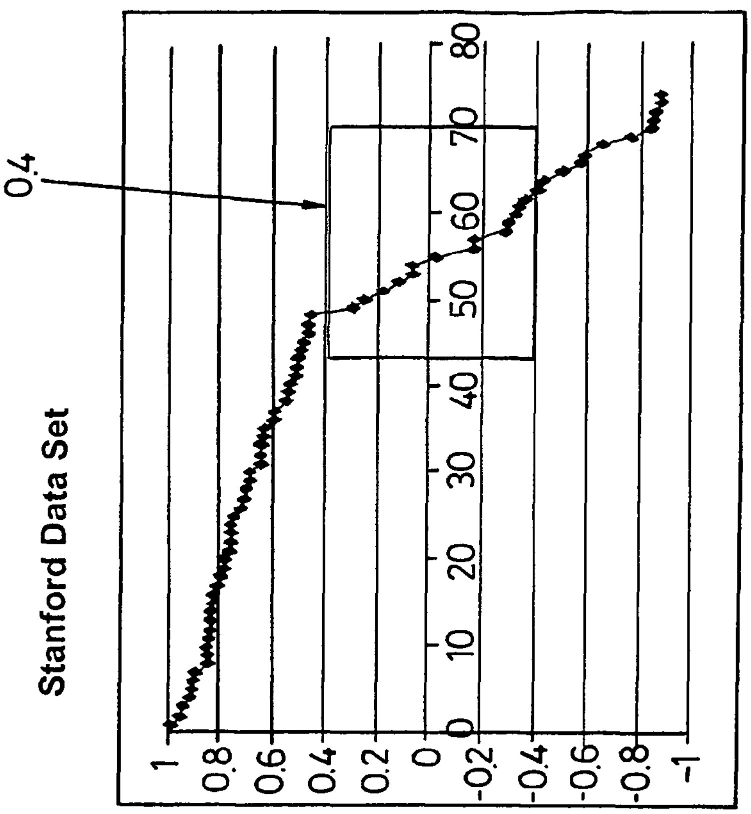
Stanford Data Set
0.4
1
0.8
0.6
0.4
0.2
0
-0.2
-0.4
-0.6
-0.8
-1
0
10
20
30
40
50
60
70
80

*Fig. 7(b)*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- GB 03000755 W

### Non-patent literature cited in the description


- **TAVASSOLI, F. A. ; SCHNITT S. J.** Pathology of the Breast. Elsevier, 1992
- **BISWAS, D.K. ; AVERBOUKH, L. ; SHENG, S. ; MARTIN, K. ; EWANIUK, D.S. ; JAWDE, T.F. ; WANG, F. ; PARDEE, A.B.** Classification of breast cancer cells on the basis of a functional assay for estrogen receptor. *Mol Med,* 1998, vol. 4, 454-467
- **GRUVBERGER, S. ; M. RINGNER ; Y. CHEN ; S. PANAVALLY ; L. H. SAAL ; A. BORG ; M. FERNO ; C. PETERSON ; P. MELTZER.** Estrogen Receptor Status in Breast Cancer is Associated with Remarkably Distinct Gene Expression Patterns. *Cancer Research,* 2001, vol. 61, 5979-5984
- **WEST, M. ; BLANCHETTE, C. ; DRESSMAN, H. ; HUANG, E. ; ISHIDA, S. ; SPANG, R. ; ZUZAN, H. ; OLSON, J.A. JR ; MARKS, J.R. ; NEVINS, J.R.** Predicting the clinical status of human breast cancer by using gene expression profiles. *Proc Natl Acad Sci U S A.,* 2001, vol. 98, 11462-67
- **PIETRAS R.J. ; ARBOLEDA, J. ; REESE, D.M. ; WONGVIPAT, N. ; PEGRAM, M.D. ; RAMOS, L. ; GORMAN, C.M. ; PARKER, M.G. ; SLIWKOWSKI, M.X. ; SLAMON, D.J.** HER-2 tyrosine kinase pathway targets estrogen receptor and promotes hormone-independent growth in human breast cancer cells. *Oncogene,* 1995, vol. 10, 2435-2446
- **KUROKAWA, H. ; ARTEAGA, C.L.** Inhibition of erbB receptor (HER) tyrosine kinases as a strategy to abrogate antiestrogen resistance in human breast cancer. *Clinical Cancer Research,* 2001, vol. 12, 4436s-4442s
- **BANGE, J. ; ZWICK, E. ; ULLRICH, A.** Molecular targets for breast cancer therapy and prevention. *Nature Medicine,* 2001, vol. 7, 548-552
- **CHIA, K. S. ; A. SEOW ; H. P. LEE ; K. SHANMUGARATNAM.** Cancer Incidence in Singapore. *Singapore Cancer Registry,* 2000, 1993-1997
- **SORLIE T ; PEROU CM ; TIBSHIRANI R ; AAS T ; GEISLER S, JOHNSEN H ; HASTIE T ; EISEN MB ; VAN DE RIJN M ; JEFFREY SS ; THORSEN T.** Gene expression patterns of breast carcinomas distinguish tumour subclasses with clinical implications. *Proc Natl Acad Sci U S A.,* 2001, vol. 98, 10869-74
- **VAN 'T VEER LJ ; DAI H ; VAN DE VIJVER MJ ; HE YD ; HART AA ; MAO M ; PETERSE HL ; VAN DER KOOY K ; MARTON MJ ; WITTEVEEN AT.** Gene expression profiling predicts clinical outcome of breast cancer. *Nature,* 2002, vol. 415, 530-6
- **TUSHER, V. G. ; R. TIBSHIRANI ; G. CHU.** Significance Analysis of Microarrays Applied to the Ionizing Radiation Response. *Proc. Natl. Acad. Sci U S A.,* 2001, vol. 98, 5116-5121
- **KALLIONIEMI A ; KALLIONIEMI OP ; PIPER J ; TANNER M ; STOKKE T ; CHEN L ; SMITH HS ; PINKEL D ; GRAY JW ; WALDMAN FM.** Detection and mapping of amplified DNA sequences in breast cancer by comparative genomic hybridization. *Proc Natal Acad Sci U S A.,* 1994, vol. 91, 2156-60
- **CHARPENTIER AH ; BEDNAREK AK ; DANIEL RL ; HAWKINS KA ; LAFLIN KJ ; GADDIS S ; MACLEOD MC ; ALDAZ CM.** Effects of estrogen on global gene expression: identification of novel targets of estrogen action. *Cancer Research,* 2000, vol. 60, 5977-83
- **BAJIC, V.B. ; TAN, S.L. ; CHONG, A. ; TANG, S. ; STROM, A. ; GUSTAFSSON, J. ; LIN, C.Y. ; LIU, E.** Dragon ERE Finder ver.2 : A tool for accurate detection and analysis of estrogen response elements in vertebrate genomes. *Nucleic Acid Res.,* 2002
- **ALIZADEH, A.A. ; M. B. EISEN ; R. E. DAVIS ; C. MA ; I. S. LOSSOS ; A. ROSENWALD ; J. C. BOLDRICK ; H. SABET ; T. TRUC ; Y. XIN.** Distinct types of diffuse large B-cell lymphoma identified by gene expression profiling. *Nature,* 2000, vol. 403, 503-511

- **BITTNER, M. ; P. MELTZER ; Y. CHEN ; Y. JIANG ; E. SEFTOR ; M. HENDEIX ; M. RADMACHER ; R. SIMON ; Z. YAKHINI ; A. BEN-DOR.** Molecular classification of cutaneous malignant melenoma by gene expression profiling. *Nature,* 2000, vol. 406, 536-540
- **GRUNT TW ; SACEDA M ; MARTIN MB ; LUPU R ; DITTRICH E ; KRUPITZA G ; HARANT H ; HUBER H ; DITTRICH C.** Bidirectional interactions between the estrogen receptor and the cerbB-2 signaling pathways: heregulin inhibits estrogenic effects in breast cancer cells. *Int J Cancer,* 1995, vol. 63, 560-567
- **STOICA GE ; FRANKE TF ; WELLSTEIN A ; MORGAN E ; CZUBAYKO F ; LIST HJ ; REITER R ; MARTIN MB ; STOICA A.** Heregulin-beta1 regulates the estrogen receptor-alpha gene expression and activity via the ErbB2/PI 3-K/Akt pathway. *Oncogene,* 2003, vol. 22, 2073-2087
- **MAZUMDAR, A. ; WANG, R.A. ; MISHRA, S.K. ; ADAM, L. ; BAGHERI-YARMAND, R. ; MANDAL, M. ; VADLAMUDI, R.K. ; KUMAR, R.** Transcriptional repression of oestrogen receptor by metastasis-associated protein 1 corepressor. *Nature Cell Biol,* 2000, vol. 3, 30-37
- **GOLUB TR ; SLONIM DK ; TAMAYO P ; HUARD C ; GAASENBEEK M ; MESIROV JP ; COLLER H ; LOH ML ; DOWNING JR ; CALIGIURI MA.** Molecular classification of cancer: class discovery and class prediction by gene expression monitoring. *Science,* 1999, vol. 286, 531-7
- **VAPNIK V.** Statistical Learning Theory. Wiley, 1998
- **RAMASWAMY S ; TAMAYO P ; RIFKIN R ; MUKHERJEE S ; YEANG CH ; ANGELO M ; LADD C ; REICH M ; LATULIPPE E ; MESIROV JP.** Multiclass cancer diagnosis using tumour gene expression signatures. *Proc Natl Acad Sci U S A.,* 2001, vol. 98, 15149-54
- **MUELLER, A. ; O'ROURKE, J. ; GRIMM, J. ; GUILLEMIN, K. ; DIXON, M.F. ; LEE, A. ; FALKOW, S.** Distinct gene expression profiles characterize the histopathological stages of disease in Helicobacter-induced mucosa-associated lymphoid tissue lymphoma. *Proc Natl Acad Sci USA,* 2003, vol. 100, 1292-1297
- **SANOUDOU, D. ; HASLETT, J.N. ; KHO, A.T. ; GUO, S. ; GAZDA, H.T. ; GREENBERG, S.A. ; LIDOV, H.G.V. ; KOHANE, I.S. ; KUNKEL, L.M. ; BEGGS, A.H.** Expression profiling reveals altered satellite cell numbers and glycolytic enzyme transcription in nemaline myopathy muscle. *Proc Natl Acad Sci U S A,* 2003, vol. 100, 4666-4671
- **ALTSCHUL,S.F. ; MADDEN,T.L. ; SCHAFFER, A.A. ; ZHANG,J. ; ZHANG,Z. ; MILLER,W. ; LIPMAN,D.J.** Gapped BLAST and PSI-BLAST: a new generation of protein database search programs. *Nucleic Acids Res.,* 1997, vol. 25, 3389-3402
- **CHONG, A. ; ZHANG, G. ; BAJIC, V.B.** Information and sequence extraction around the 5'-end and translation initiation site of human genes. *Silico Biology,* 2002, vol. 2, 461-465
- **CHONG, A. ; ZHANG, G. ; BAJIC, V.B.** FIE2: A program for the extraction of genomic DNA sequences around the start and translation initiation site of human genes. *Nucleic Acids Research,* 2003
- **EISEN MB ; SPELLMAN PT ; BROWN PO ; BOTSTEIN D.** Cluster analysis and display of genome-wide expression patterns. *Proc Natl Acad Sci USA.,* 1998, vol. 95 (25), 14863-14868